# EUROPÄISCHE PATENTSCHRIFT

(11) **EP 1 144 405 B1**
(45) Veröffentlichungstag und Bekanntmachung des Hinweises auf die Patenterteilung: **25.11.2009**
(21) Anmeldenummer: 00903574.2
(22) Anmeldetag: 12.01.2000
(51) Int. Cl.: C07D 401/12, C07D 401/14, A61K 31/41, A61P 25/16, A61P 25/18, A61P 25/24, C07D 413/14, C07D 417/14, C07D 401/06, C07D 409/14

(54) **TRIAZOLVERBINDUNGEN MIT DOPAMIN-D3-REZEPTORAFFINITÄT**
TRIAZOLE COMPOUNDS WITH DOPAMINE-D3-RECEPTOR AFFINITY
COMPOSES DE TRIAZOL PRESENTANT UNE AFFINITE POUR LE RECEPTEUR 3 DE LA DOPAMINE

(30) Priorität: 12.01.1999 DE 19900811
(43) Veröffentlichungstag der Anmeldung: 17.10.2001
(73) Patentinhaber: Abbott GmbH & Co. KG, 65205 Wiesbaden (DE)
(72) Erfinder: STARCK, Dorothea, D-67059 Ludwigshafen (DE); TREIBER, Hans-Jörg, D-68782 Brühl (DE); UNGER, Liliane, D-67065 Ludwigshafen (DE); NEUMANN-SCHULTZ, Barbara, D-68526 Ladenburg (DE); BLUMBACH, Kai, D-97337 Dettelbach (DE); SCHÖBEL, Dietmar, D-68167 Mannheim (DE)
(74) Vertreter: Kinzebach, Werner
(86) Internationale Anmeldenummer: PCT/EP2000/000177
(87) Internationale Veröffentlichungsnummer: WO 2000/042036

(56) Entgegenhaltungen:
- EP-A- 0 254 623
- EP-A- 0 887 350
- WO-A-96/02520
- WO-A-96/31512
- WO-A-97/17326
- WO-A-97/25324
- WO-A-99/02503
- DE-A- 4 425 144
- US-A- 4 338 453
- US-A- 4 577 020
- A. CZARNOCKA-JANOWICZ ET AL.: "Synthesis and pharmacological activity of 5-substituted-s-triazole-3-thiols" PHARMAZIE, Bd. 46, Nr. 2, 1991, Seiten 109-112, XP002082580 in der Anmeldung erwähnt

## Beschreibung

Die Erfindung betrifft Triazolverbindungen und die Verwendung dieser Verbindungen. Diese besitzen wertvolle therapeutische Eigenschaften und sind zur Behandlung von Erkrankungen brauchbar, die auf die Beeinflussung durch Dopamin-D₃-Rezeptor-Liganden ansprechen.

Verbindungen der hier in Rede stehenden Art mit physiologischer Aktivität sind bereits bekannt. So beschreiben die WO 94/25013; 96/02520; 97/43262; 97/47602; 98/06699; 98/49145; 98/50363; 98/50364 und 98/51671 Verbindungen, welche auf die Dopamin-Rezeptoren wirken. Weitere Verbindungen mit Aktivität als Dopamin-D₃-Rezeptor-Liganden sind aus der DE 44 25 144 A, WO 96/30333 und der WO 97/25324, WO 97/40015, WO 97/47602, WO 97/17326, EP 887 350, EP 779 284 A und aus Bioorg. & Med. Chem. Letters 9 (1999) 2059-2064 bekannt. Triazolverbindungen mit anti-allergischer oder anti-psychotischer Aktivität sind aus US 4,338,453; 4,408,049 und 4,577,020 bekannt. Die WO 93/08799 und WO 94/25013 beschreiben Verbindungen der hier in Rede stehenden Art, die Endothelin-Rezeptorantagonisten darstellen. In Pharmazie 46 (1991), 109-112, sind weitere Triazolverbindungen beschrieben, welche die Blutplättchenaggregation hemmen und blutdrucksenkend wirken. Weitere Triazolverbindungen mit physiologischer Aktivität sind aus EP 691 342, EP 556 119, WO 97/10210, WO 98/24791, WO 96/31512 und WO 92/20655 bekannt.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin.

Es liegen gesicherte Erkenntnisse über die Anwesenheit von Dopamin und dessen physiologische Funktion als Neurotransmitter vor. Störungen im dopaminergen Transmittersystem resultieren in Erkrankungen, wie z. B. Schizophrenie, Depression und Parkinson'sche Krankheit. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptoren pharmakologisch klar definiert, nämlich die D₁- und D₂-Rezeptoren.

In jüngerer Zeit wurde ein dritter Subtyp gefunden, nämlich der D₃-Rezeptor, der einige Effekte der Antipsychotika und Anti-Parkinsonmittel zu vermitteln scheint (J.C. Schwartz et al., The Dopamine D3 Receptor as a Target for Antipsychotics, in Novel Antipsychotic Drugs, H.Y. Meltzer, Ed. Raven Press, New York 1992, Seiten 135-144; M. Dooley et al., Drugs and Aging 1998, 12, 495-514).

Da D₃-Rezeptoren hauptsächlich im limbischen System exprimiert werden, wird angenommen, dass ein selektiver D₃-Ligand wohl die Eigenschaften bekannter Antipsychotika, nicht aber ihre Dopamin-D₂-Rezeptor-vermittelten neurologischen Nebenwirkungen haben sollte (P. Sokoloff et al., Localization and Function of the D3 Dopamine Receptor, Arzneim. Forsch./Drug Res. 42(1), 224 (1992); P. Sokoloff et al. Molecular Cloning and Characterization of a Novel Dopamine Receptor (D3) as a Target for Neuroleptics, Nature, 347, 146 (1990)).

Überraschenderweise wurde nun gefunden, dass bestimmte Triazolverbindungen eine hohe Affinität zum Dopamin-D₃-Rezeptor und eine geringe Affinität zum D₂-Rezeptor aufweisen. Es handelt sich somit um selektive D₃-Liganden.

Gegenstand der vorliegenden Erfindung sind daher die Verbindungen der allgemeinen Formel I: 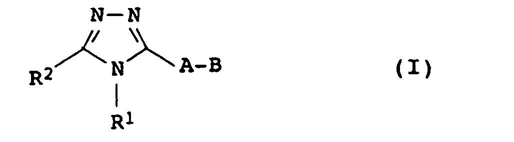 worin
- R¹: für H, C₁-C₆-Alkyl, das gegebenenfalls durch OH, OC₁-C₆-Alkyl, Halogen oder Phenyl substituiert ist, C₃-C₆-Cycloalkyl oder Phenyl steht;
- R²: für H, C₁-C₆-Alkyl, das gegebenenfalls durch OH, OC₁-C₆-Alkyl, Halogen oder Phenyl substituiert ist, C₁-C₆-Alkoxy, C₁-C₆-Al- kylthio, C₂-C₆-Alkenyl, C₂-C₆-Alkinyl, C₃-C₆-Cycloalkyl, Halo- gen, CN, COOR³, CONR³R⁴, NR³R⁴, SO₂R³, SO₂NR³R⁴ oder einen aro- matischen Rest steht, der ausgewählt ist unter Phenyl, Naph- thyl und einem 5- oder 6-gliedrigen heterocyclischen Rest mit 1, 2, 3 oder 4 Heteroatomen, die unabhängig voneinander aus- gewählt sind unter O, N und S, wobei der aromatische Rest ei- nen oder zwei Substituenten aufweisen kann, die unabhängig voneinander ausgewählt sind unter C₁-C₆-Alkyl, das gegebenen- falls durch OH, OC₁-C₆₋Alkyl, Halogen oder Phenyl substituiert ist, C₁-C₆-Alkoxy, C₂-C₆-Alkenyl, C₂-C₆-Alkinyl, C₃-C₆-Cycloal- kyl, Halogen, CN, COR³, NR³R⁴, NO₂, SO₂R³, SO₂NR³R⁴ und Phenyl, das gegebenenfalls durch ein oder zwei Reste substituiert ist, die unabhängig voneinander ausgewählt sind unter C₁-C₆-Alkyl, C₁-C₆-Alkoxy, NR³R⁴, CN, CF₃, CHF₂ oder Halogen;
- R³ und R⁴: unabhängig voneinander für H, C₁-C₆-Alkyl, das gegebe- nenfalls durch OH, OC₁-C₆-Alkyl, Halogen oder Phenyl substi- tuiert ist, oder Phenyl stehen;
- A: für -Z-CH₂CH₂CH₂-, -Z-CH₂CH₂CH₂CH₂-, -Z-CH₂CH=CHCH₂-, -Z-CH₂C(CH₃)=CHCH₂-, 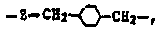 -Z-CH₂CH(CH₃)CH₂- oder für einen linearen -Z-C₇-C₁₀-Alkylenrest steht, wobei Z an den Triazolring gebunden ist und wobei Z für CH₂, O oder S steht, oder A steht für -(CH₂)₄-, -(CH₂)₅-, -CH₂CH₂CH=CHCH₂-, 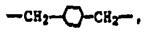 -CH₂CH₂C(CH₃)=CHCH₂- oder -CH₂CH₂CH(CH₃)CH₂-;
- B: einen Rest der folgenden Formel bedeutet: 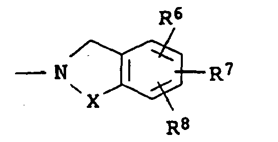 worin
X für CH₂ oder CH₂CH₂ steht;
R⁶, R⁷ und R⁸ unabhängig voneinander ausgewählt sind unter H, C₁-C₆-Alkyl, das gegebenenfalls durch OH, OC₁-C₆-Alkyl, das gegebenenfalls durch Amino, Mono- oder Di-C₁-C₄-alkylamino substituiert ist, C₁-C₆-Alkylthio, Halogen oder Phenyl substi- tuiert ist, OH, C₁-C₆-Alkoxy, OCF₃, OSO₂CF₃, SH, C₁-C₆-Alkylt- hio, C₂-C₆-Alkenyl, C₂-C₆-Alkinyl, Halogen, CN, NO₂, CO₂R³, SO₂R³, SO₂NR³R⁴, wobei R³ und R⁴ die oben angegebenen Bedeutun- gen besitzen und zusammen mit dem N-Atom, an das sie gebunden sind, auch einen gesättigten oder ungesättigten Heterocyclus mit 5 bis 7 Ringatomen und 1 oder 2 N- und/oder O-Heteroato- men bilden können, CONR³R⁴, NHSO₂R³, NR³R⁴, einem 5- oder 6-gliedrigen carbocyclischen, aromatischen oder nicht-aroma- tischen Ring und einem 5- oder 6-gliedrigen heterocyclischen, aromatischen oder nicht-aromatischen Ring mit 1 oder 2 Hete- roatomen, die unabhängig voneinander ausgewählt sind unter O, N und S, wobei der carbocyclische oder heterocyclische Ring ein oder zwei Substituenten aufweisen kann, die unabhängig voneinander ausgewählt sind unter C₁-C₆-Alkyl, Phenyl, Phe- noxy, Halogen, C₁-C₆-Alkoxy, OH, NO₂, CF₃ und CHF₂ und wobei zwei der Substituenten R⁶, R⁷ und R⁸ zusammen mit den Kohlen- stoffatomen des Phenylrings, an die sie gebunden sind, einen an den Phenylring ankondensierten Phenyl-, Cyclopentyl- oder Cyclohexylring bilden können, worin eine oder zwei der CH- oder der CH₂-Gruppen durch ein Stickstoffatom, eine NH- oder eine N-(C₁-C₆-Alkyl)-Gruppe ersetzt sein können;
sowie deren Salze mit physiologisch verträglichen Säuren.

Bei den erfindungsgemäßen Verbindungen handelt es sich um selektive Dopamin-D₃-Rezeptor-Liganden, die regioselektiv im limbischen System angreifen und aufgrund ihrer geringen Affinität zum D₂-Rezeptor nebenwirkungsärmer als die klassischen Neuroleptika sind, bei denen es sich um D₂-Rezeptorantagonisten handelt. Die Verbindungen sind daher zur Behandlung von Erkrankungen brauchbar, die auf Dopamin-D₃-Liganden ansprechen, d.h., sie sind zur Behandlung von solchen Erkrankungen wirksam, bei denen eine Beeinflussung (Modulation) der Dopamin-D₃-Rezeptoren zur Verbesserung des Krankheitsbildes oder zum Ausheilen der Krankheit führt. Derartige Erkrankungen sind z.B. Erkrankungen des Herz-Kreislaufsystems sowie der Niere, Erkrankungen des zentralen Nervensystems, insbesondere Schizophrenie, affektive Störungen, neurotische Belastungs- und somatoforme Störungen, Psychosen, Parkinson, Aufmerksamkeitsstörungen (attention deficit disorders), Hyperaktivität bei Kindern, Epilepsie, amnestische und kognitive Störungen, wie Lern und Gedächtnisschwäche (impaired cognitive function), Angstzustände, Demenz, Delirium, Persönlichkeitsstörungen, Schlafstörungen (z.B. Restless Legs Syndrome), Störungen des Sexuallebens (Impotenz des Mannes), Eßstörungen und Suchterkrankungen. Außerdem kommen sie zur Behandlung des Schlaganfalls in Betracht.

Zu Suchterkrankungen gehören die durch den Mißbrauch von psychotropen Substanzen, wie Arzneimittel oder Drogen, verursachten psychischen Störungen und Verhaltensstörungen sowie andere Suchterkrankungen, wie beispielsweise die Spielsucht (impulse control disorders not elsewhere classified). Suchterzeugende Substanzen sind beispielsweise: Opioide (z.B. Morphin, Heroin, Codein); Kokain; Nikotin; Alkohol; Substanzen, die mit dem GABA-Chlorid-Kanal-Komplex interagieren, Sedativa, Hypnotika oder Tranquilizer, beispielsweise Benzodiazepine; LSD; Cannabinoide; psychomotorische Stimulanzien, wie 3,4-Methylendioxy-N-methylamphetamin (Ecstasy); Amphetamin und amphetaminartige Substanzen wie Methylphenidat oder sonstige Stimulanzien einschließlich Koffein. Als suchterzeugende Substanzen kommen insbesondere Opioide, Kokain, Amphetamin oder amphetaminartige Substanzen, Nikotin und Alkohol in Betracht.

Vorzugsweise werden die erfindungsgemäßen Verbindungen zur Behandlung von affektiven Störungen; neurotischen, Belastungs- und somatoformen Störungen und Psychosen, z.B. Schizophrenie, eingesetzt.

Im Rahmen der vorliegenden Erfindung besitzen die nachfolgenden Ausdrücke die anschließend angegebenen Bedeutungen:
Alkyl (auch in Resten wie Alkoxy, Alkylthio, Alkylamino etc.) bedeutet eine geradkettige oder verzweigte Alkylgruppe mit 1 bis 6 Kohlenstoffatomen und insbesondere 1 bis 4 Kohlenstoffatomen. Die Alkylgruppe kann einen oder mehrere Substituenten aufweisen, die unabhängig voneinander ausgewählt sind unter OH, OC₁-C₆-Alkyl, Halogen oder Phenyl. Im Falle eines Halogensubstituenten kann die Alkylgruppe insbesondere 1, 2, 3 oder 4 Halogenatome umfassen, die sich an einem oder mehreren C-Atomen befinden können, vorzugsweise in α- oder ω-Position. Besonders bevorzugt sind CF₃, CHF₂, CF₂Cl oder CH₂F.

Beispiele für eine Alkylgruppe sind Methyl, Ethyl, n-Propyl, isoPropyl, n-Butyl, iso-Butyl, t-Butyl, etc.

Cycloalkyl steht insbesondere für C₃-C₆-Cycloalkyl, wie Cyclopropyl, Cyclobutyl, Cyclopentyl und Cyclohexyl.

Alkylen steht für geradkettige oder verzweigte Reste.

A steht erfindungsgemäß für -Z-CH₂CH₂CH₂-, -Z-CH₂CH₂CH₂CH₂-, -Z-CH₂CH=CHCH₂-, -Z-CH₂C(CH₃)=CHCH₂-, 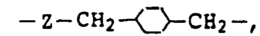 -Z-CH₂CH(CH₃)CH₂- oder für einen linearen - Z-C₇-C₁₀-Alkylenrest, wobei Z an den Triazolring gebunden ist. Z steht für CH₂, 0 und insbesondere S. Weiterhin steht A für -(CH₂)₄-, -(CH₂)₅-, -CH₂CH₂CH=CHCH₂-, 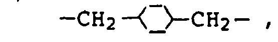 -CH₂CH₂C(CH₃)=CHCH₂- oder -CH₂CH₂CH(CH₃)CH₂-.
Halogen bedeutet F, Cl, Br oder I, vorzugsweise F oder Cl.
X steht vorzugsweise für -CH₂-CH₂- .
R¹ steht vorzugsweise für H, C₁-C₆-Alkyl oder C₃-C₆-Cycloalkyl.

Wenn R² für einen aromatischen Rest steht, handelt es sich vorzugsweise um einen der folgenden Reste: 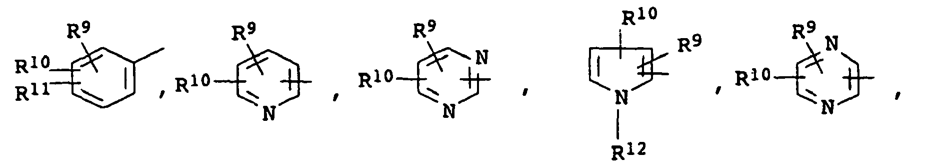 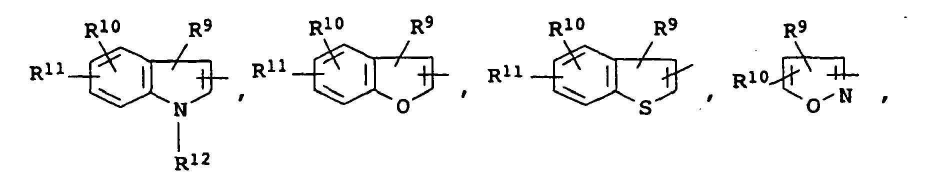 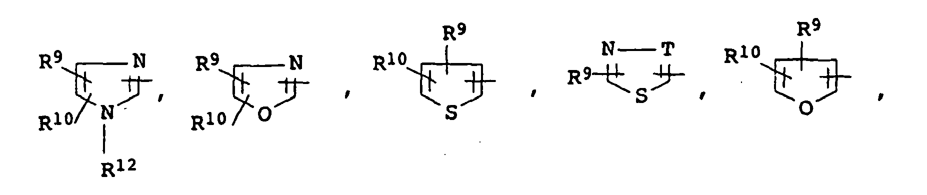 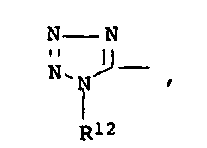 worin
- R⁹: bis R¹¹ für H oder die oben genannten Substituenten des aroma- tischen Restes stehen,
- R¹²: für H, C₁-C₆-Alkyl oder Phenyl steht und
- T: für N oder CH steht.

Wenn der Phenylrest substituiert ist, stehen die Substituenten vorzugsweise in m- oder p-Stellung.

Besonders bevorzugt handelt es sich bei dem aromatischen Rest um eine Gruppe der Formel: 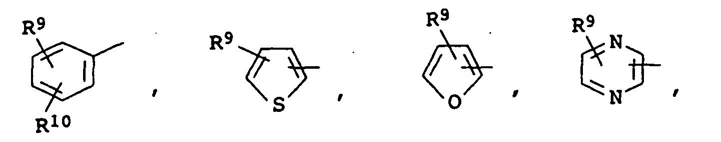 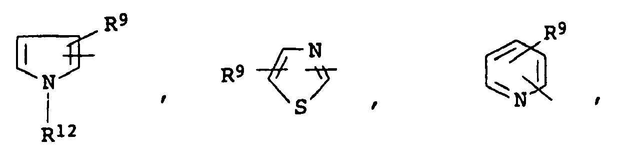 worin R⁹, R¹⁰ und R¹² die oben angegebenen Bedeutungen besitzen. Die angegebenen Phenyl-, Pyridin-, Thiazolyl- und Pyrrolreste sind insbesondere bevorzugt.

Die Reste R⁹ bis R¹¹ stehen vorzugsweise für H, C₁-C₆-Alkyl, OR³, CN, Phenyl, das gegebenenfalls durch C₁-C₆-Alkyl, C₁-C₆-Alkoxy oder Halogen substituiert ist, CF₃ und Halogen und insbesondere für H, C₁-C₆-Alkyl, OR³ und Halogen. R³ besitzt dabei die oben angegebenen Bedeutungen.

Besonders bevorzugt steht R² für H, C₁-C₆-Alkyl, NR³R⁴ (R³ und R⁴ stehen unabhängig voneinander für H oder C₁-C₆-Alkyl), Phenyl oder einen 5-gliedrigen aromatischen heterocyclischen Rest, der 1 oder 2 Heteroatome aufweist, die unabhängig ausgewählt sind unter N, S und O. Vorzugsweise handelt es sich bei dem heterocyclischen Rest um einen Pyrrol- oder Pyridinrest.

Vorzugsweise steht mindestens einer der Reste R⁶, R⁷ und R⁸ für H.

Die Reste R⁶, R⁷ und R⁸ sind vorzugsweise und unabhängig voneinander ausgewählt unter H, C₁-C₆-Alkyl, OH, C₁-C₆-Alkoxy, C₁-C₆-Alkylthio-C₁-C₆-alkyl, Halogen, CN, NO₂, SO₂R³, SO₂NR³R⁴ und CONR³R⁴. Besonders bevorzugt weist die annellierte Phenylgruppe einen oder zwei Substituenten auf, d.h. einer oder zwei der Reste R⁶, R⁷ und R⁸ stehen für C₁-C₆-Alkyl, Halogen, CN, NO₂, SO₂R³ und insbesondere SO₂NR³R⁴, wobei R³ und R⁴ zusammen mit dem N-Atom an das sie gebunden sind, auch für einen 5-, 6- oder 7-gliedrigen Heterocyclus, der neben dem Stickstoffatom auch ein oder zwei weitere Heteroatome, ausgewählt unter N, O oder S aufweisen und/oder substituiert sein kann, z.B. für Pyrrolidin, Piperidin, Morpholin oder Azepin stehen können.

Wenn einer der Reste R⁶, R⁷ und R⁸ für einen 5- oder 6-gliedrigen heterocyclischen Ring steht, so handelt es sich beispielsweise um einen Pyrrolidin-, Piperidin-, Morpholin-, Pyridin-, Pyrimidin-, Triazin-, Pyrrol-, Thiophen- oder Pyrazolrest, wobei ein Pyrrol-, Pyrrolidin-, Pyrazol- oder Thienylrest bevorzugt ist.

Wenn einer der Reste R⁶, R⁷ und R⁸ für einen carbocyclischen Rest steht, handelt es sich insbesondere um einen Phenyl-, Cyclopentyl- oder Cyclohexylrest.

Besonders bevorzugt sind die Verbindungen der Formel I, worin
- R¹: für H, C₁C₆-Alkyl oder Phenyl steht,
- R²: für H, C₁-C₆-Alkyl, Phenyl, Thienyl, Furanyl, Pyridyl, Pyrro- lyl, Thiazolyl oder Pyrazinyl steht,
- A: für -SC₃-C₁₀-Alkylen, das gegebenenfalls eine Doppelbindung umfassen kann, steht,
- R⁶, R⁷ und R⁸: ausgewählt sind unter H, C₁-C₆-Alkyl, C₁-C₆-Alkoxy, Halogen, SO₂NR³R⁴, CN, NO₂, CF₃, CONR³R⁴, CHF₂, OSO₂CF₃, OCF₃ und NHSO₂-C₁-C₆-alkyl.
Hierin steht X besonders bevorzugt für CH₂CH₂.

Die Erfindung umfasst auch die Säureadditionssalze der Verbindungen der Formel I, mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff., Birkhäuser Verlag, Basel und Stuttgart, 1966, beschrieben.

Die Verbindungen der Formel I können ein oder mehrere Asymmetriezentren aufweisen. Zur Erfindung zählen daher nicht nur die Racemate, sondern auch die betreffenden Enantiomere und Diastereomere. Auch die jeweiligen tautomeren Formen zählen zur Erfindung.

Das Verfahren zur Herstellung der Verbindungen der Formel I besteht darin, dass man
a) eine Verbindung der allgemeinen Formel (II) 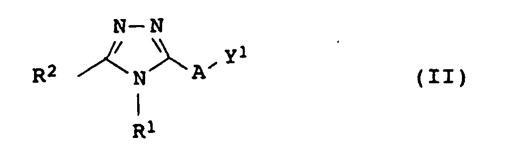 worin Y¹ für eine übliche Abgangsgruppe wie beispielsweise Hal, Alkylsulfonyloxy, Arylsulfonyloxy etc. steht, mit einer Verbindung der allgemeinen Formel (III)

   HB (III)

   umsetzt; oder
b) eine Verbindung der allgemeinen Formel (IV) 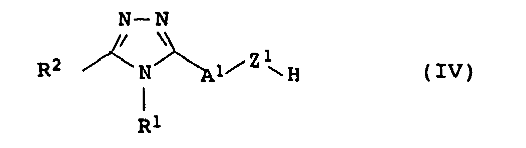 worin Z¹ für O oder S und A¹ für C₁-C₁₀-Alkylen oder eine Bindung steht, mit einer Verbindung der allgemeinen Formel (V)

   Y¹ - A² - B (V)

   wobei Y¹ die oben angegebene Bedeutung besitzt und A² für C₂-C₁₀-Alkylen steht, wobei A¹ und A² zusammen 3 bis 10 C-Atome aufweisen und A¹ und/oder A² gegebenenfalls wenigstens eine Gruppe Z umfassen, umsetzt; oder
c) eine Verbindung der allgemeinen Formel (VI) 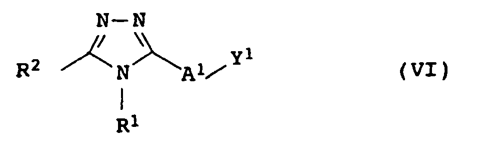 worin Y¹ und A¹ die oben angegebenen Bedeutungen besitzen, mit einer Verbindung der allgemeinen Formel (VII)

   H - Z¹ - A - B (VII)

   worin Z¹ die oben angegebenen Bedeutungen besitzt, umsetzt; oder
d) eine Verbindung der allgemeinen Formel (VIII) 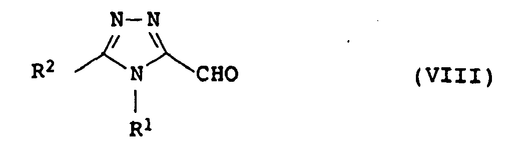 mit literaturbekannten Reagenzien, wie z. B. 1,3-Propandithiol, KCN/Wasser, TMSCN (Trimethylsilylcyanid) oder KCN/Morpholin, wie z. B. beschrieben in
   Albright Tetrahedron, 1983, 39, 3207 oder

   D. Seebach Synthesis 1969, 17 und 1979, 19 oder
   H. Stetter Angew. Chem. Int. Ed. 1976, 15, 639 oder
   van Niel et al. Tetrahedron 1989, 45, 7643
   Martin et al. Synthesis 1979, 633,
   zu den Produkten (VIIIa) (exemplarisch mit 1,3-Propandithiol) 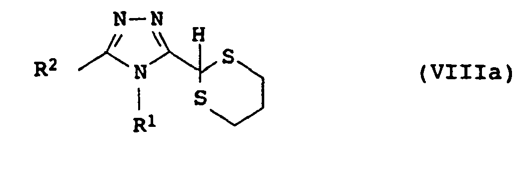 umpolt und anschließend mit Verbindungen der allgemeinen Formel (IX)

   Y¹ - A³ - B (IX)

   wobei Y¹ die oben angegebene Bedeutung besitzt und A³ für C₃-C₉-Alkylen steht, das eine Gruppe Z enthalten kann, kettenverlängert, wobei man nach Entschützen oder Reduktion
   Verbindungen der Formel (Ia) 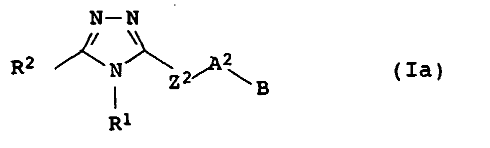 worin Z² für CO oder eine Methylengruppe steht und Z² und A² zusammen 4 bis 10 C-Atome aufweisen, erhält, oder
e) eine Verbindung der allgemeinen Formel (VIII) mit einer Verbindung der allgemeinen Formel (X)

   Y² - A - B (X)

   worin Y² für ein Phosphoran oder einen Phosphonsäureester steht, analog zu üblichen Methoden, wie zum Beispiel beschrieben in Houben Weyl "Handbuch der Organischen Chemie" 4. Auflage, Thieme Verlag Stuttgart, Band V/lb S.383 ff oder Bd V/1c S.575 ff, umsetzt, oder
f) eine Verbindung der allgemeinen Formel (XI) 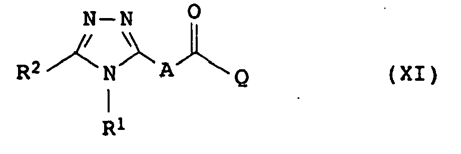 worin Q für H oder OH steht, mit einer Verbindung der Formel III unter reduktiven Bedingungen analog literaturbekannten Methoden, wie z. B. beschrieben in J. Org. Chem. 1986, 50, 1927; oder WO 92/20655 umsetzt.

Verbindungen der allgemeinen Formel B-H können hergestellt werden wie z.B. beschrieben in
Synth. Commun. 1984, 14, 1221;
S. Smith et al., Bioorg. Med. Chem. Lett. 1998, 8, 2859;
WO 97/47602 oder WO 920655 bzw.
J. Med. Chem. 1987, 30, 2111 und 2208 und 1999, 42, 118.

Die Verbindungen des Typs der Formel (IV) sind entweder bekannt oder können nach bekannten Verfahren hergestellt werden, wie z. B. beschrieben in A.R. Katritzky, C.W. Rees (ed.) "Comprehensive Heterocyclic Chemistry", Pergamon Press, oder "The Chemistry of Heterocyclic Compounds" J. Wiley & Sons Inc. NY und der dort zitierten Literatur oder in S. Kubota et al. Chem. Pharm. Bull 1975, 23, 955 oder Vosilevskii et al. Izv. Akad. Nauk. SSSR Ser. Khim 1975, 23, 955.

In den obigen Formeln besitzen R¹, R², R⁶, R⁷, R⁸, A, B und X die im Zusammenhang mit Formel I angegebenen Bedeutungen.

Die Herstellung der erfindungsgemäßen Verbindungen und der Ausgangsmaterialien und der Zwischenprodukte kann auch analog zu den in den eingangs genannten Patentpublikationen beschriebenen Methoden erfolgen.

Die oben beschriebenen Umsetzungen erfolgen im allgemeinen in einem Lösungsmittel bei Temperaturen zwischen Raumtemperatur und der Siedetemperatur des verwendeten Lösungsmittels. Brauchbare Lösungsmittel sind beispielsweise Ester, wie Ethylacetat, Ether, wie Diethylether oder Tetrahydrofuran, Dimethylformamid, Dimethylsulfoxid, Dimethoxyethan, Toluol, Xylol, Acetonitril, Ketone, wie Aceton oder Methylethylketon, oder Alkohole, wie Ethanol oder Butanol.

Gewünschtenfalls arbeitet man in Gegenwart eines säurebindenden Mittels. Geeignete säurebindende Mittel sind anorganische Basen, wie Natrium- oder Kaliumcarbonat, Natrium- oder Kaliumhydrogencarbonat, Natriummethylat, Natriumethylat, Natriumhydrid oder metallorganische Verbindungen, wie Butyllithium- oder Alkylmagnesium-Verbindungen, oder organische Basen, wie Triethylamin oder Pyridin. Letztere können gleichzeitig als Lösungsmittel dienen.

Das Verfahren (f) erfolgt unter reduzierenden Bedingungen, z.B. unter Verwendung von Natriumborhydrid, Natriumcyanoborhydrid oder Triacetoxyborhydrid, gegebenenfalls in saurem Medium oder in Gegenwart einer Lewissäure, wie z.B. Zinkchlorid oder mittels katalytischer Hydrierung.

Die Isolierung des Rohprodukts erfolgt in üblicher Weise, beispielsweise durch Filtration, Abdestillieren des Lösungsmittels oder Extraktion aus dem Reaktionsgemisch etc. Die Reinigung der erhaltenen Verbindungen kann in üblicher Weise erfolgen, beispielsweise durch Umkristallisieren aus einem Lösungsmittel, Chromatographie oder Überführen in eine Säureadditionsverbindung. Die Säureadditionssalze werden in üblicher Weise durch Mischen der freien Base mit der entsprechenden Säure, gegebenenfalls in Lösung in einem organischen Lösungsmittel, beispielsweise einem niedrigen Alkohol, wie Methanol, Ethanol oder Propanol, einem Ether, wie Methyl-t-butylether, einem Keton, wie Aceton oder Methylethylketon oder einem Ester, wie Essigsäureethylester, hergestellt.

Zur Behandlung der oben erwähnten Erkrankungen werden die erfindungsgemäßen Verbindungen in üblicher Weise oral oder parenteral (subkutan, intravenös, intramuskulär, intraperitoneal) verabreicht. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis etwa 10 bis 1000 mg pro Patient und Tag bei oraler Gabe und etwa 1 bis 500 mg pro Patient und Tag bei parenteraler Gabe.

Die Erfindung betrifft auch pharmazeutische Mittel, die die erfindungsgemäßen Verbindungen enthalten. Diese Mittel liegen in den üblichen galenischen Applikationsformen in fester oder flüssiger Form vor, beispielsweise als Tabletten, Filmtabletten, Kapseln, Pulver, Granulate, Dragees, Suppositorien, Lösungen oder Sprays. Die Wirkstoffe können dabei mit den üblichen galenischen Hilfsmitteln, wie Tablettenbindemitteln, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al., Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die nachfolgenden Beispiele dienen zur Erläuterung der Erfindung ohne sie zu begrenzen.

### Beispiel 1

### 6,7-Dimethoxy-2-{3-[(4-methyl-5-phenyl-4H-1,2,4-triazol-3-yl)sulfanyl]propyl}-1,2,3,4-tetrahydroisochinolin

### 1A Herstellung der Ausgangsstoffe

### 2-(3-Chlorpropyl)-6,7-dimethoxy-1,2,3,4-tetrahydro-isochinolin

7.2 g (37 mmol) 6,7-Dimethoxy-1,2,3,4-tetrahydroisochinolin wurden zusammen mit 4.05 ml (40 mmol) 1-Brom-3-chlorpropan, 11.3 g (81 mmol) Kaliumcarbonat und 610 mg (40 mmol) Natriumiodid in 250 ml Acetonitril vier Stunden unter Rühren auf 70°C erhitzt. Nach beendeter Reaktion destillierte man das Lösemittel ab, nahm den Rückstand in Wasser auf und extrahierte mit Methylenchlorid. Die vereinigten organischen Phasen wurden getrocknet, eingeengt und das Rohprodukt chromatographisch an Kieselgel (Laufmittel: Methylenchlorid / Methanol = 9/1) gereinigt.
Man erhielt 4.8 g (45% d.Th.) eines gelblichen Öles.

¹H-NMR (CDCl₃): δ = 2.0 (m, 2H); 2.6-2.8 (m, 6H); 3.5 (s, 2H); 3.6 (t, 2H); 3.8 (2s, 6H); 6.5 (s, 1H); 5.6 (s, 1H).
C₁₄H₂₀ClNO₂ (269)

### 1B Herstellung des Endproduktes

380 mg (1.7 mmol) 3-Mercapto-4-methyl-5-phenyl-1,2,4(4H)-triazol wurden mit 450 mg (1.7 mmol) der Chlorbase 1A und 40 mg (1.7 mmol) Lithiumhydroxid in 5 ml DMF fünf Stunden unter Rühren auf 100°C erwärmt. Zur Aufarbeitung versetzte man mit 50 ml Wasser, extrahierte mehrfach mit Methyl-tert.butylether, trocknete die vereinigten organischen Phasen, dampfte ein und reinigte chromatographisch an Kieselgel (Laufmittel: Methylenchlorid /2-5% Methanol) auf.
Ausbeute: 0.2 g (49% d.Th.)

¹H-NMR (CDCl₃): δ = 2.1 (q, 2H); 2.6 (m, 2H); 2.7 (m, 2H); 2.8 (m, 2H); 3.3 (t, 2H); 3.5 (m, 2H); 3.6 (s, 3H); 3.8 (2s, 6H); 6.3 (s, 1H); 6.5 (s, 1H); 7.5 (m, 3H); 7.8 (m, 2H).

Durch Behandlung mit etherischer Salzsäure erhielt man die Titelverbindung
C₂₃H₂₈N₄O₂S x HCl
Smp.: 180-183°C

### Beispiel 2

### 6-Methoxy-2-{3-[(4-Methyl-5-pyrrol-2yl-4H-1,2,4-triazol-3-yl)sulfanyl]propyl}-1,2,3,4-tetrahydroisochinolin

### 2A. Herstellung der Ausgangsverbindung

### 2-(3-Chlorpropyl)-6-methoxy-1,2,3,4-tetrahydroisochinolin

In analoger Weise zu 1A wurde die obige Substanz unter Verwendung von 6-Methoxy-1,2,3,4-tetrahydroisochinolin dargestellt.

¹H-NMR (CDCl₃): δ = 2.0 (q, 2H); 2.5-2.6 (m, 4H); 2.9 (m, 2H); 3.5 (s, 2H); 3.6 (m, 2H); 3.8 (s, 3H); 6.6 (d, 1H); 6.7 (dd, 1H); 6.9 (d, 1H).

### 2B Herstellung des Endproduktes

Die Herstellung erfolgte analog zu Beispiel 1 durch Reaktion der unter 2A dargestellten Chlorbase und 3-Mercapto-4-methyl-5-(2-pyrrolyl)-1,2,4(4H)-triazol.
Ausbeute: 52% d.Th.
C₂₀H₂₅N₅OS (383.5)
Fp.: 179-181°C

### Beispiel 3

### 2-{3-[(4-Methyl-5-phenyl-4H-1,2,4-triazol-3-yl)sulfanyl]propyl}-6-methoxy-1,2,3,4-tetrahydroisochinolin

### 3A Herstellung des Ausgangsmaterials

### 3-(3-Chlorpropylmercapto)-4-methyl-5-phenyl-1,2,4(4H)-triazol

Eine Suspension aus 2.6 g (16.5 mmol) 1-Brom-3-chlorpropan, 0.22 g (1.5 mmol) Natiumiodid, 2.7g (15 mmol) 3-Mercapto-4-methyl-5-phenyl-1,2,4(4H)-triazol und 2.1 g (15 mmol) Kaliumcarbonat in 70 ml Ethanol wurde eine Stunde zum Sieden erhitzt. Nach Heissfiltration engte man das Filtrat ein, nahm in Wasser auf und extrahierte mit Dichlormethan. Die vereinigten organischen Phasen wurden getrocknet, filtriert, eingeengt und der Rückstand chromatographiert (Laufmittel: Methylenchlorid / 2% Methanol).
Ausbeute: 1.35 g (34% d.Th.) weißer Feststoff

¹H-NMR (CDCl₃): δ = 2.3 (q, 2H); 3.4 (t, 2H); 3.6 (s, 3H); 3.7 (t, 2H); 7.5-7.7 (m, 5H).
C₁₂H₁₄ClN₃S (267.8)
Fp.: 137-141°C

### 3B Herstellung des Endproduktes

0.7g (2.5 mmol) der vorstehend beschriebenen Verbindung 3A wurde mit 0.6 g (2.5 mmol) 6-Methoxy-1,2,3,4-tetrahydroisochinolin-Oxalsäuresalz in Gegenwart von 1.1 ml (7.5 mmol) Triethylamin und katalytischen Mengen Natriumiodid in 6 ml Butanol bei 120°C über vier Stunden gerührt. Nach beendeter Reaktion arbeitete man mit Wasser und Methyl-tert.butylether extraktiv auf, trocknete über Natriumsulfat, engte ein und chromatographierte das Rohprodukt an Kieselgel (Laufmittel: Methylenchlorid mit 0-3% Methanol). Es wurden 110 mg eines weißen Feststoffes isoliert.
C₂₂H₂₆N₄OS (394.5) MS (m/z): 395 [M]⁺

### Beispiel 4

### 2-{3-[(4-Methyl-5-phenyl-4H-1,2,4-triazol-3-yl)sulfanyl]propyl}-7-(piperidin-1-ylsulfonyl)-1,2,3,4-tetrahydroisochinolin

### 4A Herstellung von N-Acetyl-7-(piperidin-1-ylsulfonyl)-1,2,3,4-tetrahydroisochinolin

Zu einer Lösung von 6.0g (70mmol) Piperidin und 10.9g (84 mmol) Diisopropylethylamin in 230ml THF wurden 21.1 g (77 mmol) 2-Acetyl-1,2,3,4-tetrahydroisochinolin-7-sulfonylchlorid (hergestellt wie beschrieben in G.Grunewald et al. J. Med. Chem 1999, 42, 118-134) in 50 ml THF zugetropft, und zwei Stunden unter Rückfluß erhitzt. Nach beendeter Reaktion entfernte man das Lösungsmittel im Vakuum, nahm mit Dichlormethan/Wasser auf, alkalisierte mit 10%-iger Natronlauge und trocknete nach Phasentrennung die organische Phase über Natriumsulfat. Das nach Filtration und Entfernen des Solvens verbleibende Rohprodukt wurde mittels Säulenchromatographie an Kieselgel (Laufmittel: Methylenchlorid mit 3% Methanol) gereinigt.
Ausbeute: 18.6 g (57.6 mmol); 82%
Smp.:171-174°C

### 4B 7-(Piperidin-1-ylsulfonyl)-1,2,3,4-tetrahydroisochinolin

Die vorstehend beschriebene Verbindung wurde zwei Stunden mit halbkonzentrierter Salzsäure zum Sieden erhitzt. Beim Abkühlen fiel das Produkt als weißer Niederschlag aus. Der Rückstand wurde isoliert, mit Wasser nachgewaschen, in Diethylether digeriert und unter Vakuum getrocknet.
Ausbeute: 12.1 g (38.2 mmol) 56% d.Th.

### 4C 2-(3-Chloropropyl)-7-(piperidin-4-ylsulfonyl)-1,2,3,4-tetrahydroisochinolin

12.1g (38.2 mmol) 7-(Piperidin-1-ylsulfonyl)-1,2,3,4-tetrahydroisochinolin und 8.4g (84 mmol) Triethylamin wurden in DMF bei 40°C gelöst, 9.0 g (57.2 mmol) 1,3-Brom-chlorpropan zugetropft und 7h bei 50°C gerührt. Zur Aufarbeitung wurde der Ansatz eingeengt, der Rückstand in Wasser aufgenommen und mit Dichlormethan extrahiert. Nach Trocknen über Natriumsulfat, Filtration und Entfernen des Lösemittels reinigte man chromatographisch auf (Kieselgel; Laufmittel: Methylenchlorid mit 3% Methanol) und erhielt 11.7g (323.7 mmol) eines gelblichen Öles.
Ausbeute: 86% d.Th.

### 4D Darstellung der Endverbindung

10.0 g (28.0 mmol) der vorbeschriebenen Chlorbase 4C, 6.4 g (28 mmol) 3-Mercapto-4-methyl-5-phenyl-4H-1,2,4-triazol und 0.7 g (28.0 mmol) Lithiumhydroxid wurden in 77 ml DMF drei Stunden bei 100°C erwärmt. Nach beendeter Reaktion entfernte man das Lösungsmittel, versetzte den Rückstand mit Wasser und extrahierte mit Essigester. Die vereinigten organischen Phasen wurden über Natriumsulfat getrocknet, filtriert und evaporiert. Chromatographie des Rohproduktes (Kieselgel; Laufmittel: Methylenchlorid mit 0-5% Methanol) ergab 3.9 g (7.5 mmol) eines weissen Feststoffes. Ausbeute: 27% d.Th.

¹H-NMR (CDCl₃): δ = 1.4 (m, 2H); 1.7 (m,4H); 2.1 (q, 2H); 2.7 (t, 2H); 2.8 (t,2H); 3.0 (m, 6H); 3.35 (t, 2H); 3.6 (s, 3H); 3.7 (s, 2H); 7.2 (d, 1H); 7.4 (s, 1H); 7.5 (m, 4H); 7.7 (m, 2H).
C₂₆H₃₃N₅O₂S₂ (511.7) MS (m/z): 512.3 [M+H]⁺ Smp.: 105-108°C

### Beispiel 5

### 2-[4-(4-Methyl-5-phenyl-4H-1,2,4-triazol-3-yl)butyl]-7-(morpholin-4-ylsulfonyl)-1,2,3,4-tetrahydroisochinolin Hydrochlorid

### Herstellung der Ausgangsverbindung

### 5A N-Acetyl-7-(morpholin-4-ylsulfonyl)-1,2,3,4-tetrahydroisochinolin

wurde wie unter Beispiel 4A beschrieben durch Umsetzung von Morpholin mit 2-Acetyl-1,2,3,4-tetrahydroisochinolin-7-sulfonsäurechlorid in Gegenwart von Diisopropylamin in THF erhalten und durch Erhitzen mit halbkonzentrierter Salzsäure und nach alkalischer Aufarbeitung in das entsprechende 7-(Morpholin-4-ylsulfonyl)-1,2,3,4-tetrahydroisochinolin überführt.
C₁₃H₁₉N₂O₃S (282) MS (m/z): 283 [M+H]⁺

### 5B 2-(3-Chloropropyl)-7-(morpholin-4-ylsulfonyl)-1,2,3,4-tetrahydroisochinolin

1.2 g (4.4 mmol) 7-(Morpholin-4-ylsulfonyl)-1,2,3,4-tetrahydroisochinolin und 1.0 g (10 mmol) Triethylamin wurden in DMF bei 40°C gelöst, 1.1 g (6.6 mmol) 1,3-Brom-chlorpropan zugetropft und 3h bei 40°C gerührt. Zur Aufarbeitung wurde der Ansatz eingeengt, der Rückstand in Wasser aufgenommen und mit Methyl-tert.Butylether extrahiert. Nach Trocknen über Natriumsulfat, Filtration und Entfernen des Lösemittels reinigte man chromatographisch (Kieselgel; Laufmittel: Methylenchlorid mit 2% Methanol) und erhielt 0.7 g (2 mmol) eines hellen Öles. Ausbeute: 46% d.Th.
¹H-NMR (CDCl₃): δ = 2.0 (q, 2H); 2.7 (t, 2H); 2.8 (t,2H); 3.0 (m, 6H); 3.6-3.8 (m, 8H); 7.3 (d, 1H); 7.4 (s, 1H); 7.5 (d, 1H).
C₁₆H₂₃N₂O₃S (359)

### Darstellung der Endverbindung

280 mg (1 mmol) 2-[4-Methyl-5-phenyl-1,2,4-(4H)-triazol-3-yl]-1,3-dithian (beschrieben in WO 9902503) wurden in 2.5 ml trockenem THF gelöst und bei -70°C unter Zusatz von 0.15 g Natriumiodid mit 0.75 ml (1.2 mmol) einer 15%-igen Lösung von Butyllithium in n-Hexan behandelt. Nach 45 min Rühren bei -70°C wurden 0.37 g (1 mmol) 2-[3-Chlorpropyl]-7-(morpholin-4-yl-sulfonyl)-1,2,3,4-tetrahydroisochinolin 5B gelöst in THF zugetropft. Man erwärmte nun langsam auf Raumtemperatur und heizte noch 90 min auf 40°C auf, um vollständigen Umsatz zu,erzielen. Zur Aufarbeitung wurde auf Eis/Wasser gegeben und mehrfach mit Methylenchlorid extrahiert. Nach Trocknen und Einengen hinterblieben 0.5 g (82% d.Th.) des substituierten Dithians, das anschließend mit Raney-Nickel und Wasserstoff bei 40°C im Verlauf von 3 Stunden in Tetrahydrofuran hydriert wurde. Nach Abtrennen des Katalysators wurde der Rückstand chromatographisch gereinigt (Kieselgel, Methylenchlorid-mit 5% Methanol).
Ausbeute: 120 mg (29% d.Th.)
¹H-NMR (CDCl₃): δ = 1.8 (m, 2H); 2.0 (q, 2H); 2.6 (m, 2H); 2.7 (t, 2H); 2.9 (t, 2H); 3.0 (m, 6H); 3.6 (s, 3H); 3.7 (m, 6); 7.2 (d, 1H); 7.4 (s, 1H); 7.5 (m, 4H); 7.7 (m, 2H).

Durch Versetzen mit etherischer HCl wurde die Titelverbindung erhalten
C₂₆H₃₃N₅O₃S·HCl (531.6)
Smp.: 87-89°C

In analoger Weise wurden erhalten:

### Beispiel 6

### 1-(4-Methyl-5-phenyl-4H-1,2,4-triazol-3-yl)-4-(7-(piperidin-1-yl-sulfonyl)-1,2,3,4-tetrahydroisochinolin-2yl)butan-1-on

C₂₇H₃₃N₅O₃S (507.7) MS: 508.3 [M+H]⁺

### Beispiel 7

### 2-{3-[(4-Methyl-5-phenyl-4H-1,2,4-triazol-3-yl)sulfanyl]propyl}-1,2,3,4-tetrahydroisochinolin-7-carbonitril

C₂₂H₂₃N₅S (389.5)
Smp.: 116-118°C

### Beispiel 8

### 5-[2-(Diethylammonio)ethoxy]-2-{3-[(4-methyl-5-phenyl-4H-1,2,4-triazol-3-yl)sulfanyl]propyl}-1,2,3,4-tetrahydroisochinolin Dihydrochlorid

C₂₇H₃₇N₅OS·2HCl (552.6)
Smp.: 110-112°C

### Beispiel 9

### N-Benzyl-2-(3-{[4-methyl-5-(4-methyl-1,3-thiazol-5-yl)-4H-1,2,4-triazol-3-yl]sulfanyl}propyl)-1,2,3,4-tetrahydroisochinolin-7-sulfonamid

C₂₆H₃₀N₆O₂S₃ (554.8)
Smp.: 67-70°C

### Beispiel 10

### N-Benzyl-2-{3-[(4-methyl-5-pyridin-3-yl-4H-1,2,4-triazol-3-yl)sulfanyl]propyl}-1,2,3,4-tetrahydroisochinolin-7-sulfonamid

C₂₇H₃₀N₆O₂S₂·2HCl (607.6)
Smp.: 81-84°C

### Beispiel 11

### 5-Methoxy- 2-{3-[(4-methyl-5-phenyl-4H-1,2,4-triazol-3-yl)sulfanyl]propyl}-1,2,3,4-tetra-hydroisochinolin

C₂₂H₂₆N₄OS (394.5)
Smp.: 73-75°C

### Beispiel 12

### 2-{3-[(4-Methyl-5-phenyl-4H-1,2,4-triazol-3-yl)sulfanyl]propyl}-7-nitro-1,2,3,4-tetra-hydroisochinolin

C₂₁H₂₄ClN₅O₂S (446)
Smp.: 190-192°C

### Beispiel 13

### 2-{3-[(4-Methyl-5-phenyl-4H-1,2,4-triazol-3-yl)sulfanyl]propyl}-1,2,3,4-tetra-hydro-isochinolin

¹H-NMR (CDCl₃): δ = 2.1 (q, 2H); 2.65 (t, 2H); 2.7 (t, 2H); 2.9 (t,2H); 3.4 (t, 2H); 3.5 (s, 3H); 3.7 (s, 2H); 7.0 (m, 1H); 7.2 (m, 3H); 7.5 (m, 3H); 7.7 (m, 2H).
C₂₁H₂₄N₄S (365.5)

### Beispiel 14

### 2-(3-{[4-Methyl-5-(4-methyl-1,3-thiazol-5-yl)-4H-1,2,4-triazol-3-yl]sulfanyl}propyl)-1,2,3,4-tetrahydroisochinolin

¹H-NMR (CDCl₃): δ = 2.1 (q, 2H); 2.55 (s, 3H); 2.7 (t, 2H); 2.75 (t, 2H); 2.9 (t,2H); 3.4 (t, 2H); 3.5 (s, 3H); 3.65 (s, 2H); 7.0 (m, 1H); 7.1 (m, 3H); 8.9(s, 1H).
C₁₉H₂₃N₅S₂ (386.5)

### Beispiel 15

### 2-{3-[(4-Methyl-5-pyridinium-3-yl-4H-1,2,4-triazol-3-yl)sulfa-nyl]propyl}-1,2,3,4-tetra-hydroisochinolin Dihydrochlorid

C₂₀H₂₃N₅S·2HCl (438.4)
Smp.: 87-89°C

### Beispiel 16

### 7-[(Dimethylamino)sulfonyl]-2-{3-[(4-methyl-5-phenyl-4H-1,2,4-triazol-3-yl)sulfanyl]-propyl}-1,2,3,4-tetrahydroisochinolin

¹H-NMR (CDCl₃): δ = 2.1 (q, 2H); 2.65 (m, 8H); 2.75 (t, 2H); 3.0 (t, 2H); 3.3 (t, 2H); 3.6 (s, 3H); 3.7 (s, 2H); 7.2 (d, 1H); 7.4-7.6 (m, 7H).
C₂₃H₂₉N₅O₂S₂ (472.6)

### Beispiel 17

### 7-[(Dimethylamino)sulfonyl]-2-(3-{[4-methyl-5-(4-methyl-1,3-thiazol-5-yl)-4H-1,2,4-triazol-3-yl]sulfanyl}propyl)-1,2,3,4-tetrahydroisochinolin

¹H-NMR (CDCl₃): δ = 2.1 (q, 2H); 2.5 (s, 3H); 2.6-2.8 (m, 10H); 2.9 (m, 2H); 3.4 (t, 2H); 3.5 (s, 3H); 3.7 (s, 2H); 7.2 (m, 1H); 7.5 (m, 2H); 8.9 (s, 1H).
C₂₁H₂₈N₆O₂S₃ (493.7)

### Beispiel 18

### 2-{3-[(4-methyl-5-phenyl-4H-1,2,4-triazol-3-yl)sulfanyl]propyl}-1,2,3,4-tetra-hydro-isochinolin-7-carboncäuremethylester Oxalat

C₂₃H₂₇N₄O₂S·C₂HO₄ (512.6)
Smp.: 160-163°C

### Beispiel 20

### 2-(3-{[4-Methyl-5-(4-methyl-1,3-thiazol-5-yl)-4H-1,2,4-triazol-3-yl]sulfanyl}propyl)-7-(piperidin-1-ylsulfonyl)-1,2,3,4-tetrahydroisochinolin

¹H-NMR (CDCl₃): δ = 1.4 (m, 2H); 1.7 (m, 4H); 2.1 (q, 2H); 2.5 (s, 3H); 2.6 (t, 2H); 2.7 (t, 2H); 3.0 (m, 6H); 3.3 (t, 2H); 3.5 (s, 3H); 3.6 (s, 2H); 7.2 (d, 1H); 7.45 (s, 1H); 7.5 (d, 1H); 8.9 (s, 1H).
C₂₄H₃₂N₆O₂S₃ (532.8)

### Beispiel 21

### 2-{3-[(4-Methyl-5-phenyl-4H-1,2,4-triazol-3-yl)sulfanyl]propyl}-7-(phenylsulfonyl)-1,2,3,4-tetrahydroisochinolin

¹H-NMR (CDCl₃): δ = 2.1 (q, 2H); 2.6 (t, 2H); 2.7 (t, 2H); 2.9 (t, 2H); 3.35 (t, 2H); 3.5 (s, 3H); 3.6 (m, 2H); 7.2 (d, 1H); 7.4-7.7 (m, 10H); 7.9 (d, 2H).
C₂₇H₂₈N₄O₂S₂ (504.7)

### Beispiel 22

### 2-(3-{[4-Methyl-5-(4-methyl-1,3-thiazol-5-yl)-4H-1,2,4-triazol-3-yl]sulfanyl}propyl)-1,2,3,4-tetrahydroisoquinolin-7-yl phenylsulfon

¹H-NMR (CDCl₃): δ = 2.1 (q, 2H); 2.5 (s, 3H); 2.7 (t, 2H); 2.8 (t, 2H); 2.95 (t, 2H); 3.4 (t, 2H); 3.5 (s, 3H); 3.65 (m, 2H); 7.2 (d, 1H); 7.4- 7.7 (m, 5H); 7.9 (d, 2H); 8.9 (s, 1H).
C₂₅H₂₉N₅O₂S₃ (525.7)

### Beispiel 23

### 2-{3-[(4-Methyl-5-phenyl-4H-1,2,4-triazol-3-yl)sulfanyl]propyl}-7-(morpholin-4-ylsulfonyl)-1,2,3,4-tetrahydroisochinolin

¹H-NMR (CDCl₃): δ = 2.1 (q, 2H); 2.7 (t, 2H); 2.8 (t, 2H); 3.0 (t, 4H); 3.35 (t, 2H); 3.6 (s, 3H); 3.7 (m, 6H); 7.3 (m, 1H); 7.4-7.6 (m, 5H); 7.9 (d, 2H).
C₂₅H₃₁N₅O₃S₂ (525.7)

### Beispiel 24

### 2-[4-(4-Methyl-5-phenyl-4H-1,2,4-triazol-3-yl)butyl]-7-(phenylsulfonyl)-1,2,3,4-tetrahydroisochinolin

C₂₈H₃₀N₄O₂S (486.6)

### Beispiel 25

### 2-{3-[(4-Methyl-5-pyridin-3-yl-4H-1,2,4-triazol-3-yl)sulfanyl]propyl}-N-phenyl-1,2,3,4-tetrahydroisochinolin-7-sulfonamid

¹H-NMR (CDCl₃): δ = 1.3 (m, NH); 2.1 (q, 2H); 2.6 (m, 4H); 2.8 (t, 2H); 3.3 (t, 2H); 3.6 (s, 3H); 3.7 (m, 6H); 7.3 (m, 1H); 7.4- 7.6 (m, 5H); 7.9 (d, 2H).
C₂₆H₂₈N₆O₂S₂ (520.7)
Smp.: 58-61°C

### Beispiel 26

### 2-(3-{[4-Methyl-5-(4-methyl-1,3-thiazol-5-yl)-4H-1,2,4-triazol-3-yl]sulfanyl}propyl)-N-phenyl-1,2,3,4-tetrahydroisochinolin-7-sulfonamid

¹H-NMR (CDCl₃): δ = 2.1 (q, 2H); 2.5 (s, 3H); 2.7 (m, 4H); 2.9 (m, 2H); 3.3 (t,2H); 3.5 (s, 3H); 3.6 (s, 32H); 7.0-7.2 (m, 6H); 7.5 (m, 2H); 8.9 (s, 1H).
C₂₅H₂₈N₆O₂S₃ (540.7)
Smp.: 77-81°C

### Beispiel 27

### 2-(3-{[5-(2,4-Dimethoxy)phenyl)-4-methyl-4H-1,2,4-triazol-3-yl]sulfanyl}propyl)-7-(methyl-sulfonyl)-1,2,3,4-tetrahydroisochinolin

¹H-NMR (CDCl₃): δ = 2.2 (q, 2H); 2.9 (m, 2H); 3.0 (m, 2H); 3.05 (s, 3H); 3.1 (m, 2H); 3.3 (m, 5H); 3.7 (s, 3H); 3.85 (s, 3H); 3.9 (s, 2H); 6.5 (s, 1H); 6.65 (d, 1H); 7.25 (d, 1H); 7.3 (d, 1H); 7.7 (s, 1H); 7.8 (d, 1H).
C₂₄H₃₀N₄O₄S₂ (502.7) MS: 503.5 [M+H]⁺

### Beispiel 28

### 6,7-Dichloro-2-{3-[(4-methyl-5-phenyl-4H-1,2,4-triazol-3-yl)sulfanyl]propyl}-1,2,3,4-tetrahydroisochinolin

C₂₁H₂₂Cl₂N₄S (433.4)
Smp.: 138-139°C

### Beispiel 29

### 7,8-Dichloro-2-{3-[(4-methyl-5-phenyl-4H-1,2,4-triazol-3-yl)sulfanyl]propyl}-1,2,3,4-tetrahydroisochinolin Hydrochlorid

1H-NMR (CDCl3): δ = 2.1 (q, 2H); 2.7 (m,4H); 2.9 (t, 2H); 3.3 (t,2H); 3.6 (s, 3H); 3.7 (s, 2H); 6.95 (d, 1H); 7.2 (d, 1H); 7.5 (m, 3H); 7.7 (m, 2H), [freie Base].
Salzfällung mit etherischer HCl führte zur Titelverbindung
C₂₁H₂₂Cl₂N₄S·x HCl (469.9)
Smp.: 109°C

### Beispiel 30

### 7-Cyno-2-[4-(4-methyl-5-phenyl-4H-1,2,4-triazol-3-yl)butyl]-1,2,3,4-tetrahydroisochinolin Hydrochlorid

C₂₃H₂₅N₅·HCl (407.9)
Smp.: 175°C

### Beispiel 31

### 2-{3-[(4-Methyl-5-thien-3-yl-4H-1,2,4-triazol-3-yl)sulfanyl]propyl}-6-(trifluoromethyl)-1,2,3,4-tetrahydroisochinolin Hydrochlorid

C₂₀H₂₁F₃N₄S₂·Cl x HCl (475)
Smp.: 184-185°C

### Beispiel 32

### 1-{2-[3-({4-Methyl-5-[4-(trifluoromethyl)phenyl]-4H-1,2,4-triazol-3-yl}sulfanyl)propyl]-1,2,3,4-tetrahydroisoquinolin-7-yl}-ethanon

¹H-NMR (CDCl₃): δ = 2.15 (q, 2H); 2.4 (s, 3H); 2.7 (t, 2H); 2.8 (t, 2H); 3.0 (t, 2H); 3.3 (t, 2H); 3.6 (s, 3H); 3.75 (s, 2H); 7.1 (d, 1H); 7.6 -7.8 (m, 6H).
C₂₄H₂₅F₃N₄OS (474.5)
Das Hydrochlorid der Titelverbindung erhielt man durch Behandlung mit etherischer Salzsäure:
Smp.: 183°C

### Beispiel 33

### 6,7-Dichloro-2-(3-{[4-methyl-5-(4-methylphenyl)-4H-1,2,4-triazol-3-yl]sulfanyl}propyl)-1,2,3,4-tetrahydroisochinolin Hydrochlorid

¹H-NMR (CDCl₃): δ = 2.1 (q, 2H);2.4 (s, 3H); 2.7 (m, 4H); 2.8 (t, 2H); 3.3 (t, 2H); 3.5 (s, 2H); 3.6 (s, 3H); 7.1 (s, 1H); 7.2 (s, 1H); 7.3 (d, 2H); 7.5 (d, 2H); [freie Base].
Durch Behandlung mit etherischer Salzsäure erhielt man die Titelverbindung
C₂₂H₂₄Cl₂N₄S·HCl (483.9)
Smp.: 207-210°C

### Beispiel 34

### 6-Chloro-2-{3-[(4-methyl-5-phenyl-4H-1,2,4-triazol-3-yl)sulfanyl]propyl}-1,2,3,4-tetrahydroisochinolin Hydrochlorid

¹H-NMR (CDCl₃): δ = 2.1 (q, 2H); 2.4 (s, 3H); 2.7 (m, 4H); 2.8 (t, 2H); 3.3 (t, 2H); 3.5 (s, 2H); 3.6 (m, 5H); 6.9 (d, 1H); 7.1 (m, 2H); 7.5 (d, 3H); 7.5 (d, 2H); [freie Base].
Salzfällung mit etherischer HCl führte zur Titelverbindung
C₂₁H₂₃ClN₄S·HCl (435.4)
Smp.: 188-191°C

### Beispiel 35

### 2-(3-{[4-methyl-5-(1-methyl-1H-pyrrol-2-yl)-4H-1,2,4-triazol-3-yl]sulfanyl}propyl)-7-(piperidin-1-ylsulfonyl)-1,2,3,4-tetrahydroisochinolin

¹H-NMR (CDCl₃): δ = 1.4 (m, 2H); 1.7 (m, 4H); 2.1 (q, 2H); 2.7 (t, 2H); 2.8 (t, 2H); 3.0 (m, 6H); 3.35 (t, 2H); 3.6 (s, 3H); 3.7 (s, 2H); 3.9 (s, 3H); 6.2 (m,1H); 6.4 (m,1H); 6.8 (m,1H); 7.2 (d, 1H); 7.4 (s, 1H); 7.5 (m, 2H).
C₂₅H₃₄N₆O₂S₂ (514.7)
Smp.: 96-100°C

### Beispiel 36

### 2-[4-(4-Methyl-5-phenyl-4H-1,2,4-triazol-3-yl)butyl]-7-(piperidin-1-ylsulfonyl)-1,2,3,4-tetrahydroisochinolin

C₂₇H₃₅N₅O₂S (493.7) MS: 494.3 [M+H]⁺

### Beispiel 37

### 2-(3-{[4-Methyl-5-thien-3-yl)-4H-1,2,4-triazol-3-yl]sulfanyl}propyl)-7-(piperidin-1-ylsulfonyl)-1,2,3,4-tetrahydroisochinolin

¹H-NMR (CDCl₃): δ = 1.4 (m, 2H); 1.7 (m,4H); 2.15 (q, 2H); 2.7 (t, 2H); 2.8 (t, 2H); 3.0 (m, 6H); 3.3 (t, 2H); 3.7 (m, 5H); 7.2 (d, 1H); 7.4 (s, 1H); 7.5 (m, 3H); 7.7 (s, 1H).
C₂₄H₃₁N₅O₂S₃ (517.7) MS: 518.3 [M+H]⁺
Smp.: 192-195°C

### Beispiel 38

### 2-{3-[(4-Methyl-5-phenyl-4H-1,2,4-triazol-3-yl)sulfanyl]propyl}-N-phenyl-1,2,3,4-tetrahydroisochinolin-7-sulfonamide

¹H-NMR (CDCl₃): δ = 2.1 (q, 2H); 2.6 (t, 2H); 2.7 (t, 2H); 2.9 (t, 2H); 3.3 (t,2H); 3.55 (s, 2H); 3.6 (s, 3H); 7.0 (m, 2H); 7.2 (m, 4H); 7.5 (m, 5H); 7.7 (m, 2H).
C₂₇H₂₉N₅O₂S₂ (519.7) MS: 520.3 [M+H]⁺

### Beispiel 39

### 6-Chloro-2-{3-[(4-methyl-5-thien-3-yl-4H-1,2,4-triazol-3-yl)sulfanyl]propyl}-1,2,3,4-tetrahydroisochinolin

C₁₉H₂₁ClN₄S₂ (405)
Smp.: 99-100°C

### Beispiel 40

### 7-[(Diethylammonio)-methyl]-2-{3-[(4-methyl-5-phenyl-4H-1,2,4-triazol-3-yl)sulfanyl]propyl}-1,2,3,4-tetrahydroisochinolin Dihydrochlorid

C₂₆H₃₅N₅S·2HCl (522.6)
Smp.: 75°C

### Beispiel 41

### 2-{3-[(4-Methyl-5-thien-3-yl-4H-1,2,4-triazol-3-yl)sulfanyl]propyl}-7-(trifluoromethyl)-1,2,3,4-tetrahydroisochinolin Hydrochlorid

### Herstellung des Ausgangsmaterials

### 41A 7-Trifluormethyl-1,2,3,4-tetrahydroisochinolin

Zu einer Lösung von 1.77g (6.2 mmol) N-Trifluoracetyl-2-(4-trifluorphenyl)ethylamin [hergestellt aus 2-(4-Trifluorphenyl)ethylamin und Trifluoressigsäureanhydrid bei -5°C] in 7.5 ml Eisessig tropfte man langsam 10.0 ml konzentrierte Schwefelsäure und versetzte tropfenweise unter Eiskühlung mit 2ml Formalinlösung. Nach 18 Stunden bei Raumtemperatur gab man die Reaktionsmischung auf 130ml Eiswasser, extrahierte mit Dichlormethan, wusch die vereinigten organischen Phasen mit Natriumhydrogencarbonat-Lösung, dann mit Wasser. Nach Trocknen über Natriumsulfat, Filtration und Evaporation wurden 1.7 g 2-Trifluoracetyl-7 trifluormethyl-1,2,3,4-tetrahydroisoquinolin isoliert, die durch Erhitzen unter Rückfluß in Ethanol /3N HCl (1:1) und alkalischer Aufarbeitung in 7-Trifluormethyl-1,2,3,4-tetrahydroisochinolin überführt wurden. Ausbeute: 1.0 (4.7 mmol) 75% d.Th.

¹H-NMR (CDCl₃): δ = 2.0 (sbr, 1H); 2.9 (t, 2H); 3.2 (t, 2H); 4.0 (s, 2H); 7.2 (d, 1H); 7.3 (s, 1H); 7.4 (s, 1H).

### 41B 2-(3-Chloropropyl)-7-trifluormethyl-1,2,3,4-tetrahydroisochinolin

0.95 g (4.7 mmol) der vorstehend beschriebenen Verbindung wurden in an sich gleicher Weise wie unter Beispiel 4B beschrieben mit 1-Brom-3-chlorpropan bei Raumtemperatur umgesetzt und chromatographisch gereinigt (Kieselgel, Laufmittel Dichlormethan mit 2% Methanol).
Ausbeute: 0.9 g (3.2 mmol) 69% d.Th.

¹H-NMR (CDCl₃): δ = 2.0 (m, 2H); 2.65 (m, 2H); 2.75 (m, 2H); 2.9 (m, 2H); 3.65 (m, 4H); 7.2 (dd, 1H); 7.3 (d, 1H); 7.4 (dd, 1H).

### 41C Herstellung des Endproduktes

0.45 g (1.6 mmol) 2-(3-Chloropropyl)-7-trifluormethyl-1,2,3,4-tetrahydroisochinolin, 0.36 g (1.6 mmol) 3-Mercapto-4-methyl-5-thien-3-yl-4H-1,2,4-triazol und 40mg Lithiumhydroxid wurden in 6 ml DMF 4 Stunden bei 100°C gerührt. Zur Aufarbeitung goß man auf Eis/Wasser, extrahierte mit Methyl-tert.Butylether, trocknete über Natriumsulfat und reinigte nach Filtration und Eindampfen säulenchromatographisch (Kieselgel, Laufmittel Dichlormethan mit 3-5% Methanol) auf.
Ausbeute: 0.3 g (0.7 mmol) 42% d.Th.

¹H-NMR (CDCl₃): δ = 2.1 (m, 2H); 2.7 (t, 2H); 2.8 (t, 2H); 3.0 (m, 2H); 3.35 (t, 2H); 3.7 (m, 5H); 7.1 (d, 1H); 7.2 (s,1H); 7.3 (d,1H); 7.5 (m, 2H); 7.7 (s, 1H); [freie Base].

Durch Behandlung mit etherischer HCl erhielt man die Titelverbindung
C₂₀H₂₁F₃N₄S₂·HCl (475)
Smp.: 192-194°C

### Beispiel 42

### 2-{3-[(4-Methyl-5-phenyl-4H-1,2,4-triazol-3-yl)sulfanyl]propyl}-8-(trifluoromethyl)-1,2,3,4-tetrahydroisochinolin Hydrochlorid

### Darstellung der Ausgangsmaterialien

### 42A 6/8-Trifluormethyl-1,2,3,4-tetrahydroisochinolin

5.3 g (18.6 mmol) N-Trifluoracetyl-2-(3-trifluorphenyl)ethylamin [hergestellt aus 2-(3-trifluorphenyl)ethylamin und Trifluoressigsäureanhydrid bei -5°C] und 0.9 g (29 mmol) Paraformaldehyd gab man zu einer Mischung von 22 ml Eisessig und 30 ml konzentrierter Schwefelsäure. Nach 18 Stunden bei Raumtemperatur gab man die Reaktionsmischung auf 350 ml Eiswasser, extrahierte mit Essigester, wusch die vereinigten organischen Phasen mit Natriumhydrogencarbonat-Lösung, dann mit Wasser. Nach Trocknen über Natriumsulfat, Filtration und Evaporation wurden 5.4 g eines Gemisches von 2-Trifluoracetyl-6-und-8-trifluormethyl-1,2,3,4-tetrahydroisoquinolin isoliert. Durch Erhitzen unter Rückfluß in Ethanol/3N HCl (1:1) wurde die Schutzgruppe abgespalten. Nach Aufarbeitung und chromatographischer Reinigung (Kieselgel, Laufmittel Dichlormethan mit 2-4% Methanol) wurden die beiden Isomere getrennt:
F1 1.2 g (5.7 mmol) 32% d.Th. 8-Trifluormethyl-1,2,3,4-tetrahydroisochinolin
   ¹H-NMR (CDCl₃): δ = 1.9 (sbr, 1H); 2.8 (t, 2H); 3.1 (t, 2H); 4.2 (s, 2H) 7.2 (m, 2H); 7.5 (d, 1H).
F2 1.4 g (6.8 mmol) 38% d.Th. 6-Trifluormethyl-1,2,3,4-tetrahydroisochinolin
   ¹H-NMR (CDCl₃): δ = 1.8 (sbr, 1H); 2.8 (t, 2H); 3.1 (t, 2H); 4.0 (s, 2H) 7.1 (d, 1H); 7.4 (m, 2H).
42 B 2-(3-Chloropropyl)-8-trifluormethyl-1,2,3,4-tetrahydroisochinolin
   2-(3-Chloropropyl)-8-trifluormethyl-1,2,3,4-tetrahydroisochinolin erhielt man durch Umsetzung von 42-A F1 mit Bromchlorpropan in analoger Weise wie unter Beispiel 4C beschrieben in 73% Ausbeute.

¹H-NMR (CDCl₃): δ = 2.0 (q, 2H); 2.7-2.8 (m, 4H); 3.0 (t, 2H); 3.6 (t, 2H); 3.8 (s, 2H); 7.2-7.3 (m, 2H); 7.4 (d, 1H).

### 42C Darstellung der Endverbindung

Umsetzung von 0.7 g (3.0 mmol) 3-Mercapto-4-methyl-5-phenyl-1,2,4(4H)-triazol mit 0.83 g (3.0 mmol) 2-(3-Chloropropyl)-8-trifluormethyl-1,2,3,4-tetrahydroisochinolin [42B1] in 10 ml DMF in Gegenwart von 70 mg Lithiumhydroxid bei 100°C ergab nach Aufarbeitung wie unter 4D beschrieben 0.84 g (1.9 mmol) der Endverbindung.
Ausbeute: 0.84 g (1.9 mmol) 65% d.Th.

### ¹H-NMR (CDCl₃): δ = 2.1 (q, 2H); 2.6-2.7 (m, 4H); 2.9 (t, 2H); 3.4 (t, 2H); 3.6 (s, 3H); 3.8 (s, 2H); 7.1 (t, 1H); 7.25 (d, 1H); 7.4 (d, 1H), 7.5 (m, 3H); 7.6 (m, 2H).

Durch Behandlung mit etherischer HCl erhielt man die Titelverbindung
C₂₂H₂₃F₃N₄S·HCl (469)
Smp.: 118°C

### Beispiel 43

### 2-{3-[(4-Methyl-5-phenyl-4H-1,2,4-triazol-3-yl)sulfanyl]propyl}-6-(trifluoromethyl)-1,2,3,4-tetrahydroisochinolin Hydrochlorid

### Darstellung der Ausgangsmaterialien

### 43 B2 2-(3-Chloropropyl)-6-trifluormethyl-1,2,3,4-tetrahydroisochinolin

2-(3-Chloropropyl)-6-trifluormethyl-1,2,3,4-tetrahydroisochinolin erhielt man durch Umsetzung von 6-Trifluormethyl-1,2,3,4-tetrahydroisochinolin [42AF2] (erhalten wie unter 42A beschrieben) mit Bromchlorpropan in analoger Weise wie unter 4C beschrieben in 96% Ausbeute.

¹H-NMR (CDCl₃): δ = 2.0 (m, 2H); 2.6-2.8 (m, 4H); 2.9 (t, 2H); 3.6 (m, 4H) 7.1 (d, 1H); 7.4 (m, 2H).

### 43C Darstellung der Endverbindung

Umsetzung von 0.7 g (3.0 mmol) 3-Mercapto-4-methyl-5-phenyl-1,2,4(4H)-triazol mit 0.83 g (3.0 mmol) 2-(3-Chloropropyl)-8-trifluormethyl-1,2,3,4-tetrahydroisochinolin in 10 ml DMF in Gegenwart von 70 mg Lithiumhydroxid bei 100°C ergab nach Aufarbeitung wie unter 4D beschrieben 0.75 g (1.7 mmol) der Endverbindung. Ausbeute: 0.75 g (1.7 mmol) 58% d.Th.

¹H-NMR (CDCl₃): δ = 2.1 (q, 2H); 2.6 (t, 2H); 2.7 (t, 2H); 2.9 (t, 2H); 3.3 (t,2H); 3.6 (s, 3H); 3.7 (s, 2H); 7.1 (d, 1H); 7.3 (m, 2H); 7.5 (m, 3H); 7.7 (m, 2H); [freie Base].

Durch Behandlung mit etherischer HCl erhielt man die Titelverbindung
C₂₂H₂₃F₃N₄S·HCl (469)
Smp.: 200-202°C

### Beispiel 44

### 2-{3-[(4-Methyl-5-phenyl-4H-1,2,4-triazol-3-yl)sulfanyl]propyl}-7-(trifluoromethyl)-1,2,3,4-tetrahydroisochinolin Hydrochlorid

C₂₂H₂₃F₃N₄S·HCl (469)
Smp.: 205-207°C

### Beispiel 45

### 2-{3-[(4-Methyl-5-(thien-3-yl)-4H-1,2,4-triazol-3-yl)sulfanyl]propyl}-7-(4-methyl-piperazin-1-ylsulfonyl)-1,2,3,4-tetrahydroisochinolin

¹H-NMR (CDCl₃): δ = 2.1 (q, 2H); 2.2 (s, 3H); 2.4 (m, 4H); 2.7 (t, 2H); 2.8 (t, 2H); 2.9 (t, 2H); 3.0 (m, 4H); 3.3 (t, 2H); 3.6 (m, 5H); 7.2 (d, 2H); 7.45 (m, 4H); 7.7 (m, 1H).
C₂₄H₃₂N₆O₂S₃ (538.8)

### Beispiel 46

### 2-{3-[(4-Methyl-5-(phenyl)-4H-1,2,4-triazol-3-yl)sulfanyl]propyl}-7-(4-methyl-piperazin-1-ylsulfonyl)-1,2,3,4-tetrahydroisochinolin

¹H-NMR (CDCl₃): δ = 2.1 (q, 2H); 2.2 (s, 3H); 2.5 (m, 4H); 2.7 (t, 2H); 2.8 (t, 2H); 2.9- 3.0 (m, 6H); 3.3 (t, 2H); 3.6 (s, 3H); 3.7 (s, 2H); 7.2 (d, 1H); 7.5 (m, 5H); 7.6 (m, 2H).
C₂₆H₃₄N₆O₂S₃ (564.8)

### Beispiel 47

### 2-{3-[(4-Methyl-5-(thien-3-yl)-4H-1,2,4-triazol-3-yl)sulfanyl]propyl}-7-(1,2,3,4-tetrahydroisochinolin 1-ylsulfonyl)-1,2,3,4-tetrahydroisochinolin

¹H-NMR (CDCl₃): δ = 2.1 (q, 2H); 2.7 (t, 2H); 2.8 (t, 2H); 2.9 (t, 2H); 3.2-3,3 (m, 4H); 3.6 (m, 2H); 3.7 (m, 5H); 4.2 (m, 2H); 7.1 (m, 4H); 7.2 (d, 1H); 7.4-7.6 (m, 4H); 7.7 (m, 1H).
C₂₈H₃₁N₅O₂S₃ (565)

### Beispiel 48

### 2-{3-[(4-Methyl-5-(pyrid-3-yl)-4H-1,2,4-triazol-3-yl)sulfanyl]propyl}-7-(1,2,3,4-tetrahydroisochinolin 1-ylsulfonyl)-1,2,3,4-tetrahydroisochinolin

¹H-NMR (CDCl₃) δ = 2.1 (q, 2H); 2.7 (t, 2H); 2.8 (t, 2H); 2.9 (m, 4H); 3.3 (m, 4H); 3.6 (s, 3H); 3.7 (s, 2H); 4.2 (s, 2H); 7.0-7.2 (m, 5H); 7.2 (m, 1H); 7.4-7.6 (m, 3H); 8.0 (m, 1H); 8.7 (m, 1H); 8.9 (m, 1H).
C₂₉H₃₂N₆O₂S₂ (558)

### Beispiel 49

### 7-[(3,3-dimethylpiperidin-1-yl)sulfonyl]-2-{3-[(4-methyl-5-phenyl-4H-1,2,4-triazol-3-yl)sulfanyl]propyl}-1,2,3,4-tetrahydroisochinolin

C₂₈H₃₇N₅O₂S₂ (539.8)
Smp.: 75-76°C

### Beispiel 50

### 2-{3-[(4-Cyclopropyl-5-phenyl-4H-1,2,4-triazol-3-yl)sulfanyl]propyl}-7-[(3,3-dimethylpiperidin-1-yl)sulfonyl]-1,2,3,4-tetrahydroisochinoline

C₃₀H₃₉N₅O₂S₂ (558)

### Beispiel 51

### 2-[(4-{[(4-Methyl-5-(1-methyl-1H-pyrrol-3-yl)-4H-1,2,4-triazol-3-yl)sulfanyl]methyl}cyclohexyl)methyl]- 7-nitro-1,2,3,4-tetrahydroisochinolin

C₂₆H₃₁N₅O₂S (477.6)
Smp.: 160°C

### Beispiel 52

### 2-{(E)-4-[(4-Methyl-5-pyridin-3-yl-4H-1,2,4-triazol-3-yl)sulfanyl]but-2-enyl}-7-nitro-1,2,3,4-tetrahydroisochinolin

C₂₁H₂₂N₆O₂S (422) MS: 423 [M+H]⁺

### Beispiel 53

### 2-[(4-{[(4-methyl-5-pyridin-3-yl-4H-1,2,4-triazol-3-yl)sulfanyl]-methyl}cyclohexyl)methyl]-1,2,3,4-tetrahydroisochinolin-7-carbonitril

C₂₇H₃₁N₅S (457.6)
Smp.: 156-158°C

### Beispiel 54

### 1-(2-{3-[(4-Methyl-5-(3-cyano)phenyl-4H-1,2,4-triazol-3-yl)sulfanyl]propyl}-1,2,3,4-tetrahydroisochinolin-7-yl)ethanon Hydrochlorid

C₂₄H₂₅N₅OS x HCl (468)
Smp.: 185°C

### Beispiel 55

### 7-Nitro-2-[(4-{[(4-methyl-5-pyridin-3-yl-4H-1,2,4-triazol-3-yl)-sulfanyl]-methyl}cyclo-hexyl)methyl]-1,2,3,4-tetrahydroisochinolin

C₂₆H₃₁N₆O₂S (477.6)
Smp.: 160°C

### Beispiel 56

### 1-{2-[3-({4-Methyl-5-phenyl]-4H-1,2,4-triazol-3-yl}sulfanyl)propyl]-1,2,3,4-tetrahydroisochinolin-7-yl}ethanon Hydochlorid

C₂₃H₂₇N₄OS x HCl (443)
Smp.: 165°C

### Beispiel 57

### 7,8-Dichloro-2-{3-[(4-methyl-5-phenyl-4H-1,2,4-triazol-3-yl)sulfanyl]propyl}-1,2,3,4-tetrahydroisochinolin

C₂₁H₂₂ClN₄S (399)
Smp.: 72-75°C

### Beispiel 58

### 1-{2-[3-({5-(2,4-Dinitrophenyl)-4-methyl]-4H-1,2,4-triazol-3-yl}-sulfanyl)propyl]-1,2,3,4-tetrahydroisochinolin-7-yl}ethanon Hydochlorid

C₂₃H₂₅N₆O₅S x HCl (500.6)
Smp.: 193°C

### Beispiel 59

### 2-{3-[(4-Methyl-5-phenyl-4H-1,2,4-triazol-3-yl)sulfanyl]propyl}-7-(octahydroisochinolin-2(1H)-ylsulfonyl)-1,2,3,4-tetrahydroisochinolin

C₃₀H₃₉N₅O₂S₂ (565.8) MS: 567 [M+H]⁺

### Beispiel 60

### 2-{3-[(4-Methyl-5- pyridin-3-yl -4H-1,2,4-triazol-3-yl)sulfanyl]propyl}-7-(octahydroisoguinolin-2(1H)-ylsulfonyl)-1,2,3,4-tetrahydroisochinolin

C₂₉H₃₈N₆O₂S₂ (566.8) MS: 568 [M+H]⁺

### Beispiel 61

### 2-{3-[(4-Cyclopropyl-5-phenyl-4H-1,2,4-triazol-3-yl)sulfanyl]propyl}-7-(azepan-1-ylsulfonyl)-1,2,3,4-tetrahydroisochinolin

C₂₉H₃₇N₅O₂S₂ (551.8) MS: 552 [M]⁺

### Beispiel 62

### 2-{3-[(4-Methyl-5-phenyl-4H-1,2,4-triazol-3-yl)sulfanyl]propyl}-7-(pyrrolidin-1-ylsulfonyl)-1,2,3,4-tetrahydroisochinolin

C₂₅H₃₁N₅O₂S₂ (497.7)

### Beispiel 63

### 2-{3-[(4-Methyl-5-phenyl-4H-1,2,4-triazol-3-yl)sulfanyl]propyl}-7-(azepan-1-ylsulfonyl)-1,2,3,4-tetrahydroisochinolin

C₂₇H₃₅N₅O₂S₂ (525.7)

### Beispiel 64

### 7-Chlor-2-(3-{[4-methyl-5-phenyl- -4H-1,2,4-triazol-3-yl]sulfanyl}but-2-en-yl)- 1,2,3,4-tetrahydroisochinolin

C₂₁H₂₃ClN₄S (399)
Smp.: 72-75°C

### Beispiel 65

### 2-(3-{[4-Methyl-5-methylamino-4H-1,2,4-triazol-3-yl]sulfanyl}propyl)-7-(azepan-1-ylsulfonyl)-1,2,3,4-tetrahydroisochinolin

### Beispiel 66

### N,4-Dimethyl-5-{[3-(7-(piperidin-1-ylsulfonyl)-3,4-dihydroisochinolin-2(1H)-yl)propyl]sulfanyl}-4H-1,2,4-triazol-3-amin

### Beispiel 67

### 7-tert-Butyl-2-(3-{[4-methyl-5-(4-methyl-1,3-thiazol-5-yl)-4H-1,2,4-triazol-3-yl]sulfanyl}propyl)-1,2,3,4-tetrahydroisochinolin

### Beispiel 68

### 2-{3-[(4-Methyl-5-pyridin-3-yl-4H-1,2,4-triazol-3-yl)sulfanyl]-propyl}-7-(azepan-1-ylsulfonyl)-1,2,3,4-tetrahydroisochinolin

### Beispiel 69

### 7-({4-[2-tert-Butyl-6-(trifluoromethyl)pyrimidin-4-yl]piperazin-1-yl}sulfonyl)-2-{3-[(4-methyl-5-phenyl-4H-1,2,4-triazol-3-yl)sulfanyl]propyl}-1,2,3,4-tetrahydroisochinolin

### Beispiel 70

### 8-Brom-2-(3-{[5-cyclohexyl-4-methyl-4H-1,2,4-triazol-3-yl]sulfanyl}but-2-en-yl)- 1,2,3,4-tetrahydroisochinolin

### Beispiel 71

### 4-Methyl-5-phenyl-N-[4-(7-(pyrrolidin-1-ylsulfonyl)-1,2,3,4-tetrahydroisochinolin-2-yl)butyl]-4H-1,2,4-triazole-3-carboxamid

### Beispiel 72

### 6-Methyl-2-(3-{[4-methyl-5-(1-methyl-1H-pyrrol-3-yl)-4H-1,2,4-triazol-3-yl]sulfanyl}-propyl)-7-(pyrrolidin-1-ylsulfonyl)-1,2,3,4-tetrahydroisochinolin

### Beispiel 73

### 7-Cyano-2-[(2-{[(4-Methyl-5-pyridin-3-yl-4H-1,2,4-triazol-3-yl)-sulfanyl]-methyl}-cyclopropyl)methyl]-1,2,3,4-tetrahydroisochinolin

### Beispiel 74

### 1-(2-{3-[(4-Methyl-5-(3-methoxy)phenyl-4H-1,2,4-triazol-3-yl)-oxy]propyl}-1,2,3,4-tetrahydro-isoquinolin-7-yl)ethanon

### Beispiel 75

### 4-(7-(Pyrrolidin-1-ylsulfonyl)-1,2,3,4-tetrahydroisochinolin-2-yl)butyl-4-methyl-5-phenyl-4H-1,2,4-triazole-3-carboxylat

### Beispiel 76

### 2-[2-({[5-(N-Methylpyrrol-2-yl)-4-methyl-4H-1,2,4-triazol-3-yl]-sulfanyl}methyl)prop-2-enyl]-1,2,3,4-tetrahydroisochinolin-7-carboxamide

### Beispiel 77

### 2-{3-[(4-Cyclopropyl-5-(4-methylsulfonyl)phenyl-4H-1,2,4-triazol-3-yl)sulfanyl]propyl}-7-(pyrrolidin-1-ylsulfonyl)-1,2,3,4-tetrahydroisochinolin

### Beispiel 78

### 6-tert-Butyl-2-(3-{[5-(2,4-dinitrophenyl)-4-methyl-4H-1,2,4-triazol-3-yl]sulfanyl}propyl)- 1,2,3,4-tetrahydroisochinolin

### Beispiel 79

### N-[2-(8-{[5-(dimethylamino)-4-butyl-4H-1,2,4-triazol-3-yl]sulfanyl}octyl)-1,2,3,4-tetrahydroisochinolin-7-yl]methansulfonamid

### Beispiel 80

### 2-{3-[(4-Methyl-5-pyrazin-2-yl-4H-1,2,4-triazol-3-yl)sulfanyl]propyl}-7-(octahydro-isoquinolin-2(1H)-ylsulfonyl)-1,2,3,4-tetrahydroisochinolin

### Beispiel 81

### 7-Cyano-2-{3-[(4-methyl-5-(2-methyloxazol-4-yl)-4H-1,2,4-triazol -3-yl)sulfanyl]propyl}-1,2,3,4-tetrahydroisochinolin

### Beispiel 82

### 2-{6-[(5-(2,5-Dimethylfuran-3-yl)-4-methyl-4H-1,2,4-triazol -3-yl)sulfanyl]hexyl}-7-trifluormethansulfonyloxy-1,2,3,4-tetrahydroisochinolin

### Beispiel 83

### 2-[2-({[4-methyl-5-phenyl-4H-1,2,4-triazol-3-yl]sulfanyl}methyl)prop-2-enyl]-7-nitro-1,2,3,4-tetrahydroisochinolin Hydrochlorid

C₂₂H₂₃N₅O₂S x HCl (460)
Smp.: 146-150°C

### Beispiel 84

### N-[2-(3-{[4-Methyl-5-phenyl-4H-1,2,4-triazol-3-yl]sulfanyl}propyl)-1,2,3,4-tetrahydroisochinolin-7-yl]methansulfonamid

C₂₂H₂₇N₅O₂S₂ x HCl (494.1)
Smp.: 90°C

In prinzipiell analoger Weise können hergestellt werden:

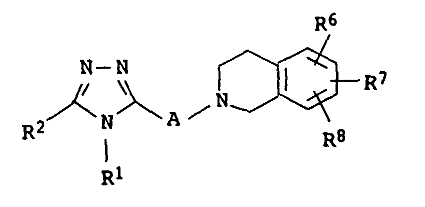

**Tabelle 1:**

| Bsp | R¹ | R² | A | R⁶ | R⁷ | R⁸ |
|---|---|---|---|---|---|---|
| 85 | Me | Ethoxycarbonyl | S-(CH₂)₃- | 7-(piperidin-1-yl-sulfonyl) | 8-methyl | |
| 86 | Me | N,N-Dimethylamino- | S-CH₂-CH=CH-CH₂- | 6-methyl | 7-cyano | |
| 87 | Et | tert.Butyl | (CH₂)₄- | 7-cyano | | |
| 88 | Butyl | Methylsulfanyl | (CH₂)₄- | 6-fluor | | |
| 89 | cycProp | Methyl | S-(CH₂)₃- | 6-chlor | 7-chlor | |
| 90 | Me | 2,5-Di-methyl-furanyl-3- | S-CH₂-CH=CH-CH₂- | 7-(piperidin-1-yl-sulfonyl) | | |
| *91 | Me | 3-Thienyl | COO-(CH₂)₃- | 7-(piperidin-1-yl-sulfonyl) | | |
| 92 | Me | Phenyl- | (CH₂)₄- | 7-(3,3-dimethyl-piperidin-1-yl-sulfonyl) | | |
| 93 | Me | 2,4-Dimethoxyphenyl | S-(CH₂)₃- | 7-methansulfonamid | | |
| 94 | Me | Amino- | S-CH₂-C(=CH₂)-CH₂ | 7-(piperidin-1-yl-sulfonyl) | | |
| 95 | Prop | Phenyl | S-CH₂-C(CH₃)=CH-CH₂- | 8-trifluormethyl | | |
| 96 | Me | 2-Me-4-Oxazolyl- | S-(CH₂)₃- | 7-(dimethylamino-sulfonyl) | | |
| 97 | Me | 3-Benzthienyl- | S-(CH₂)₆- | 7-(pyrolidin-1-yl-sulfonyl) | | |
| 98 | Me | Phenyl- | S-(CH₂)₇- | 7-(pyrolidin-1-yl-sulfonyl) | | |
| *99 | Me | Phenyl | CONH-(CH₂)₄- | 7-(piperidin-1-yl-sulfonyl) | | |
| 100 | Me | 2-Pyrazinyl- | S-(CH₂)₃- | 7-trifluormethyl | | |
| 101 | Phenyl | Methyl | (CH₂)₄- | 7-(morpholin-1-yl-sulfonyl) | | |
| 102 | Me | Tetrazolyl- | S-(CH₂)₃- | 7-methoxy | | |
| 103 | Et | 4-Methylthiazol-5-yl | S-(CH₂)₃- | 7-methylsulfonyl | | |
| 104 | Et | 3-Jod-phenyl | S-(CH₂)₃- | 7-methansulfonamid | | |
| 105 | Et | 4-Methylphenyl | S-CH₂-C(=CH₂)-CH₂ | 7-(piperidin-1-yl-sulfonyl) | | |
| 106 | Me | N-Methyl-2-Pyrrolyl- | S-(CH₂)₃- | 7-(dimethylamino-sulfonyl) | | |
| 107 | Me | 4-Methylthia-zol-5-yl | S-CH₂-C(=CH₂)-CH₂ | 7-(pyrrolidin-1-yl-sulfonyl) | | |
| 108 | Me | 2,5-Di-methyl-furanyl-3- | S-(CH₂)₃- | 7-phenylsulfonyl | | |
| *109 | Me | 2-Me-4-Oxazolyl- | (CH₂)₂-CH(CH₃)-CH₂-CH ₂- | 7-(morpholin-1-yl-sulfonyl) | | |
| 110 | Me | Phenyl- | S-(CH₂)₇- | 7-(pyrolidin-1-yl-sulfonyl) | | |
| 111 | Hexyl | 3-Pyridyl- | S-(CH₂)₃- | 6-chlor | 7-chlor | |
| 112 | Me | 3-Cyano-phenyl | S-(CH₂)₃- | 7-(dimethylamino-sulfonyl) | | |
| *113 | Me | 2-Pyrazinyl- | CO-(CH₂)₃- | 7-(morpholin-1-yl-sulfonyl) | | |
| 114 | Prop | Phenyl | S-(CH₂)₄- | 7-(morpholin-1-yl-sulfonyl) | | |
| 115 | Me | 3-Metoxyphenyl | (CH₂)₄- | 6-triflourmethyl | | |
| 116 | Me | 3-Pyrrolyl | S-(CH₂)₃- | 7-nitro | | |
| 117 | Et | 3-Pyridyl | S-(CH₂)₇- | 6-methyl | 7-cyano | |
| 118 | Me | 4-Methylthiazol-5-yl | O-(CH₂)₃- | 7-(piperidin-1-yl-sulfonyl) | | |
| *119 | Me | Phenyl | CONH-(CH₂)₄- | 7-cyano | | |
| 120 | Et | 2,5-Di-methyl-furanyl-3- | S-(CH₂)₃- | 7-nitro | | |
| 121 | Et | N-Methyl-2-Pyrrolyl- | S-(CH₂)₃- | 7-nitro | | |
| 122 | Prop | Phenyl- | S-(CH₂)₃- | 6-methyl | 7-(azepan-1-yl-sulfonyl) | |
| 123 | Et | N-Propyl-tetrazolyl- | S-(CH₂)₃- | 7-cyano | | |
| 124 | Me | 3-Thienyl | S-(CH₂)₃- | 7-methylsulfonyl | | |
| 125 | Me | 4-Methoxyphenyl | S-(CH₂)₃- | 4-methoxy | | |
| 126 | Me | Tetrazolyl- | S-(CH₂)₃- | 7-(dimethylamino-sulfonyl) | | |
| *127 | Me | 4-Methylthiazol-5-yl | S-CH₂-cycHex-CH₂-CH₂- | 7-phenylsulfonyl | | |
| *128 | Me | 2-Chlor-phenyl | CO-(CH₂)₃- | 7-trifluormethoxy | | |
| 129 | Et | Phenyl- | S-(CH₂)₃- | 6-CH₂-CH₂-CH₂-CH₂-7 | | |
| *130 | Et | 4-Methoxyphenyl | (CH₂)₂-CH(CH₃)-CH₂-CH ₂- | 7-(piperidin-1-yl-sulfonyl) | | |
| 131 | Et | 4-Methylthiazol-5-yl | S-CH₂-C(=CH₂)-CH₂ | 7-(azepan-1-yl-sulfonyl) | | |
| 132 | Me | 2-Me-4-Oxazolyl- | S-(CH₂)₆- | 7-nitro | | |
| 133 | Me | 5-Methyl imidazol-4-yl- | S-(CH₂)₃- | 7-(piperidin-1-yl-sulfonyl) | | |
| 134 | Me | 3-Jod-phenyl | S-(CH₂)₃- | 7-(dimethylamino-sulfonyl) | | |
| 135 | Me | Phenylmethyl | S-CH₂-CH=CH-CH₂- | 7-(azepan-1-yl-sulfonyl) | | |
| 136 | Et | Phenyl- | S-(CH₂)₃- | 6-CH(CH₃)CH₂-N(CH₃)-7 | | |
| 137 | Et | 3-Thienyl | S-(CH₂)₃- | 7-(dimethylamino-sulfonyl) | | |
| 138 | Me | 3-Jod-phenyl | S-(CH₂)₃- | 7-(piperidin-1-yl-sulfonyl) | | |
| 139 | Et | Phenyl | S-(CH₂)₃- | 8-trifluormethyl | | |
| *140 | Me | Phenyl | CONH-(CH₂)₅- | 8-trifluormethyl | | |
| 141 | Me | Phenyl- | S-CH₂-CH=CH-CH₂- | 7-(piperidin-1-yl-sulfonyl) | | |
| 142 | Me | Cyclohexyl- | S-(CH₂)₃- | 7-nitro | | |
| 143 | iProp | 3-Pyridyl | S-(CH₂)₇- | 7-chlor | 8-chlor | |
| 144 | Me | Amino- | S-(CH₂)₃- | 7-cyano | | |
| 145 | Me | 2-Aminothiazol-4yl- | S-(CH₂)₃- | 7-cyano | | |
| *146 | Me | 3-Pyrrolyl | S-CH₂-cycProp-CH₂- | 6-triflourmethyl | | |
| 147 | cycProp | Phenyl- | S-(CH₂)₃- | 6-CH₂-CH₂-CH₂-CH₂-7 | | |
| 148 | Me | 2-Pyrazinyl- | S-(CH₂)₃- | 7-(piperidin-1-yl-sulfonyl) | | |
| 149 | Me | Cyclohexyl- | S-(CH₂)₃- | 7-cyano | | |
| *150 | Me | 5-Methyl imidazol-4-yl- | (CH₂)₂-CH(CH₃)-CH₂-CH ₂- | tert-Butyl | | |
| 151 | Me | Methylamino- | S-(CH₂)₃- | 7-cyano | | |
| 152 | Me | 3-Benzthienyl- | S-(CH₂)₃- | 7-(dimethylamino-sulfonyl) | | |
| *153 | Me | Phenyl | S-CH₂-cycHex-CH₂-CH₂- | 5-methoxy | | |
| 154 | Me | Pyridin-4-yl- | S-(CH₂)₃- | 7-(piperidin-1-yl-sulfonyl) | | |
| 155 | Prop | Phenyl- | S-CH₂-C(=CH₂)-CH₂ | 7-(azepan-1-yl-sulfonyl) | | |
| 156 | Me | 3-Pyridinyl | S-(CH₂)₈- | 7-CHF₂ | | |
| 157 | Me | Tetrazolyl- | (CH₂)₄- | 7-(pyrolidin-1-yl-sulfonyl) | | |
| *158 | Me | 4-Phenyl | S-CH₂-cyc-Prop-(CH₂)₂- | 7-Brom | | |
| *159 | Me | 4-Methylphenyl | COO-(CH₂)₄- | 7-nitro | | |
| *160 | Et | 3-Cyano-phenyl | S-CH₂-cycHex-CH₂-CH₂- | 6-Methyl | | |
| 161 | Et | 2-Aminothiazol-4yl- | S-(CH₂)₃- | 7-(piperidin-1-yl-sulfonyl) | | |
| 162 | Et | Phenyl- | (CH₂)₄- | 7-(3,3-dimethyl-piperidin-1-yl-sulfonyl) | | |
| 163 | Me | 4-Methylthiazol-5-yl | S-(CH₂)₃- | 7-trifluormethyl | | |
| 164 | Me | Oxadiazol-2-yl | S-(CH₂)₃- | 7-(dimethylamino-sulfonyl) | | |
| 165 | Me | 6-Chlor-biphenyl-2- | S-(CH₂)₃- | 7-methylsulfonyl | | |
| 166 | Et | 3-Pyridinyl | S-(CH₂)₈- | 7-CHF₂ | | |
| 167 | Me | Pyridin-3-yl- | S-(CH₂)₃- | 7-methylsulfonyl | | |
| *168 | Me | Phenyl | CONH-(CH₂)₄- | 7-Phenylsulfonyl | | |
| 169 | Et | 2-Me-4-Oxazolyl- | S-(CH₂)₃- | 8-trifluormethyl | | |
| 170 | Me | 5-Methyl imidazol-4-yl- | S-(CH₂)₃- | 7-nitro | | |
| 171 | iProp | Phenyl | S-(CH₂)₃- | 6-Brom | | |
| 172 | Prop | 4-Imidazolyl- | S-(CH₂)₃- | 7-methoxy | | |
| 173 | Me | Tetrazolyl- | S-(CH₂)₃- | 7-cyano | | |
| *174 | Et | Phenyl | CONH-(CH₂)₄- | 6-chlor | 7-chlor | |
| 175 | Me | 2-Pyrazinyl- | S-(CH₂)₃- | 7-methoxy | | |
| 176 | Prop | Phenyl- | S-(CH₂)₃- | 6-methyl | 7-nitro | |
| *177 | Me | 4-Jod-phenyl | COO-(CH₂)₄- | 7-cyano | | |
| 178 | iProp | 4-Imidazolyl- | S-CH₂-CH=CH-CH₂- | 7-(azepan-1-yl-sulfonyl) | | |
| 179 | Et | 4-Methylsulfonylphenyl | S-(CH₂)₈- | 7-(piperidin-1-yl-sulfonyl) | | |
| 180 | Butyl | N-Propyl-tetrazolyl- | S-(CH₂)₃- | 7-cyano | | |
| 181 | Me | 2-Me-4-Oxazolyl- | S-CH₂-C(CH₃)=CH-CH₂- | 7-(azepan-1-yl-sulfonyl) | | |
| 182 | Et | 3-Pyrrolyl | S-(CH₂)₃- | 7-nitro | | |
| 183 | Me | N-Propyl-tetrazolyl- | S-CH₂-C(=CH₂)-CH₂ | 7-(piperidin-1-yl-sulfonyl) | | |
| *184 | Me | Propyl | CO-(CH₂)₃- | 5-methoxy | | |
| 185 | Me | 2-Pyrazinyl- | O-(CH₂)₃- | 7-(piperidin-1-yl-sulfonyl) | | |
| 186 | Me | Oxadiazol-2-yl | S-(CH₂)₃- | 7-nitro | | |
| 187 | Prop | 2-Me-4-Oxazolyl- | S-(CH₂)3- | 7-(piperidin-1-yl-sulfonyl) | | |
| 188 | Hexyl | Phenyl | (CH₂)₄- | 8-nitro | | |
| 189 | Prop | Phenyl | O-(CH₂)₃- | 7-methoxy | | |
| 190 | Me | 3-Pyridyl | S-(CH₂)₇- | 7-chlor | 8-chlor | |
| 191 | Et | Oxadiazol-2-yl | S-(CH₂)₃- | 7-nitro | | |
| 192 | Et | Phenyl- | S-(CH₂)₃- | 6-CH(CH₃)CH₂-NH-7 | | |
| 193 | Me | 3-Jod-phenyl | S-(CH₂)₃- | 7-methansulfonamid | | |
| 194 | Me | Pyridin-4-yl- | S-(CH₂)₃- | 7-nitro | | |
| 195 | Me | 4-Imidazolyl- | S-(CH₂)3- | 7-(dimethylamino-sulfonyl) | | |
| 196 | Me | Phenyl | (CH₂)₄- | 8-nitro | | |
| 197 | Me | 4-Methylphenyl | S-(CH₂)₃- | 7-(piperidin-1-yl-sulfonyl) | | |
| 198 | cycProp | Phenyl | S-(CH₂)₃- | 7-Carboxamid | | |
| 199 | Me | 3-Jod-phenyl | O-(CH₂)₃- | 7-(piperidin-1-yl-sulfonyl) | | |
| 200 | Me | Cyclohexyl- | S-(CH₂)₆- | 7-(piperidin-1-yl-sulfonyl) | | |
| 201 | Me | 3-Jod-phenyl | S-CH₂-C(CH₃)=CH-CH₂- | 7-(piperidin-1-yl-sulfonyl) | | |
| 202 | Me | 3-Jod-phenyl | S-(CH2)3- | 7-phenylsulfonyl | | |
| 203 | Butyl | Pyridin-3-yl- | O-(CH₂)₃- | 7-(piperidin-1-yl-sulfonyl) | | |
| 204 | cycProp | 2,4-Dimethoxyphenyl | S-(CH₂)₃- | 7-methansulfonamid | | |
| 205 | Me | N-Propyl-tetrazolyl- | S-(CH₂)₃- | 7-cyano | | |
| 206 | Et | 4-Methoxyphenyl | S-CH₂-CH=CH-CH₂- | 7-(piperidin-1-yl-sulfonyl) | | |
| 207 | Et | Phenyl- | S-(CH₂)₃- | 6-methyl | 7-nitro | |
| *208 | Et | Phenyl- | (CH₂)₂-CH(CH₃)-CH₂-CH ₂- | 6-methoxy | | |
| 209 | Me | 3-Br-Pyridin-5-yl- | S-(CH₂)₃- | 7-nitro | | |
| *210 | Me | Methylamino- | S-CH₂-cycHex-CH₂-CH₂- | 7-cyano | | |
| *211 | Et | tert.-Butyl | CO-(CH₂)₃- | 6-methoxy | | |
| 212 | Me | Phenyl | S-(CH₂)₃- | 6-Fluor | | |
| 213 | Me | Phenylmethyl | S-(CH₂)₃- | 7-(piperidin-1-yl-sulfonyl) | | |
| 214 | iProp | 4-Methoxyphenyl | S-CH₂-CH=CH-CH₂- | 7-(piperidin-1-yl-sulfonyl) | | |
| 215 | iProp | 4-Cyano-phenyl | S-CH₂-C(CH₃)=CH-CH₂- | 7-(piperidin-1-yl-sulfonyl) | | |
| 216 | Me | 3-Br-Pyridin-5-yl- | S-(CH₂)₃- | 7-(dimethylamino-sulfonyl) | | |
| 217 | Me | Phenyl- | S-(CH₂)₃- | 6-CH₂-CH₂-CH₂-CH₂-7 | | |
| 218 | Me | 3-Cyano-phenyl | S-(CH₂)₃- | 7-(piperidin-1-yl-sulfonyl) | | |
| 219 | Me | 3-Thienyl | S-(CH₂)₃- | 7-(dimethylamino-sulfonyl) | | |
| 220 | Et | Phenyl | (CH₂)₄- | 8-nitro | | |
| 221 | Me | Amino | S-(CH₂)₃- | 7-nitro | | |
| 222 | Me | 4-Methylsulfonylphenyl | S-(CH₂)₈- | 7-(piperidin-1-yl-sulfonyl) | | |
| 223 | Me | 4-Methylsulfonylphenyl | S-(CH₂)₃- | 7-(dimethylamino-sulfonyl) | | |
| 224 | Me | 4-Methylthiazol-5-yl | S-(CH₂)₃- | 7-methoxy | | |
| 225 | Me | 2-Me-4-Oxazolyl- | S-(CH₂)₃- | 7-methylsulfonyl | | |
| 226 | Me | 2,5-Di-methyl-furanyl-3- | S-(CH₂)₃- | 7-methoxy | | |
| 227 | Me | 3-Pyrrolyl | S-(CH₂)₃- | 7-cyano | | |
| 228 | Phenyl | Cyano | S-(CH₂)₃- | 7-(pyrrolidin-1-yl-sulfonyl) | | |
| 229 | Me | Tetrazolyl- | O-(CH₂)₃- | 7-(piperidin-1-yl-sulfonyl) | | |
| 230 | Me | Phenyl- | S-(CH₂)₃- | 6-methyl | 7-cyano | |
| 231 | Et | Carboxamido | S-(CH₂)₃- | 7-cyano | | |
| 232 | Me | Pyridin-3-yl- | S-CH₂-C(CH₃)=CH-CH₂- | 7-(azepan-1-yl-sulfonyl) | | |
| 233 | Et | Phenyl | S-(CH₂)₃- | 6-Brom | | |
| 234 | Prop | 2-Aminothiazol-4yl- | S-(C-H₂)₃- | 7-(piperidin-1-yl-sulfonyl) | | |
| 235 | Me | Pyridin-4-yl- | S-(CH₂)₃- | 7-(dimethylamino-sulfonyl) | | |
| 236 | Me | 4-Methylthiazol-5-yl | S-(CH₂)₃- | 7-cyano | | |
| 237 | cycProp | Phenyl- | S-(CH₂)₃- | 6-CH₂-CH₂-CH₂-7 | | |
| 238 | Me | Pyridin-3-yl- | S-CH₂-C(=CH₂)-CH₂ | 7-(azepan-1-yl-sulfonyl) | | |
| 239 | Et | 5-Methyl imidazol-4-yl- | S-(CH₂)₁₀- | 7-(piperidin-1-yl-sulfonyl) | | |
| 240 | Me | Methylamino | S-(CH₂)₃- | 7-nitro | | |
| 241 | Me | Pyridin-4-yl- | S-(CH₂)₆- | 7-(piperidin-1-yl-sulfonyl) | | |
| 242 | Butyl | Phenyl- | S-(CH₂)₃- | 6-methyl | 7-cyano | |
| 243 | Phenyl | 3-Pyridyl- | S-(CH₂)₆- | 7-(piperidin-1-yl-sulfonyl) | | |
| 244 | Me | Tetrazolyl- | O-(CH₂)₃- | 7-cyano | | |
| 245 | Hexyl | 3-Jodphenyl- | S-(CH₂)₃- | 6-chlor | 7-chlor | |
| *246 | Me | 4-Methylsulfonylphenyl | S-CH₂-cycProp-CH₂- | 7-cyano | | |
| 247 | Phenyl | tert-Butyl | S-(CH₂)₃- | 7-(pyrrolidin-1-yl-sulfonyl) | | |
| 248 | Me | tert.-Butyl | (CH₂)₄- | 6-methoxy | | |
| *249 | cycProp | tert.-Butyl | CO-(CH₂)₃- | 6-methoxy | | |
| 250 | Me | Amino- | S-(CH₂)₃- | 7-methylsulfonyl | | |
| 251 | Me | Amino- | S-(CH₂)₃- | 6-methoxy | | |
| 252 | Et | N-Methyl-2-Pyrrolyl- | S-(CH₂)8- | 7-cyano | | |
| 253 | Me | Methylamino- | S-(CH₂)₃- | 7-methoxy | | |
| 254 | Me | Phenyl | S-(CH₂)₃- | 8-ethenyl | | |
| 255 | Et | Phenyl | S-CH₂-cycHex-CH₂- | 7-trifluormethoxy | | |
| *256 | Me | N-Methyl-2-Pyrrolyl- | S-CH₂-cycProp-CH₂- | 8-triflourmethyl | | |
| 257 | Prop | 3-Jod-phenyl | S-(CH₂)₃- | 7-methansulfonamid | | |
| 258 | Me | Methylamino- | S-(CH₂)₃- | 7-trifluormethyl | | |
| 259 | Me | Tetrazolyl- | S-CH₂-cycHex-CH₂- | 7-(morpholin-1-yl-sulfonyl) | | |
| 260 | Me | Methylamino- | S-(CH₂)₃- | 7-(piperidin-1-yl-sulfonyl) | | |
| 261 | Me | N-Methyl-2-Pyrrolyl- | S-(CH₂)₃- | 7-trifluormethyl | | |
| 262 | Me | 2-Aminothiazol-4yl- | S-(CH₂)₃- | 7-(dimethylamino-sulfonyl) | | |
| 263 | Me | 3-Pyrrolyl | S-(CH₂)₃- | 7-methylsulfonyl | | |
| 264 | Me | 4-Imidazolyl- | S-CH₂-CH=CH-CH₂- | 7-(azepan-1-yl-sulfonyl) | | |
| 265 | Me | Propyl | (CH₂)₄- | 5-methoxy | | |
| 266 | Me | Oxadiazol-2-yl | S-(CH₂)₃- | 6-trifluormethyl | | |
| 267 | Me | 4-Methylphenyl | O-(CH₂)₃- | 7-cyano | | |
| 268 | cycProp | Phenyl | (CH₂)₄- | 8-nitro | | |
| 269 | Me | 3-Br-Pyridin-5-yl- | S-(CH₂)₃- | 7-(piperidin-1-yl-sulfonyl) | | |
| 270 | iProp | Phenyl | S-(CH₂)₃- | 7-Acetyl | | |
| 271 | Me | 4-Methylsulfonylphenyl | S-(CH₂)₈- | 7-(piperidin-1-yl-sulfonyl) | | |
| 272 | Me | 3-Cyano-phenyl | S-(CH₂)₃- | 7-nitro | | |
| 273 | Me | 4-Methylthiazol-5-yl | S-(CH₂)₃- | 7-methansulfonamid | | |
| 274 | Me | 3-Cyano-phenyl | S-(CH₂)₃- | 7-cyano | | |
| 275 | Me | Oxadiazol-2-yl | S-(CH₂)₃- | 7-cyano | | |
| 276 | Me | Phenyl- | S-(CH₂)₇- | 6-methyl | 7-(pyrolidin-1-yl-sulfonyl) | |
| *277 | Me | Phenyl- | CO-(CH₂)₃- | 7-cyano | | |
| 278 | cycProp | 4-Methoxyphenyl | (CH₂)₄- | 8-ethenyl | | |
| 279 | Me | Phenyl | S-CH₂-C(CH₃)=CH-CH₂- | 7-(piperidin-1-yl-sulfonyl) | | |
| 280 | Me | 6-Chlor-biphenyl-2- | S-(CH₂)₃- | 7-nitro | | |
| 281 | Me | 4-Imidazolyl- | S-(CH₂)₃- | 8-trifluormethyl | | |
| 282 | Me | 3-Br-Pyridin-5-yl- | S-(CH₂)₃- | 7-cyano | | |
| 283 | Pentyl | 3-Pyridyl- | S-(CH₂)₃- | 6-chlor | 7-chlor | |
| 284 | Me | Pyridin-3-yl- | O-(CH₂)₃- | 7-(piperidin-1-yl-sulfonyl) | | |
| 285 | Me | 3-Pyrrolyl | S-(CH₂)₃- | 7-methoxy | | |
| 286 | Me | 2-Pyrazinyl- | O-(CH₂)₃- | 7-cyano | | |
| *287 | Et | Phenyl- | CO-(CH₂)₃- | 7-cyano | | |
| 288 | Me | 2-Me-4-Oxazolyl- | S-(CH₂)₃- | 7-(piperidin-1-yl-sulfonyl) | | |
| 289 | Me | 4-Methylsulfonylphenyl | S-(CH₂)₃- | 7-methylsulfonyl | | |
| *290 | Me | Phenyl | COO-(CH₂)₄- | 7-(piperidin-1-yl-sulfonyl) | | |
| 291 | Me | Oxadiazol-2-yl | S-(CH₂)₃- | 7-methylsulfonyl | | |
| 292 | Me | 2-Aminothiazol-4yl- | S-(CH₂)₃- | 7-methoxy | | |
| *293 | Me | 4-Methylphenyl | CONH-(CH₂)₄- | 7-cyano | | |
| 294 | Me | 3-Pyrrolyl | S-CH₂-CH=CH-CH₂- | 7-(piperidin-1-yl-sulfonyl) | | |
| 295 | Me | 3-Cyano-phenyl | S-(CH₂)₃- | 7-methansulfonamid | | |
| *296 | Me | 2-Pyrazinyl- | S-CH₂-cyc-Prop-(CH₂)₂- | 7-(pyrolidin-1-yl-sulfonyl) | | |
| 297 | Me | Pyridin-3-yl- | S-CH₂-C(=CH₂)-CH₂ | 7-(piperidin-1-yl-sulfonyl) | | |
| 298 | Me | 2-Me-4-Oxazolyl- | S-(CH₂)₃- | 7-(piperidin-1-yl-sulfonyl) | | |
| 299 | Et | 3-Br-Pyridin-5-yl- | S-(CH₂)₃- | 7-cyano | | |
| 300 | Me | 6-Chlor-biphenyl-2- | S-(CH₂)₃- | 7-trifluormethyl | | |
| 301 | iProp | Phenyl- | S-(CH₂)₇- | 6-methyl | 7-(pyrolidin-1-yl-sulfonyl) | |
| 302 | Me | 3-Benzthienyl- | S-(CH₂)₃- | 7-nitro | | |
| *303 | Me | Phenyl | CONH-(CH₂)₄- | 7-nitro | | |
| 304 | Me | Cyclohexyl- | S-(CH₂)₆- | 7-(piperidin-1-yl-sulfonyl) | | |
| 305 | Me | 3-Pyrrolyl | S-CH₂-CH=CH-CH₂- | 6-chlor | | |
| 306 | Et | 2-Pyrazinyl- | O-(CH₂)₃- | 7-(piperidin-1-yl-sulfonyl) | | |
| 307 | Me | 4-Imidazolyl- | S-(CH₂)₃- | 7-(piperidin-1-yl-sulfonyl) | | |
| 308 | Me | 3-Pyridinyl | S-(CH₂)₈- | 7-CHF₂ | | |
| *309 | Me | 3-Pyridyl | COO-(CH₂)₃- | 7-cyano | | |
| 310 | Me | 3-Benzthienyl- | S-(CH₂)₃- | 7-cyano | | |
| 311 | Me | 3-Pyrrolyl | S-(CH₂)₃- | 7-(piperidin-1-yl-sulfonyl) | | |
| *312 | Me | 4-Methoxyphenyl | (CH₂)₂-CH(CH₃)-CH₂-CH 2- | 5-hydroxy | | |
| 313 | Me | Amino- | S-(CH₂)₃- | 7-trifluormethyl | | |
| *314 | Me | 4-Methylthiazol-5-yl | S-CH₂-cycProp-CH₂- | 7-(piperidin-1-yl-sulfonyl) | | |
| 315 | Me | Tetrazolyl- | S-(CH₂)₃- | 7-phenylsulfonyl | | |
| 316 | Me | Phenyl | S-CH₂-cycHex-CH₂- | 7-trifluormethoxy | | |
| 317 | Phenyl | 3-Thienyl | S-(CH₂)₃- | 7-nitro | | |
| 318 | Me | Pyridin-3-yl- | S-(CH₂)₃- | 7-(dimethylamino-sulfonyl) | | |
| 319 | Me | 4-Methylphenyl | S-CH₂-C(=CH₂)-CH₂ | 7-(piperidin-1-yl-sulfonyl) | | |
| 320 | Prop | 3-Benzthienyl- | S-(CH₂)₃- | 7-(dimethylamino-sulfonyl) | | |
| 321 | Me | 4-Methylthiazol-5-yl | S-(CH₂)₃- | 7-methylsulfonyl | | |
| 322 | Me | 4-Methoxyl-phenyl | S-(CH₂)₈- | 7-(piperidin-1-yl-sulfonyl) | | |
| 323 | Me | Oxadiazol-2-yl | S-(CH₂)₇- | 7-azepan-1-yl-sulfonyl) | | |
| *324 | Me | Methylamino- | S-CH₂-cycProp-CH₂- | 7-(piperidin-1-yl-sulfonyl) | | |
| 325 | Me | 4-methoxyphenyl | S-(CH₂)₃- | 7-cyano | | |
| 326 | Butyl | 2-Aminothiazol-4yl- | S-(CH₂)₃- | 7-(piperidin-1-yl-sulfonyl) | | |
| 327 | iProp | 3-Pyrrolyl | S-CH₂-CH=CH-CH₂- | 7-(piperidin-1-yl-sulfonyl) | | |
| *328 | Me | Phenyl | CONH-(CH₂)₄- | 7-chlor | | |
| 329 | Butyl | Phenyl- | S-(CH₂)₃- | 7-(piperidin-1-yl-sulfonyl) | 8-chlor | |
| 330 | Et | 4-Imidazolyl- | S-(CH₂)₃- | 7-methoxy | | |
| *331 | Me | Phenyl | S-CH₂-cycProp-CH₂- | 6-methoxy | | |
| * 332 | Me | 3-Furanyl | S-CH₂-cycProp-CH₂- | 7-(N-methylanilin-1-sulfonyl) | | |
| 333 | Me | 2-Pyrazinyl- | S-(CH₂)₃- | 7-cyano | | |
| 334 | cycProp | 2-Pyrazinyl- | O-(CH₂)₃- | 7-(piperidin-1-yl-sulfonyl) | | |
| 335 | Et | Phenyl | S-(CH₂)₄- | 7-(morpholin-1-yl-sulfonyl) | | |
| 336 | Me | Phenyl- | S-(CH₂)₃- | 7-methylsulfonyl | | |
| 337 | Me | 4-Methylphenyl | O-(CH₂)₃- | 7-(piperidin-1-yl-sulfonyl) | | |
| 338 | Butyl | Phenyl | S-(CH₂)₃- | 7-acetyl | | |
| 339 | Et | 4-Cyano-phenyl | S-CH₂-C(CH₃)=CH-CH₂- | 7-(piperidin-1-yl-sulfonyl) | | |
| 340 | Butyl | Phenyl- | S-(CH₂)₃- | 6-Methyl | 7-(pyrolidin-1-yl-sulfonyl) | |
| 341 | Butyl | Phenyl | S-(CH₂)₃- | 8-chlor | | |
| 342 | Et | Pyridin-3-yl- | O-(CH₂)₃- | 7-(piperidin-1-yl-sulfonyl) | | |
| 343 | Me | 3-Thienyl | S-(CH₂)₃- | 7-methoxy | | |
| 344 | Me | N-Methyl-2-Pyrrolyl- | S-CH₂-cycHex-CH₂-CH₂- | 5-methoxy | | |
| 345 | Me | 4-Imidazolyl- | S-(CH₂)₃- | 7-methoxy | | |
| *346 | cycProp | Phenyl | CONH-(CH₂)₅- | 8-trifluormethyl | | |
| 347 | Me | 6-Chlor-biphenyl-2- | S-(CH₂)₃- | 7-(dimethylamino-sulfonyl) | | |
| 348 | Et | 3-Pyridyl | S-(CH₂)₇- | 7-chlor | 8-chlor | |
| 349 | Me | 4-Methylsulfonylphenyl | S-CH₂-cycHex-CH₂- | 6-methoxy | | |
| 350 | Me | Methylamino- | S-(CH₂)₃- | 7-methylsulfonyl | | |
| 351 | Et | 2-Me-4-Oxazolyl- | S-(CH₂)₃- | 7-methoxy | | |
| 352 | Et | Phenyl- | S-(CH₂)₃- | 6-CH₂-CH₂-CH₂-7 | | |
| 353 | Et | Phenyl | S-(CH₂)₄- | 7-(pyrrolidin-1-yl-sulfonyl) | | |
| 354 | Butyl | 2-Pyrazinyl- | O-(CH₂)₃- | 7-(piperidin-1-yl-sulfonyl) | | |
| 355 | Me | 4-Methoxyl-phenyl | S-(CH₂)₈- | 7-(piperidin-1-yl-sulfonyl) | | |
| *356 | Me | Phenyl- | (CH₂)₂-CH(CH₃)-CH₂-CH ₂- | 6-methoxy | | |
| 357 | Me | 2-Aminothiazol-4yl- | S-(CH₂)₃- | 7-trifluormethyl | | |
| 358 | Prop | Phenyl | S-(CH₂)₃- | 7-Acetyl | | |
| *359 | Me | 4-Methylphenyl | COO-(CH₂)₃- | 7-(piperidin-1-yl-sulfonyl) | | |
| *360 | Et | 2-Me-4-Oxazolyl- | (CH₂)₂-CH(CH₃)-CH₂-CH ₂- | 7-(morpholin-1-yl-sulfonyl) | | |
| 361 | Butyl | Carboxamido | S-(CH₂)₃- | 7-cyano | | |
| 362 | Me | Pyridin-4-yl- | S-(CH₂)₃- | 6-trifluormethyl | | |
| 363 | Hexyl | 3-Pyridyl- | S-(CH₂)₃- | 7-chlor | 8-chlor | |
| 364 | Me | N-Propyl-tetrazolyl- | S-(CH₂)₃- | 7-methylsulfonyl | | |
| 365 | Et | Phenyl- | S-CH₂-C(=CH₂)-CH₂ | 7-(azepan-1-yl-sulfonyl) | | |
| 366 | cycProp | Phenyl- | (CH₂)₄- | 7-(3,3-dimethyl-piperidin-1-yl-sulfonyl) | | |
| *367 | Me | Phenyl | CONH-(CH₂)₄- | 6-chlor | 7-chlor | |
| 368 | Et | 4-Imidazolyl- | S-CH₂-CH=CH-CH₂- | 7-(azepan-1-yl-sulfonyl) | | |
| 369 | Me | Cyclohexyl- | S-(CH₂)₃- | 7-methoxy | | |
| 370 | Me | 2-Pyrazinyl- | S-(CH₂)₃- | 7-(dimethylamino-sulfonyl) | | |
| 371 | Prop | 2-Me-4-Oxazolyl- | S-(CH₂)₃- | 8-trifluormethyl | | |
| 372 | Me | 2,4-Dimethoxyphenyl | S-CH₂-C(CH₃)=CH-CH₂- | 7-(piperidin-1-yl-sulfonyl) | | |
| 373 | Me | Cyclohexyl- | S-(CH₂)₃- | 7-(dimethylamino-sulfonyl) | | |
| 374 | Me | Pyridin-3-yl- | S-(CH₂)₃- | 7-methoxy | | |
| 375 | Me | Phenyl- | S-(CH₂)₃- | 7-methoxy | | |
| 376 | Me | 2-Pyrazinyl- | S-CH₂-cycHex-CH₂- | 7-(morpholin-1-yl-sulfonyl) | | |
| 377 | Me | N-Propyl-tetrazolyl- | S-(CH₂)₃- | 7-nitro | | |
| 378 | Me | Phenyl- | (CH₂)₄- | 8-triflourmethyl | | |
| 379 | Prop | 4-Methoxyphenyl | (CH₂)₄- | 8-ethenyl | | |
| 380 | Me | Phenyl- | S-(CH₂)₇- | 7-(pyrolidin-1-yl-sulfonyl) | | |
| 381 | iProp | 4-Methylthiazol-5-yl | S-(CH₂)₃- | 7-methylsulfonyl | | |
| 382 | iProp | Phenyl- | S-(CH₂)₇- | 7-(piperidin-1-yl-sulfonyl) | 8-chlor | |
| 383 | iProp | Phenyl | S-(CH₂)₃- | 7-carboxamid | | |
| 384 | Me | Phenyl | S-CH₂-C(CH₃)=CH-CH₂- | 7-trifluormethyl | | |
| *385 | Et | Phenyl | CONH-(CH₂)₅- | 8-trifluormethyl | | |
| 386 | iProp | 3-Pyrrolyl | S-(CH₂)₆- | 7-cyano | | |
| 387 | Me | Phenyl- | S-(CH₂)₇- | 7-(piperidin-1-yl-sulfonyl) | 8-chlor | |
| 388 | Et | 3-Benzthienyl- | S-(CH₂)₃- | 7-(dimethylamino-sulfonyl) | | |
| 389 | Me | 2-Me-4-Oxazolyl- | S-(CH₂)₃- | 7-methoxy | | |
| 390 | Me | 2-Aminothiazol-4yl- | S-(CH₂)₃- | 7-nitro | | |
| 391 | Prop | 3-Br-Pyridin-5-yl- | S-(CH₂)₃- | 7-cyano | | |
| 392 | Me | 3-Thienyl | S-(CH₂)₃- | 7-nitro | | |
| *393 | Et | Phenyl | CONH-(CH₂)₄- | 7-chlor | | |
| 394 | Me | 4-Methylthiazol-5-yl | S-(CH₂)₃- | 7-nitro | | |
| 395 | Me | 2-Me-4-Oxazolyl- | S-(CH₂)₃- | 7-(dimethylamino-sulfonyl) | | |
| 396 | Me | 6-Chlor-biphenyl-2- | S-(CH₂)₃- | 7-cyano | | |
| 397 | Me | Tetrazolyl- | S-(CH₂)₃- | 7-(piperidin-1-yl-sulfonyl) | | |
| 398 | Me | 3-Benzthienyl- | S-(CH₂)₃- | 7-methylsulfonyl | | |
| 399 | Me | 3-Thienyl | S-CH₂-CH=CH-CH₂- | 7-(pyrrolidin-1-yl-sulfonyl) | | |
| 400 | Hexyl | Phenyl- | S-(CH₂)₃- | 6-methyl | 7-cyano | |
| 401 | Me | 3-Pyridyl | S-(CH₂)₇- | 6-methyl | 7-cyano | |
| 402 | Me | 2-Me-4-Oxazolyl- | S-(CH₂)₃- | 7-methylsulfonyl | | |
| 403 | Me | 3-Thienyl | O-(CH₂)₃- | 7-cyano | | |
| 404 | Prop | Phenyl- | S-(CH₂)₃- | 6-methyl | 7-(piperidin-1-yl-sulfonyl) | |
| 405 | Et | 2,4-Dimethoxyphenyl | S-(CH₂)₃- | 7-methansulfonamid | | |
| 406 | Me | Phenyl- | S-(CH₂)₃- | 7-trifluormethyl | | |
| *407 | Me | 4-Methoxyphenyl | (CH₂)₂-CH(CH₃)-CH₂-CH₂- | 7-(piperidin-1-yl-sulfonyl) | | |
| *408 | Me | Phenyl | S-CH₂-cyc-Prop-(CH₂)₂- | 5-methoxy | | |
| 409 | Phenyl | 3-Thienyl | (CH₂)₄- | 7-(piperidin-1-yl-sulfonyl) | | |
| 410 | Me | 3-Thienyl | S-(CH₂)₃- | 7-methansulfonamid | | |
| 411 | Me | Pyridin-3-yl- | S-(CH₂)₃- | 7-trifluormethyl | | |
| 412 | Phenyl | tert-Butyl | O-(CH₂)₃- | 7-(pyrrolidin-1-yl-sulfonyl) | | |
| 413 | Me | 3-Pyrrolyl | S-(CH₂)₃- | 7-trifluormethyl | | |
| 414 | Me | N-Methyl-2-Pyrrolyl- | S-(CH₂)₃- | 7-nitro | | |
| 415 | iProp | Phenyl | S-(CH₂)₃- | 8-trifluormethyl | | |
| 416 | Butyl | 3-Thienyl | S-(CH₂)₈- | 7-(pyrrolidin-1-yl-sulfonyl) | | |
| 417 | Me | Phenyl- | S-CH₂-C(=CH₂)-CH₂ | 7-(piperidin-1-yl-sulfonyl) | | |
| 418 | Me | 2-Me-4-Oxazolyl- | S-(CH₂)₃- | 7-nitro | | |
| 419 | Me | 2-Aminothiazol-4yl- | S-(CH₂)₃- | 7-phenylsulfonyl | | |
| 420 | Me | 4-Methylthiazol-5-yl | O-(CH₂)₃- | 7-cyano | | |
| 421 | Me | 4-Methylsulfonylphenyl | S-(CH₂)₃- | 7-trifluormethyl | | |
| 422 | Me | 4-methylsulfonylphenyl | O-(CH₂)₃- | 7-(piperidin-1-yl-sulfonyl) | | |
| 423 | Butyl | 3-Pyridyl- | S-(CH₂)₃- | 7-chlor | 8-chlor | |
| 424 | Me | Methylamino- | S-CH₂-C(CH₃)=CH-CH₂- | 7-(piperidin-1-yl-sulfonyl) | | |
| 425 | Me | Carboxamido | S-(CH₂)₃- | 7-cyano | | |
| 426 | Me | 4-Methoxyphenyl | S-(CH₂)₃- | 7-phenylsulfonyl | | |
| 427 | Et | 3-Pyrrolyl | S-CH₂-CH=CH-CH₂- | 7-(piperidin-1-yl-sulfonyl) | | |
| 428 | Me | 3-Cyano-phenyl | S-(CH₂)₃- | 7-trifluormethyl | | |
| 429 | Me | 5-Methyl imidazol-4-yl- | S-(CH₂)₃- | 7-cyano | | |
| 430 | Prop | N-Propyl-tetrazolyl- | S-(CH₂)₃- | 7-cyano | | |
| 431 | Me | 2,5-Di-methyl-furanyl-3- | S-(CH₂)₃- | 7-(dimethylamino-sulfonyl) | | |
| 432 | Prop | Pyridin-3-yl- | S-CH₂-C(=CH₂)-CH₂ | 7-(azepan-1-yl-sulfonyl) | | |
| 433 | Me | 4-Methylsulfonylphenyl | S-(CH₂)₃- | 7-(piperidin-1-yl-sulfonyl) | | |
| 434 | Butyl | Phenyl | (CH₂)₄- | 8-nitro | | |
| *435 | Me | 4-Methylphenyl | COO-(CH₂)₄- | 7-(piperidin-1-yl-sulfonyl) | | |
| *436 | Me | 3-Furanyl | S-CH₂-cycHex-CH₂-CH₂- | 7-phenylsulfonyl | | |
| 437 | Me | 3-Jod-phenyl | S-(CH₂)₃- | 7-trifluormethyl | | |
| 438 | Et | 2-Pyrazinyl- | O-(CH₂)₃- | 8-ethenyl | | |
| 439 | Me | 3-Benzthienyl- | S-(CH₂)₃- | 7-(piperidin-1-yl-sulfonyl) | | |
| 440 | Me | Cyclohexyl- | S-(CH₂)₃- | 7-(piperidin-1-yl-sulfonyl) | | |
| 441 | Me | Pyridin-3-yl- | S-CH₂-cycHex-CH₂- | 6-methoxy | | |
| 442 | Me | 2-Me-4-Oxazolyl- | S-(CH₂)₃- | 7-nitro | | |
| 443 | Me | 2-Pyrazinyl- | (CH₂)₄- | 7-(morpholin-1-yl-sulfonyl) | | |
| 444 | Prop | 2-Pyrazinyl- | S-(CH₂)₃- | 8-ethenyl | | |
| 445 | Me | 4-Methoxyphenyl | S-(CH₂)₃- | 7-trifluormethyl | | |
| 446 | Me | 4-Imidazolyl- | S-(CH₂)₃- | 7-methylsulfonyl | | |
| 447 | Me | Phenyl- | S-(CH₂)₇- | 7-(pyrolidin-1-yl-sulfonyl) | | |
| 448 | Me | Cyclohexyl- | S-(CH₂)₃- | 7-trifluormethyl | | |
| 449 | Butyl | Phenyl- | (CH₂)₄- | 7-(3,3-dimethyl-piperidin-1-yl-sulfonyl) | | |
| 450 | Et | Phenyl | S-(CH₂)₃- | 8-ethenyl | | |
| 451 | Me | 4-Methoxyphenyl nyl) | S-(CH₂)₃- | 7-(piperidin-1-yl-sulfo- | | |
| 452 | iProp | Phenyl | S-(CH₂)₄- | 7-(morpholin-1-yl-sulfonyl) | | |
| 453 | Me | Cyano | S-(CH₂)₈- | 6,7-dimethoxy | | |
| 454 | Me | 2-Aminothiazol-4yl- | S-CH₂-CH=CH-CH₂- | 7-(piperidin-1-yl-sulfonyl) | | |
| *455 | Et | Phenyl | COO-(CH₂)₃- | 7-(piperidin-1-yl-sulfonyl) | | |
| 456 | Me | 3-Cyano-phenyl | S-(CH₂)₃- | 7-methylsulfonyl | | |
| 457 | Me | 2-Pyrazinyl- | S-(CH₂)₃- | 7-nitro | | |
| 458 | Me | 3-Cyano-phenyl | S-CH₂-C(=CH₂)-CH₂ | 7-(piperidin-1-yl-sulfonyl) | | |
| 459 | cycProp | N-Methyl-2-Pyrrolyl- | S-(CH₂)₈- | 7-cyano | | |
| 460 | Me | 4-Methoxyphenyl | S-(CH₂)₃- | 7-nitro | | |
| 461 | Me | Oxadiazol-2-yl | S-(CH₂)₃- | 7-(piperidin-1-yl-sulfonyl) | | |
| 462 | Me | Tetrazolyl- | S-(CH₂)₇- | 7-(piperidin-1-yl-sulfonyl) | | |
| 463 | Butyl | Phenyl | (CH₂)₄- | 7-(pyrrolidin-1-yl-sulfonyl) | | |
| 464 | Prop | 4-Methylphenyl | S-CH₂-C(=CH₂)-CH₂ | 7-(piperidin-1-yl-sulfonyl) | | |
| 465 | Me | Phenyl- | S-(CH₂)₃- | 6-CH₂-CH₂-CH₂-7 | | |
| 466 | Me | N-Methyl-2-Pyrrolyl- | S-(CH₂)₃- | 7-methylsulfonyl | | |
| 467 | Me | 3-Thienyl | S-(CH₂)₃- | 7-trifluormethyl | | |
| 468 | Et | Cyano | S-(CH₂)₈- | 6-methoxy | 7-methoxy | |
| 469 | cycProp | Phenyl- | S-(CH₂)₃- | 6-CH(CH₃)CH₂-NH-7 | | |
| 470 | Me | 3-Br-Pyridin-5-yl- | S-(CH₂)₃- | 7-methylsulfonyl | | |
| 471 | Me | Phenyl- | S-(CH₂)₃- | 6-CH(CH₃)CH₂-N(CH₃)-7 | | |
| 472 | Et | 4-Methoxyphenyl | (CH₂)₄- | 8-ethenyl | | |
| 473 | Me | Tetrazolyl- | S-(CH₂)₃- | 7-trifluormethyl | | |
| 474 | Me | 6-Chlor-biphenyl-2- | S-(CH₂)₃- | 7-methoxy | | |
| 475 | Me | 4-Pyridyl- | (CH₂)₄- | 7-(pyrolidin-1-yl-sulfonyl) | | |
| *476 | cycProp | Phenyl | CONH-(CH₂)₄- | 6-chlor | 7-chlor | |
| 477 | Me | 2-Me-4-Oxazolyl- | S-(CH₂)₆- | 7-nitro | | |
| 478 | Me | 2-Me-4-Oxazolyl- | S-(CH₂)₃- | 7-methoxy | | |
| 479 | Me | 4-Methoxyphenyl | S-CH₂-CH=CH-CH₂- | 7-(piperidin-1-yl-sulfonyl) | | |
| 480 | Me | Cyano | S-(CH₂)₈- | 6-methoxy | 7-methoxy | |
| *481 | Me | tert.-Butyl | CO-(CH₂)₃- | 6-methoxy | | |
| 482 | Et | 3-Cyano-phenyl | S-(CH₂)₃- | 7-methansulfonamid | | |
| 483 | Prop | Cyano | S-(CH₂)₈- | 6-methoxy | 7-methoxy | |
| *484 | Me | 3-Pyrrolyl | S-CH₂-cycHex-CH₂-CH₂- | 7-cyano | | |
| 485 | Me | Methylamino- | S-(CH2)₃- | 7-(dimethylamino-sulfonyl) | | |
| 486 | Me | 2,5-Di-methyl-furanyl-3- | S-(CH₂)₃- | 7-(piperidin-1-yl-sulfonyl) | | |
| 487 | Me | 2,5-Di-methyl-furanyl-3- | S-(CH₂)₃- | 7-nitro | | |
| 488 | iProp | 4-Methoxyphenyl | (CH₂)₄- | 8-ethenyl | | |
| 489 | Et | Tetrazolyl- | S-(CH₂)₃- | 7-nitro | | |
| *490 | Me | Phenyl | COO-(CH₂)₃- | 7-(piperidin-1-yl-sulfonyl) | | |
| 491 | Me | 4-Imidazolyl- | S-(CH₂)₃- | 7-nitro | | |
| 492 | Me | 3-Thienyl | O-(CH₂)₃- | 7-(piperidin-1-yl-sulfonyl) | | |
| 493 | Et | Phenyl | S-CH₂-C(CH₃)=CH-CH₂- | 8-trifluormethyl | | |
| 494 | Me | Pyridin-4-yl- | S-(CH₂)₆- | 7-nitro | | |
| 495 | Me | N-Methyl-2-Pyrrolyl- | S-(CH₂)₃- | 7-methansulfonamid | | |
| 496 | Et | Phenyl- | S-(CH₂)₃- | 6-methyl | 7-cyano | |
| 497 | Prop | 4-Methylthiazol-5-yl | S-(CH₂)₃- | 7-trifluormethyl | | |
| 498 | Me | Phenyl | O-(CH₂)₃- | 7-(piperidin-1-yl-sulfonyl) | | |
| 499 | Me | 4-Cyano-phenyl | S-CH₂-C(CH₃)=CH-CH₂- | 7-(piperidin-1-yl-sulfonyl) | | |
| 500 | Et | Phenyl | S-(CH₂)₃- | 7-Carboxamid | | |
| 501 | Me | N-Propyl-tetrazolyl- | S-(CH₂)₃- | 7-(piperidin-1-yl-sulfonyl) | | |
| 502 | Me | Amino- | S-(CH₂)₃- | 7-(dimethylaminosulfonyl) | | |
| 503 | Me | 2,4-Dimethoxyphenyl | S-(CH₂)₃- | 7-(piperidin-1-yl-sulfonyl) | | |
| *504 | Me | 3-Benzthienyl- | CO-(CH₂)₃- | 7-phenylsulfonyl | | |
| 505 | Me | 4-Imidazolyl- | S-(CH₂)₃- | 7-cyano | | |
| *506 | Et | Phenyl | S-CH₂-cycHex-CH₂-CH₂- | 5-methoxy | | |
| 507 | Et | 3-Pyrrolyl | S-(CH₂)₆- | 7-cyano | | |
| 508 | Me | 3-Pyrrolyl | S-(CH₂)₃- | 7-methansulfonamid | | |
| 509 | Me | Tetrazolyl- | S-(CH₂)₇- | 7-(piperidin-1-yl-sulfonyl) | | |
| 510 | Me | Tetrazolyl- | S-(CH₂)₃- | 7-methansulfonamid | | |
| *511 | Me | 3-Thienyl | COO-(CH₂)₄- | 7-(piperidin-1-yl-sulfonyl) | | |
| 512 | Et | 2-Me-4-Oxazolyl- | S-(CH₂)₃- | 7-(piperidin-1-yl-sulfonyl) | | |
| 513 | Me | Pyridin-4-yl- | S-(CH₂)₃- | 7-methylsulfonyl | | |
| 514 | Butyl | N-Methyl-2-Pyrrolyl- | S-(CH₂)₈- | 7-cyano | | |
| 515 | Me | Phenyl- | S-(CH₂)₃- | 6-CH(CH3)CH2-NH-7 | | |
| 516 | Me | Pyridin-4-yl- | S-(CH₂)₃- | 7-cyano | | |
| *517 | Me | 3-Thienyl | S-CH₂-cyc-Prop-(CH₂)₂- | 7-(3,3-dimethyl-piperidin-1-yl-sulfonyl) | | |
| 518 | Me | 2,4-Dimethoxyphenyl | O-(CH₂)₃- | 7-cyano | | |
| 519 | Me | 4-Methylsulfonylphenyl | O-(CH₂)₃- | 7-cyano | | |
| 520 | Me | 4-Methylthiazol-5-yl | S-CH₂-C(=CH₂)-CH₂ | 7-(azepan-1-yl-sulfonyl) | | |
| 521 | Me | Amino- | S-(CH₂)₃- | 7-(piperidin-1-yl-sulfonyl) | | |
| 522 | Prop | N-Methyl-2-Pyrrolyl- | S-(CH₂)₈- | 7-cyano | | |
| 523 | Me | 5-Methyl imidazol-4-yl- | S-(CH₂)₃- | 7-trifluormethyl | | |
| 524 | Me | Cyclohexyl- | S-(CH₂)₃- | 7-methylsulfonyl | | |
| 525 | Et | Pyridin-3-yl- | S-CH₂-C(=CH₂)-CH₂ | 7-(azepan-1-yl-sulfonyl) | | |
| 526 | Prop | Phenyl | S-(CH₂)₃- | 8-ethenyl | | |
| 527 | Me | 5-Methyl imidazol-4-yl- | S-CH₂-C(CH₃)=CH-CH₂- | 7-(pyrrolidin-1-yl-sulfonyl) | | |
| *528 | Me | Tetrazolyl- | S-CH₂-cycProp-CH₂- | 6-methoxy | | |
| 529 | Me | Phenyl- | S-CH₂-C(=CH₂)-CH₂ | 7-(azepan-1-yl-sulfonyl) | | |
| 530 | Me | 6-Chlor-biphenyl-2- | S-(CH₂)₃- | 7-(piperidin-1-yl-sulfonyl) | | |
| 531 | Et | Phenyl- | S-(CH₂)₇- | 6-methyl | 7-(pyrolidin-1-yl-sulfonyl) | |
| 532 | Me | Pyridin-3-yl- | S-(CH₂)₃- | 7-methansulfonamid | | |
| 533 | Me | 2-Pyrazinyl- | S-CH₂-C(=CH₂)-CH₂ | 7-(piperidin-1-yl-sulfonyl) | | |
| 534 | Et | 3-Jod-phenyl | O-(CH₂)₃- | 7-cyano | | |
| 535 | Me | 3-Benzthienyl- | S-(CH₂)₃- | 6-methoxy | | |
| 536 | Me | Oxadiazol-2-yl | S-(CH₂)₃- | 7-methoxy | | |
| 537 | Me | 6-Chlor-biphenyl-2- | S-CH₂-C(CH₃)=CH-CH₂- | 7-(piperidin-1-yl-sulfonyl) | | |
| 538 | cycProp | 4-Methylthiazol-5-yl | S-CH₂-C(=CH₂)-CH₂ | 7-(azepan-1-yl-sulfonyl) | | |
| 539 | Me | Pyridin-3-yl- | S-(CH₂)₃- | 7-(piperidin-1-yl-sulfonyl) | | |
| 540 | Et | 4-Methylthiazol-5-yl | S-(CH₂)₃- | 7-trifluormethyl | | |
| 541 | Me | 3-Pyrrolyl | S-(CH₂)₃- | 7-(dimethylamino-sulfonyl) | | |
| *542 | Me | 3-Pyridyl | COO-(CH₂)₄- | 7-(piperidin-1-yl-sulfonyl) | | |
| 543 | Prop | Carboxamido | S-(CH₂)₃- | 7-cyano | | |
| *544 | Me | 4-Jod-phenyl | COO-(CH₂)₃- | 7-cyano | | |
| 545 | Hexyl | Phenyl- | (CH₂)₄- | 7-(3,3-dimethyl-piperidin-1-yl-sulfonyl) | | |
| 546 | Me | 2-Me-4-Oxazolyl- | S-(CH₂)₃- | 7-trifluormethyl | | |
| 547 | Et | Phenyl- | S-(CH₂)₇- | 7-(piperidin-1-yl-sulfonyl) | 8-chlor | |
| 548 | Prop | Phenyl | S-(CH₂)₄- | 7-(pyrrolidin-1-yl-sulfonyl) | | |
| 549 | Me | N-Propyl-tetrazolyl- | S-(CH₂)₃- | 7-methoxy | | |
| 550 | Me | 2-Pyrazinyl- | S-(CH₂)₃- | 7-methylsulfonyl | | |
| 551 | Me | Phenyl | S-CH₂-C(CH₃)=CH-CH₂- | 8-trifluormethyl | | |
| *552 | Butyl | tert.-Butyl | CO-(CH₂)₃- | 6-methoxy | | |
| 553 | Prop | 5-Methyl imidazol-4-yl- | S-(CH₂)₁₀- | 7-(piperidin-1-yl-sulfonyl) | | |
| 554 | Me | 4-Jod-phenyl | S-(CH₂)₃- | 7-cyano | | |
| 555 | Me | 5-Methyl imidazol-4-yl- | S-(CH₂)₃- | 7-(dimethylamino-sulfonyl) | | |
| 556 | Me | 3-Benzthienyl- | (CH₂)₄- | 7-phenylsulfonyl | | |
| 557 | Me | Pyridin-3-yl- | O-(CH₂)₃- | 7-cyano | | |
| 558 | Me | Tetrazolyl- | S-(CH₂)₃- | 7-nitro | | |
| 559 | Me | 3-Benzthienyl- | S-(CH₂)₆- | 7-(pyrolidin-1-yl-sulfonyl) | | |
| 560 | cycProp | Phenyl | S-(CH₂)₃- | 7-Acetyl | | |
| 561 | iProp | Phenyl | S-(CH₂)₄- | 7-(pyrrolidin-1-yl-sulfonyl) | | |
| 562 | Me | Phenyl- | S-(CH₂)₃- | 7-(piperidin-1-yl-sulfonyl) | | |
| 563 | Me | 2-Me-4-Oxazolyl- | S-(CH₂)₃- | 7-cyano | | |
| 564 | Me | 5-Methyl imidazol-4-yl- | S-(CH₂)₃- | 7-methoxy | | |
| 565 | Prop | Phenyl- | S-(CH₂)₃- | 6-CH(CH₃)CH₂-N(CH₃)-7 | | |
| 566 | Me | N-Propyl-tetrazolyl- | S-(CH₂)₃- | 7-trifluormethyl | | |
| 567 | Me | 2,5-Di-methyl-furanyl-3- | S-(CH₂)₃- | 7-trifluormethyl | | |
| 568 | Me | Phenyl | O-(CH₂)₃- | 7-cyano | | |
| 569 | Me | 4-Jod-phenyl | S-(CH₂)₃- | 7-nitro | | |
| 570 | Me | N-Methyl-2-Pyrrolyl- | S-(CH₂)₃- | 7-cyano | | |
| 571 | Prop | 3-Pyridyl | S-(CH₂)₇- | 6-methyl | 7-cyano | |
| 572 | Me | 2,5-Di-methyl-furanyl-3- | S-(CH₂)₃- | 7-cyano | | |
| 573 | Me | 2-Pyrazinyl- | S-(CH₂)₃- | 7-methansulfonamid | | |
| 574 | Me | 2-Me-4-Oxazolyl- | S-(CH₂)₃- | 7-cyano | | |
| 575 | Et | Phenyl | O-(CH₂)₃- | 7-cyano | | |
| 576 | Me | Methylamino- | S-(CH₂)₃- | 7-methansulfonamid | | |
| 577 | Me | 3-Thienyl | S-(CH₂)₃- | 7-cyano | | |
| 578 | Me | 2-Chlor-phenyl | (CH₂)₄- | 7-trifluormethoxy | | |
| 579 | Butyl | 3-Pyrrolyl | S-CH₂-CH=CH-CH₂- | 7-(piperidin-1-yl-sulfonyl) | | |
| 580 | cycProp | 3-Cyano-phenyl | S-(CH₂)₃- | 7-methansulfonamid | | |
| 581 | Me | N-Propyl-tetrazolyl- | S-(CH₂)₃- | 7-(dimethylamino-sulfonyl) | | |
| *582 | Me | 4-Methylphenyl | COO-(CH₂)₄- | 7-trifluormethyl | | |
| 583 | Me | 5-Methyl imidazol-4-yl- | S-(CH₂)₃- | 7-methylsulfonyl | | |
| 584 | Me | 3-Br-Pyridin-5-yl- | S-(CH₂)₃- | 7-methoxy | | |
| 585 | Me | 3-Thienyl | S-CH₂-cycHex-CH₂- | 7-trifluormethoxy | | |
| 586 | Me | Pyridin-3-yl- | S-(CH₂)₃- | 7-nitro | | |
| *587 | Et | 3-Thienyl | COO-(CH₂)₃- | 7-(piperidin-1-yl-sulfonyl) | | |
| 588 | Prop | 3-Thienyl | S-(CH₂)₃- | 7-(dimethylaminosulfonyl) | | |
| 589 | Butyl | 3-Br-Pyridin-5-yl- | S-(CH₂)₃- | 7-cyano | | |
| 590 | Me | Pyridin-3-yl- | S-(CH₂)₃- | 7-cyano | | |
| 591 | Et | 3-Cyano-phenyl | S-(CH₂)₃- | 7-nitro | | |
| 592 | Prop | Phenyl | S-(CH₂)₁₀- | 7-Carboxamid | | |
| *593 | Et | 3-Furanyl | S-CH₂-cycHex-CH₂-CH₂- | 7-phenylsulfonyl | | |
| 594 | Me | N-Methyl-2-Pyrrolyl- | S-(CH₂)₃- | 7-methoxy | | |
| *595 | Me | 3-Cyano-phenyl | S-CH₂-cycHex-CH₂-CH₂- | 6-Methyl | | |
| 596 | Me | 4-Methylsulfonylphenyl | S-(CH₂)₃- | 7-methansulfonamid | | |
| 597 | Me | 2-Aminothiazol-4yl- | S-(CH₂)₃- | 7-(piperidin-1-yl-sulfonyl) | | |
| 598 | Prop | Phenyl | S-(CH₂)₃- | 6-Brom | | |
| 599 | Prop | 4-Methylthiazol-5-yl | S-(CH₂)₃- | 7-methylsulfonyl | | |
| 600 | Me | 2,4-Dimethoxyphenyl | O-(CH₂)₃- | 7-(piperidin-1-yl-sulfonyl) | | |
| 601 | Et | Pyridin-4-yl- | S-(CH₂)₃- | 7-nitro | | |
| 602 | Me | N-Methyl-2-Pyrrolyl- | S-CH₂-C(CH₃)=CH-CH₂- | 7-(piperidin-1-yl-sulfonyl) | | |
| 603 | Me | 3-Br-Pyridin-5-yl- | S-(CH₂)₆- | 7-(piperidin-1-yl-sulfonyl) | | |
| 604 | iProp | Phenyl- | S-(CH₂)₃- | 6-methyl | 7-cyano | |
| *605 | Et | 2-Pyrazinyl- | CO-(CH₂)₃- | 7-(morpholin-1-yl-sulfonyl) | | |
| 606 | Me | Phenyl- | S-(CH₂)₃- | 6-methyl | 7-nitro | |
| 607 | Butyl | 4-Methylthiazol-5-yl | S-CH₂-C(=CH₂)-CH₂ | 7-(azepan-1-yl-sulfonyl) | | |
| 608 | Me | 4-Methoxyphenyl | S-(CH₂)₃- | 7-(dimethylamino-sulfonyl) | | |
| 609 | Me | 3-Br-Pyridin-5-yl- | S-(CH₂)₆- | 7-(piperidin-1-yl-sulfonyl) | | |
| 610 | Me | Pyridin-4-yl- | S-(CH₂)₃- | 7-methoxy | | |
| 611 | cycProp | Pyridin-3-yl- | O-(CH₂)₃- | 7-(piperidin-1-yl-sulfonyl) | | |
| 612 | Me | 3-Br-Pyridin-5-yl- | S-(CH₂)₃- | 7-trifluormethyl | | |
| 613 | Me | 2-Me-4-Oxazolyl- | S-(CH₂)₃- | 8-trifluormethyl | | |
| 614 | Prop | Phenyl | S-(CH₂)₃- | 8-trifluormethyl | | |
| 615 | Me | 3-Benzthienyl- | S-(CH₂)₃- | 7-trifluormethyl | | |
| 616 | Et | Phenyl | S-(CH₂)₃- | 7-acetyl | | |
| *617 | Me | Pyridin-3-yl- | S-CH₂-cycProp-CH₂- | 7-(pyrolidin-1-yl-sulfonyl) | | |
| 618 | Me | Oxadiazol-2-yl | S-(CH₂)₇- | 7-(piperidin-1-yl-sulfonyl) | | |
| 619 | Phenyl | 3-Thienyl | S-(CH₂)₃- | 7-cyano | | |
| 620 | Me | 3-Jod-phenyl | O-(CH₂)₃- | 7-cyano | | |
| *621 | Me | Phenyl | CONH-(CH₂)₄- | 6-methoxy | 8-methyl | |
| 622 | Me | 3-Thienyl | S-(CH₂)₃- | 6-CH(CH₃)CH₂-NH-7 | | 5-methyl |
| 623 | Me | 3-Thienyl | S-(CH₂)₃- | 6-CH₂-CH₂-CH₂-CH₂-7 | | 8-brom |
| 624 | Me | 4-Pyridyl | S-(CH₂)₃- | 6-CH₂-CH₂-CH₂-7 | | 8-ethenyl |
| 625 | Me | 3-Pyridyl- | S-(CH₂)₃- | 5-methoxy | 7-chlor | 8-chlor |
| 626 | Me | 3-Phenyl- | O-(CH₂)₃- | 6-chlor | 7-chlor | 8-methyl |

| | | | | | | |
|---|---|---|---|---|---|---|
| R⁷ und R⁸ stehen, soweit in dieser Tabelle für sie keine Bedeutung angegeben ist, für Wasserstoff. In dieser und der folgenden Tabelle werden die folgenden Abkürzungen verwendet: Me = Methyl Et = Ethyl cycProp = Cyclopropyl Prop = n-Propyl iProp = Isopropyl cycHex = Cyclohexyl * = Referenz-Beispiel | | | | | | |

In prinzipiell analoger Weise können hergestellt werden:

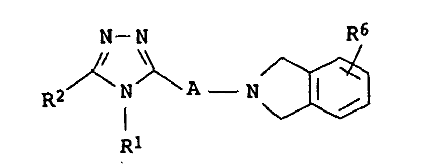

**Tabelle 2**

| Bsp | R¹ | R² | A | R⁶ |
|---|---|---|---|---|
| *627 | Me | Phenyl | CONH-(CH₂)4- | 5-nitro |
| 628 | Butyl | Methylamino | S-(CH₂)₃- | 5-fluor |
| 629 | Me | Oxadiazol-2-yl | S-(CH₂)₇- | 5-(piperidin-1-yl-sulfonyl) |
| 630 | Me | Tetrazolyl- | S-(CH₂)₇- | 5-(piperidin-1-yl-sulfonyl) |
| 631 | Me | 3-Cyano-phenyl | S-(CH₂)₃- | 5-fluor |
| 632 | Et | 3-Thienyl | S-(CH₂)₃- | 5-methoxy |
| 633 | Me | Carboxamid | S-CH₂-C(CH₃)=CH-CH₂- | 5-methoxy |
| *634 | Butyl | Cyclohexyl- | S-CH₂-cycProp-(CH2)₂- | 5-chor |
| 635 | Me | 3-Pyrrolyl | S-(CH₂)₃- | 5-nitro |
| 636 | Me | 2-Pyrazinyl- | S-(CH₂)₃- | 5-nitro |
| *637 | Pentyl | tert.-Butyl | CO-(CH₂)₃- | 6-methoxy |
| *638 | Me | Pyridin-3-yl- | CO-(CH₂)₃- | 5-fluor |
| 639 | Me | 4-Jod-phenyl | S-(CH₂)₃- | 5-fluor |
| 640 | Me | 4-Methylsulfonyl-phenyl | S-(CH₂)₈- | 5-(piperidin-1-yl-sulfonyl) |
| 641 | iProp | N-Propyl-tetrazolyl- | S-(CH₂)₃- | 5-fluor |
| *642 | cycProp | tert.-Butyl | CO-(CH₂)₃- | 6-methoxy |
| 643 | Me | 2-Aminothiazol-4yl- | S-(CH₂)₃- | 5-fluor |
| 644 | cycProp | 4-Methylsulfonyl-phenyl | S-(CH₂)₈- | 5-(piperidin-1-yl-sulfonyl) |
| 645 | Me | Pyridin-3-yl- | S-CH₂-CH=CH-CH₂- | 5-methoxy |
| 646 | Me | N-Propyl-tetrazolyl- | S-CH₂-CH=CH-CH₂- | 5-nitro |
| 647 | cycProp | Carboxamido | S-(CH₂)₃- | 5-fluor |
| 648 | Me | N-Methyl-2-Pyrrolyl- | S-(CH₂)₃- | 5-nitro |
| 649 | Me | 2-Me-4-Oxazolyl- | S-(CH₂)₃- | 5-nitro |
| 650 | Me | Pyridin-4-yl- | S-(CH₂)₃- | 5-nitro |
| 651 | Me | 3-Br-Pyridin-5-yl- | S-CH₂-CH=CH-CH₂- | 5-methoxy |
| 652 | Me | Phenyl- | S-(CH₂)₃- | 5-fluor |
| 653 | Me | 4-Jod-phenyl | S-(CH₂)₃- | 5-methoxy |
| 654 | Me | 3-Pyrrolyl | S-(CH₂)₃- | 5-methoxy |
| 655 | Me | Phenyl- | S-CH₂-CH=CH-CH₂- | 5-(piperidin-1-yl-sulfonyl) |
| 656 | Me | 3-Cyano-phenyl | S-(CH₂)₃- | 5-methoxy |
| 657 | Me | N-Methyl-2-Pyrrolyl- | S-CH₂-cycProp-(CH₂)₂- | 5-methoxy |
| 658 | Me | Phenyl | O-(CH₂)₃- | 5-cyano |
| 659 | Pentyl | Cyclohexyl- | S-(CH₂)₃- | 5-chlor |
| *660 | Me | 3-Benzthienyl- | S-CH₂-cycProp-(CH₂)₂- | 5-fluor |
| 661 | Pentyl | Carboxamido | S-(CH₂)₃- | 5-chlor |
| 662 | Et | 5-Methyl imidazol-4-yl- | S-CH₂-CH=CH-CH₂- | 5-methoxy |
| 663 | iProp | Cyclohexyl- | S-(CH₂)₃- | 5-fluor |
| 664 | Me | 3-Benzthienyl- | S-(CH₂)₃- | 5-nitro |
| *665 | Butyl | Cyclohexyl- | S-CH₂-cycProp-(CH₂)₂- | 5-methoxy |
| 666 | Me | 4-Methoxyphenyl | S-(CH₂)₃- | 5-methoxy |
| 667 | Prop | N-Propyl-tetrazolyl- | S-CH₂-CH=CH-CH₂- | 5-fluor |
| *668 | Pentyl | Phenyl | CONH-(CH₂)₄- | 5-cyano |
| *669 | Me | Phenyl- | CO-(CH₂)₃- | 5-methoxy |
| 670 | Prop | Cyclohexyl- | S-(CH₂)₃- | 5-fluor |
| 671 | Butyl | Methylamino | S-(CH₂)₃- | 5-methoxy |
| 672 | Me | 4-Methylthiazol-5-yl | S-(CH₂)₃- | 5-cyano |
| 673 | cycProp | N-Propyl-tetrazolyl- | S-CH₂-CH=CH-CH₂- | 5-nitro |
| *674 | cycProp | Propyl | CO-(CH₂)₃- | 5-methoxy |
| 675 | Me | Oxadiazol-2-yl | S-CH₂-CH=CH-CH₂- | 5-nitro |
| 676 | Me | 3-Pyridyl | S-(CH₂)₇- | 5-chlor |
| 677 | Me | 5-Methyl imidazol-4-yl- | S-(CH₂)₃- | 5-fluor |
| 678 | Me | 5-Methyl imidazol-4-yl- | S-CH₂-C(CH₃)=CH-CH₂- | 5-(pyrrolidin-1-yl-sulfonyl) |
| 679 | Me | 3-Pyrrolyl | S-(CH₂)₃- | 5-fluor |
| 680 | Me | Cyclohexyl- | O-(CH₂)₃- | 5-nitro |
| 681 | Me | Methylamino- | S-CH₂-C(CH₃)=CH-CH₂- | 5-(piperidin-1-yl-sulfonyl) |
| 682 | iProp | 6-Chlor-biphenyl-2- | S-(CH₂)₃- | 5-fluor |
| 683 | Me | 3-Cyano-phenyl | S-(CH₂)₃- | 5-nitro |
| 684 | Pentyl | N-Propyl-tetrazolyl- | S-CH₂-CH=CH-CH₂- | 5-chlor |
| *685 | Me | Phenyl | CONH-(CH₂)₄- | 5-cyano |
| *686 | cycProp | Phenyl | COO-(CH₂)₃- | 5-(piperidin-1-yl-sulfonyl) |
| 687 | Me | Amino | S-(CH₂)₃- | 5-nitro |
| *688 | Me | Phenyl | CONH-(CH₂)₄- | 5-chlor |
| 689 | Me | 2-Pyrazinyl- | S-(CH₂)₃- | 5-fluor |
| 690 | Me | 4-Jod-phenyl | S-CH₂-CH=CH-CH₂- | 5-nitro |
| *691 | Me | 2-Pyrazinyl- | S-CH₂-cycProp-(CH₂)₂- | 5-(pyrolidin-1-yl-sulfonyl) |
| 692 | Me | Pyridin-4-yl- | S-(CH₂)₃- | 5-methoxy |
| 693 | Pentyl | 4-Methylsulfonyl-phenyl | S-(CH₂)₈- | 5-(piperidin-1-yl-sulfonyl) |
| 694 | Pentyl | N-Methyl-2-Pyrrolyl- | S-(CH₂)₃- | 5-chlor |
| 695 | cycProp | Phenyl | O-(CH₂)₃- | 5-cyano |
| 696 | Me | 4-Imidazolyl- | S-(CH₂)₃- | 5-nitro |
| 697 | Me | 3-Pyrrolyl | S-CH₂-CH=CH-CH₂- | 6-chlor |
| *698 | Me | Oxadiazol-2-yl | (CH2)₂-CH(CH₃)-CH₂-CH₂- | 5-fluor |
| 699 | Me | 6-Chlor-biphenyl-2- | S-(CH₂)₃- | 5-nitro |
| 700 | Butyl | 4-Methoxyphenyl | S-(CH₂)₃- | 5-methoxy |
| 701 | Me | 3-Br-Pyridin-5-yl- | S-(CH₂)₃- | 5-nitro |
| 702 | Prop | N-Propyl-tetrazolyl- | S-CH₂-CH=CH-CH₂- | 5-chlor |
| 703 | Me | 5-Methyl imidazol-4-yl- | (CH₂)₂-CH(CH₃)-CH₂-CH₂- | tert-Butyl |
| 704 | Pentyl | Carboxamido | S-(CH₂)₃- | 5-fluor |
| 705 | Me | 2-Pyrazinyl- | S-(CH₂)₃- | 5-methoxy |
| *706 | Pentyl | Phenyl- | CO-(CH₂)₃- | 6-methoxy |
| 707 | Me | 4-Imidazolyl- | S-(CH₂)₃- | 5-methoxy |
| *708 | Me | Phenyl- | CO-(CH₂)₃- | 6-methoxy |
| 709 | Me | Tetrazolyl- | S-CH₂-C(=CH₂)-CH₂ | 5-nitro |
| 710 | cycProp | N-Methyl-2-Pyrrolyl- | S-(CH₂)₃- | 5-chlor |
| 711 | cycProp | Cyclohexyl- | S-(CH₂)₃- | 5-nitro |
| 712 | cycProp | Carboxamido | S-(CH₂)₃- | 5-chlor |
| 713 | iProp | 2,5-Di-methyl-furanyl-3- | S-(CH₂)₃- | 5-fluor |
| *714 | Me | Amino | S-CH₂-cycProp-(CH₂)₂- | 5-fluor |
| *715 | Me | 3-Thienyl | (CH₂)₂-CH(CH₃)-CH₂-CH₂- | 5-fluor |
| 716 | Me | 3-Thienyl | O-(CH₂)₃- | 5-nitro |
| *717 | Me | tert.-Butyl | CO-(CH₂)₃- | 6-methoxy |
| 718 | Me | Amino | S-(CH₂)₃- | 5-methoxy |
| 719 | Me | 2-Me-4-Oxazolyl- | S-(CH₂)₃- | 5-fluor |
| 720 | Et | Tetrazolyl- | S-CH₂-C(CH₃)=CH-CH₂- | 5-methoxy |
| 721 | Prop | Carboxamido | S-(CH₂)₃- | 5-chlor |
| 722 | Et | 4-Methylthiazol-5-yl | S-(CH₂)₃- | 5-methoxy |
| 723 | Me | 4-Imidazolyl- | S-(CH₂)₃- | 5-fluor |
| 724 | Me | 4-Methylthiazol-5-yl | S-(CH₂)₃- | 5-fluor |
| 725 | Et | 2-Me-4-Oxazolyl- | S-(CH₂)₃- | 5-methoxy |
| 726 | Butyl | 4-Methoxyphenyl | S-(CH₂)₃- | 5-fluor |
| 727 | Me | 2,5-Di-methyl-furanyl-3- | S-(CH₂)₃- | 5-methoxy |
| 728 | Me | 3-Br-Pyridin-5-yl- | S-(CH₂)₃- | 5-fluor |
| 729 | Pentyl | N-Methyl-2-Pyrrolyl- | S-(CH₂)₃- | 5-fluor |
| 730 | cycProp | N-Methyl-2-Pyrrolyl- | S-(CH₂)₃- | 5-nitro |
| 731 | Prop | Cyclohexyl- | S-(CH₂)₃- | 5-chlor |
| 732 | cycProp | 3-Pyridyl | S-(CH₂)₇- | 5-chlor |
| 733 | cycProp | Cyclohexyl- | S-(CH₂)₃- | 5-chlor |
| 734 | Me | N-Propyl-tetrazolyl- | S-(CH₂)₃- | 5-methoxy |
| 735 | Me | Methylamino | S-(CH₂)₃- | 5-nitro |
| 736 | Me | Pyridin-3-yl- | S-(CH₂)₃- | 5-nitro |
| 737 | Me | 2-Aminothiazol-4yl- | S-(CH₂)₃= | 5-nitro |
| 738 | Et | Oxadiazol-2-yl | S-(CH₂)₃- | 5-methoxy |
| 739 | Me | 3-Cyano-phenyl | S-CH₂-C(=CH₂)-CH₂ | 5-(piperidin-1-yl-sulfonyl) |
| *740 | cycProp | Phenyl- | CO-(CH₂)₃- | 6-methoxy |
| *741 | Me | 2-Me-4-Oxazolyl- | (CH₂)₂-CH(CH₃)-CH₂-CH₂- | 5-(morpholin-1-yl-sulfonyl) |
| 742 | Et | 2-Aminothiazol-4yl- | S-CH₂-CH=CH-CH₂- | 5-methoxy |
| 743 | Me | 2,5-Di-methyl-furanyl-3- | S-(CH₂)₃- | 5-nitro |
| *744 | Me | Phenyl | COO-(CH₂)₃- | 5-(piperidin-1-yl-sulfonyl) |
| 745 | Me | 2-Me-4-Oxazolyl- | S-(CH₂)₃- | 5-fluor |
| 746 | iProp | Pyridin-4-yl- | S-(CH₂)₃- | 5-fluor |
| *747 | Me | Methylamino | S-CH₂-cycProp-(CH₂)₂- | 5-methoxy |
| 748 | Me | 5-Methyl imidazol-4-yl- | S-(CH₂)₃- | 5-nitro |
| 749 | Me | 2-Me-4-Oxazolyl- | S-(CH₂)₃- | 5-cyano |
| 750 | cycProp | Carboxamido | S-(CH₂)₃- | 5-cyano |
| 751 | Me | Tetrazolyl- | S-(CH₂)₃- | 5-fluor |
| 752 | Pentyl | Cyclohexyl- | S-(CH₂)₃- | 5-fluor |
| 753 | Prop | N-Methyl-2-Pyrrolyl- | S-(CH₂)₃- | 5-chlor |
| 754 | Me | N-Methyl-2-Pyrrolyl- | S-(CH₂)₃- | 5-fluor |
| 755 | cycProp | N-Propyl-tetrazolyl- | S-CH₂-CH=CH-CH₂- | 5,6-dichlor |
| *756 | Pentyl | Propyl | CO-(CH₂)₃- | 5-methoxy |
| 757 | Me | 4-Methoxyphenyl | S-(CH₂)₃- | 5-nitro |
| *758 | Me | Propyl | CO-(CH₂)₃- | 5-methoxy |
| 759 | Me | 2-Me-4-Oxazolyl- | S-(CH₂)₃- | 5-methoxy |
| *760 | cycProp | Phenyl | CONH-(CH₂)₄- | 5-cyano |
| 761 | Me | Carboxamido | S-(CH₂)₃- | 5-cyano |
| *762 | Et | tert.Butyl | S-CH₂-cycProp-(CH₂)₂- | 5-methoxy |
| 763 | cycProp | N-Methyl-2-Pyrrolyl- | S-(CH₂)₃- | 5-fluor |

### Beispiele für galenische Applikationsformen

### A) Tabletten

Auf einer Tablettenpresse werden in üblicher Weise Tabletten folgender Zusammensetzung gepresst:

| | |
|---|---|
| 40 mg | Substanz des Beispiels 1 |
| 120 mg | Maisstärke |
| 13,5mg | Gelatine |
| 45 mg | Milchzucker |
| 2,25 mg | Aerosil® (chemisch reine Kieselsäure in submikroskopisch feiner Verteilung) |
| 6,75 mg | Kartoffelstärke (als 6%-iger Kleister) |

### B) Dragees

| | |
|---|---|
| 20 mg | Substanz des Beispiels 3 |
| 60 mg | Kernmasse |
| 70 mg | verzuckerungsmasse |

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus
5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Biologische Untersuchungen - Rezeptorbindungsstudien

### 1) D₃-Bindungstest

Für die Bindungsstudien wurden klonierte humane D₃-Rezeptorexprimierende CCL 1,3 Mäusefibroblasten, erhältlich bei Res. Biochemicals Internat. One Strathmore Rd., Natick, MA 01760-2418 USA, eingesetzt.

### Zellpräparation

Die D₃ exprimierenden Zellen wurden in RPMI-1640 mit 10% fötalem Kälberserum (GIBCO Nr. 041-32400 N); 100 E/ml Penicillin und 0,2 % Streptomycin (GIBCO BRL, Gaithersburg, MD, USA) vermehrt. Nach 48 h wurden die Zellen mit PBS gewaschen und mit 0,05% trypsinhaltiger PBS 5 min inkubiert. Danach wurde mit Medium neutralisiert und die Zellen durch Zentrifugation bei 300 g gesammelt. Zur Lyse der Zellen wurde kurz das Pellet mit Lysispuffer (5mM Tris-HCl, pH 7,4 mit 10% Glycerin) gewaschen und danach in einer Konzentration von 10⁷-Zellen / ml Lysispuffer 30 min bei 4°C inkubiert. Die Zellen wurden bei 200 g 10 min zentrifugiert und das Pellet in flüssigem Stickstoff gelagert.

### Bindungstests

Für den D₃-Rezeptorbindungstest wurden die Membranen in Inkubationspuffer (50 mM Tris-HCl, pH 7,4 mit 120 mM NaCl, 5 mM KCl, 2 mM CaCl₂, 2 mM MgCl₂, 10µM Quinolinol, 0,1% Ascorbinsäure und 0,1% BSA) in einer Konzentration von ca. 10⁶ Zellen/250 µl Testansatz suspendiert und bei 30°C mit 0,1 nM ¹²⁵Jodsulpirid in Anwesenheit und Abwesenheit von Testsubstanz inkubiert. Die unspezifische Bindung wurde mit 10⁻⁶M Spiperon bestimmt.

Nach 60 min wurde der freie und der gebundene Radioligand durch Filtration über GF/B Glasfaserfilter (Whatman, England) an einem Skatron-zellsammler (Skatron, Lier, Norwegen) getrennt und die Filter mit eiskaltem Tris-HCl-Puffer, pH 7,4 gewaschen. Die auf den Filtern gesammelte Radioaktivität wurde mit einem Packard 2200 CA Flüssigkeitszintillationszähler quantifiziert.

Die Bestimmung der Kᵢ-werte erfolgte über nichtlineare Regressionsanalyse mit dem Programm LIGAND.

### 2) D₂-Bindungstest

### Zellkultur

HEK-293 Zellen mit stabil exprimierten humanen Dopamin-D2A-Rezeptoren wurden in RPMI 1640 mit Glutamax I™ und 25 mM HE-PES mit 10% fötalem Kälberserumalbumin kultiviert. Alle Medien enthielten 100 Einheiten pro ml Penicillin und 100 µg/ml Streptomycin. Die Zellen wurden in feuchter Atmosphäre mit 5% CO₂ bei 37°C gehalten.

Die Zellpräparation für Bindungsstudien erfolgte durch Trypsinisierung (0,05% Trypsinlösung) für 3-5 Minuten bei Raumtemperatur. Danach wurden die Zellen bei 250 g 10 Minuten zentrifugiert und 30 Minuten bei 4°C mit Lysispuffer (5 mM Tris-HCl, 10% Glycerol, pH 7,4) behandelt. Nach Zentrifugation bei 250 g für 10 Minuten wurde der Rückstand bei -20°C bis zum Gebrauch aufbewahrt.

### Rezeptorbindungstests

Dopamin-D₂-Rezeptor "low affinity state" mit ¹²⁵I-Spiperon (81 TBq/mmol, Du Pont de Nemours, Dreieich)

Die Ansätze (1 ml) setzten sich zusammen aus 1 x 10⁵ Zellen in Inkubationspuffer (50 mM Tris, 120 mM NaCl, 5 mM KCl, 2 mM MgCl₂ und 2 mM CaCl₂, pH 7,4 mit HCl) und 0,1 nM ¹²⁵I-Spiperon (totale Bindung) oder zusätzlich 1 µM Haloperidol (unspezifische Bindung) oder Prüfsubstanz.

Nach erfolgter Inkubation bei 25°C für 60 Minuten wurden die Ansätze über GF/B Glasfaserfilter (Whatman, England) an einem Skatron-Zellsammler (Fa. Zinsser, Frankfurt) filtriert und die Filter mit eiskaltem 50 mM Tris-HCl-Puffer, pH 7,4 gewaschen. Die auf den Filtern gesammelte Radioaktivität wurde mit einem Packard 2200 CA Flüssigkeitszintillationszähler quantifiziert.

### Die Auswertung erfolgte wie unter a).

Die Bestimmung der Ki-Werte erfolgte über nichtlineare Regressionsanalyse mit dem Programm LIGAND oder durch Umrechnung der IC₅₀-Werte mit Hilfe der Formel von Cheng und Prusoff.

Die erfindungsgemäßen Verbindungen zeigen in diesen Tests sehr gute Affinitäten am D₃-Rezeptor (< 1 µmolar, insbesondere < 100 nmolar) und binden selektiv an den D₃-Rezeptor.

In Tabelle 3 sind für die Verbindungen der Beispiele 3, 4 und 7 die pKᵢ(D₃)-Werte (negativ dekadischer Logarithmus der Bindungskonstante an den D₃-Rezeptor) und die Selektivität gegenüber der Bindung am D₂A-Rezeptor (Kᵢ(D₂) / Kᵢ(D₃)) angegeben.

**Tabelle 3:**

| Beispiel | pKᵢ(D₃) | Selektivität |
|---|---|---|
| 3 | 8,02 | 78 |
| 4 | 7,96 | 67 |
| 7 | 8,37 | 81 |

## Patentansprüche

1. Triazolverbindungen der allgemeinen Formel I 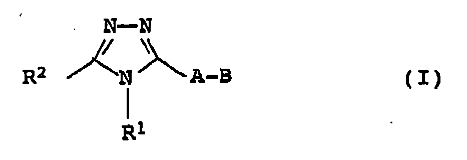 worin
R¹ für H, C₁-C₆-Alkyl, das gegebenenfalls durch OH, OC₁-C₆-Alkyl, Halogen oder Phenyl substituiert ist, C₃-C₆-Cycloalkyl oder Phenyl steht;
R² für H, C₁-C₆-Alkyl, das gegebenenfalls durch OH, OC₁-C₆-Alkyl, Halogen oder Phenyl substituiert ist, C₁-C₆-Alkoxy, C₁-C₆-Alkylthio, C₂-C₆-Alkenyl, C₂-C₆-Alki- nyl, C₃-C₆-Cycloalkyl, Halogen, CN, COOR³, CONR³R⁴, NR³R⁴, SO₂R³, SO₂NR³R⁴ oder einen aromatischen Rest steht, der ausgewählt ist unter Phenyl, Naphthyl und einem 5- oder 6-gliedrigen heterocyclischen Rest mit 1, 2, 3 oder 4 He- teroatomen, die unabhängig voneinander ausgewählt sind unter O, N und S, wobei der aromatische Rest einen oder zwei Substituenten aufweisen kann, die unabhängig vonein- ander ausgewählt sind unter C₁-C₆-Alkyl, das gegebenen- falls durch OH, OC₁-C₆₋Alkyl, Halogen oder Phenyl substi- tuiert ist, C₁-C₆-Alkoxy, C₂-C₆-Alkenyl, C₂-C₆-Alkinyl, C₃-C₆-Cycloalkyl, Halogen, CN, COR³, NR³R⁴, NO₂, SO₂R³, SO₂NR³R⁴ und Phenyl, das gegebenenfalls durch ein oder zwei Reste substituiert ist, die unabhängig voneinander ausgewählt sind unter C₁-C₆-Alkyl, C₁-C₆-Alkoxy, NR³R⁴, CN, CF₃, CHF₂ oder Halogen;
R³ und R⁴ unabhängig voneinander für H, C₁-C₆-Alkyl, das gege- benenfalls durch OH, OC₁-C₆-Alkyl, Halogen oder Phenyl substituiert ist, oder Phenyl, stehen;
A für -Z-CH₂CH₂CS₂-, -Z-CH₂CH₂CH₂CH₂-, -Z-CH₂CH=CHCH₂-, -Z-CH₂C(CH₃)=CHCH₂-, 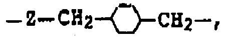 -Z-CH₂CH(CH₃)CH₂- oder für einen linearen -Z-C₇-C₁₀-Alkylenrest steht, wobei Z an den Triazolring gebunden ist und wobei Z für CH₂, O oder S steht, oder A steht für -(CH₂)₄-, -(CH₂)₅-, -CH₂CH₂CH=CHCH₂-, 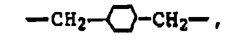 -CH₂CH₂C(CH₃)=CHCH₂- oder -CH₂CH₂CH(CH₃)CH₂-;
B einen Rest der folgenden Formel bedeutet: 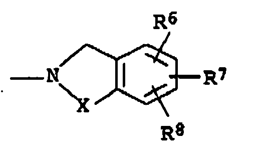 worin
X für CH₂ oder CH₂CH₂ steht;
R⁶, R⁷ und R⁸ unabhängig voneinander ausgewählt sind unter H, C₁-C₆-Alkyl, das gegebenenfalls durch OB, OC₁-C₆-Alkyl, das gegebenenfalls durch Amino, Mono- oder Di-C₁-C₄-alky- lamino substituiert ist, C₁-C₆-Alkylthio, Halogen oder Phenyl substituiert ist, OB, C₁-C₆-Alkoxy, OCF₃, OSO₂CF₃, SH, C₁-C₆-Alkylthio, C₂-C₆-Alkenyl, C₂-C₆-Alkinyl, Halo- gen, CN, NO₂, CO₂R³, SO₂R³, SO₂NR³R⁴, wobei R³ und R⁴ die oben angegebenen Bedeutungen besitzen und zusammenmit dem N-Atom, an das sie gebunden sind, auch einen gesättigten oder ungesättigten Heterocyclus mit 5 bis 7 Ringatomen und 1 oder 2 N- und/oder O-Heteroatomen bilden können, CONR³R⁴, NHSO₂R³, NR³R⁴, einem 5- oder 6-gliedrigen carbo- cyclischen, aromatischen oder nicht-aromatischen Ring und einem 5- oder 6-gliedrigen heterocyclischen, aromatischen oder nicht-aromatischen Ring mit 1 oder 2 Heteroatomen, die unabhängig voneinander ausgewählt sind unter 0, N und S, wobei der carbocyclische oder heterocyclische Ring ein oder zwei Substituenten aufweisen kann, die unabhängig voneinander ausgewählt sind unter C₁-C₆-Alkyl, Phenyl, Phenoxy, Halogen, C₁-C₆-Alkoxy, OH, NO₂, CF₃ und CHF₂ und wobei zwei der Substituenten R⁶, R⁷ und R⁸ zusammen mit den Kohlenstoffatomen des Phenylrings, an die sie gebun- den sind, einen an den Phenylring ankondensierten Phe- nyl-, Cyclopentyl- oder Cyclohexylring bilden können, wo- rin eine oder zwei der CH- oder der CH₂-Gruppen durch ein Stickstoffatom oder eine NH- oder N-C₁-C₆-Alkylgruppe er- setzt sein können;
sowie deren Salze mit physiologisch verträglichen Säuren.

2. Verbindungen nach Anspruch 1 der Formel I, worin X für CH₂CH₂ steht.

3. Verbindungen nach einem der vorhergehenden Ansprüche der Formel I, worin R² für einen aromatischen Rest steht, der unsubstituiert ist oder einen oder zwei Substituenten aufweist, die unabhängig voneinander ausgewählt sind unter C₁-C₆-Alkyl, OH, C₁-C₆-Alkoxy, Phenyl, CN und Halogen.

4. Verbindungen nach einem der vorhergehenden Ansprüche der Formel I, worin R² für H, C₁-C₆-Alkyl, Phenyl, Thienyl, Furanyl, Tetrazolyl, Pyrrolyl, Pyridyl oder Pyrazinyl steht.

5. Verbindungen nach einem der vorhergehenden Ansprüche der Formel I, worin R¹ für H, C₁-C₆-Alkyl oder C₃-C₆-Cycloalkyl steht.

6. Verbindungen nach einem der vorhergehenden Ansprüche der Formel I, worin R⁶, R⁷ und R⁸ unabhängig voneinander ausgewählt sind unter H, C₁-C₆-Alkyl, OH, C₁-C₆-Alkoxy, C₁-C₆-Alkylthio-C₁-C₆-alkyl, Halogen, CN, NO₂, SO₂R³, SO₂NR³R⁴ und CONR³R⁴.

7. Verbindungen der Formel I, worin
R¹ für H, C₁C₆-Alkyl oder Phenyl steht,
R² für H, C₁-C₆-Alkyl, Phenyl, Thienyl, Furanyl, Tetrazolyl, Pyrrolyl, Thiazolyl oder Pyrazinyl steht,
A für -SC₃-C₁₀-Alkylen, das gegebenenfalls eine Doppelbin- dung umfassen kann, steht, und
B einen Rest der folgenden Formel bedeutet: 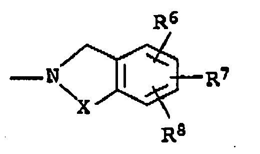 worin
X für CH₂ oder CH₂CH₂ steht;
R⁶, R⁷ und R⁸ ausgewählt sind unter H, C₁-C₆-Alkyl, C₁-C₆-Al- koxy, Halogen, SO₂NR³R⁴, CN, NO₂, CF₃, CONR³R⁴, CEF₂, OSO₂CF₃, OCF₃ und NHSO₂-C₁-C₆-alkyl.

8. Verbindung ausgewählt unter 1-{2-[3-({4-Methyl-5-[4-(trifluoromethyl)phenyl]-4H-1,2,4-triazol-3-yl}sulfanyl)propyl]-1,2,3,4-tetrahydroisochinolin-7-yl}-ethanon;
1-(2-{3-[(4-Methyl-5-(3-cyano)phenyl-4H-1,2,4-triazol-3-yl)sufanyl]propyl}-1,2,3,4-tetrahydroisochinolin-7-yl)ethanon;
1-{2-[3-({4-Methyl-4-phenyl-4H-1,2,4-triazol-3-yl}sulfanyl)propyl]-1,2,3,4-tetrahydroisochinolin-7-yl}ethanon;
1-{2-[3-({5-(2,4-Dinitrophenyl)-4-methyl]-4H-1,2,4-triazol-3-yl}-sulfanyl)propyl]-1,2,3,4-tetrahydroisochinolin-7-yl}ethanon;
1-(2-{3-[(4-Methyl-5-(3-methoxy)phenyl-4H-1,2,4-triazol-3-yl)-oxy]propyl}-1,2,3,4-tetrahydro-isochinolin-7-yl)ethanon;
1-{2-[3-({4-Isopropyl-5-phenyl-4H-1,2,4-triazol-3-yl}sulfanyl)propyl]-1,2,3,4-tetrahydroisochinolin-7-yl}ethanon;
1-{2-[3-({4-Butyl-5-phenyl-4H-1,2,4-triazol-3-yl}sulfanyl)propyl]-1,2,3,4-tetrahydroisochinolin-7-yl}ethanon;
1-{2-[3-({5-Phenyl-4-pxopyl-4H-1,2,4-triazol-3-yl}sulfanyl)propyl]-1,2,3,4-tetrahydroisochinolin-7-yl}ethanon;
1-{2-[3-({4-Cyclopropyl-5-phenyl-4H-1,2,4-triazol-3-yl}sulfanyl)propyl]-1,2,3,4-tetrahydroisochinolin-7-yl}ethanon;
1-{2-[3-({4-Ethyl-5-phenyl-4H-1,2,4-triazol-3-yl}sulfanyl)propyl]-1,2,3,4-tetrahydroinochinolin-7-yl}ethanon;
1-(4-Methyl-5-phenyl-4H-1,2,4-triazol-3-yl)-4-(7-(piperidin-1-yl-sulfonyl)-1,2,3,4-tetrahydroisochinolin-2-yl)butan-1-on und
2-[2-({[4-Methyl-5-phenyl-4H-1,2,4-triazol-3-yl]sulfanyl}methyl)prop-2-enyl]-7-nitro-1,2,3,4-tetrahydroisochinolin Hydrochlorid.

9. Pharmazeutisches Mittel, enthaltend wenigstens eine Verbindung nach einem der Ansprüche 1 bis 8, gegebenenfalls zusammen mit physiologisch akzeptablen Trägern und/oder Hilfsstoffen.

10. Verwendung wenigstens einer Verbindung nach einem der Ansprüche 1 bis 8 zur Herstellung eines pharmazeutischen Mittels zur Behandlung von Erkrankungen, die auf die Beeinflussung durch Dopamin-D₃-Rezeptorantagonisten bzw. -agonisten ansprechen.

## Claims

1. Triazole compounds of the formula I 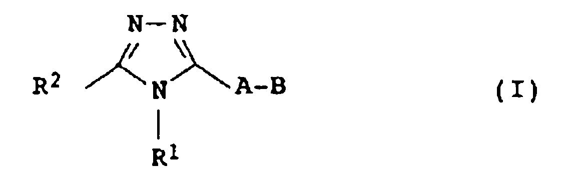 where
R¹ is H, C₁-C₆-alkyl, which may be substituted by OH, OC₁-C₆-alkyl, halogen or phenyl, C₃-C₆- cycloalkyl or phenyl;
R² is H, C₁-C₆-alkyl, which may be substituted by OH, OC₁-C₆-alkyl, halogen or phenyl, C₁-C₆- alkoxy, C₁-C₆-alkylthio, C₂-C₆-alkenyl, C₂-C₆- alkynyl, C₃-C₆-cycloalkyl, halogen, CN, COOR³, CONR³R⁴, NR³R⁴, SO₂R³, SO₂NR³R⁴ or an aromatic radical which is selected from phenyl, naphthyl and a 5- or 6-membered heterocyclic radical having 1, 2, 3 or 4 heteroatoms which are selected, independently of each other, from O, N and S, with it being possible for the aromatic radical to have one or two substituents which are selected, independently of each other, from C₁-C₆-alkyl, which may be substituted by OH, OC₁-C₆-alkyl, halogen or phenyl, C₁-C₆-alkoxy, C₂-C₆-alkenyl, C₂-C₆-alkynyl, C₃-C₆-cycloalkyl, halogen, CN, COR³, NR³R⁴, NO₂, SO₂R³, SO₂NR³R⁴ and phenyl which may be substituted by one or two radicals which are selected, independently of each other, from C₁-C₆-alkyl, C₁-C₆-alkoxy, NR³R⁴, CN, CF₃, CHF₂ or halogen;
R³ and R⁴ are, independently of each other, H, C₁-C₆-alkyl, which may be substituted by OH, OC₁-C₆-alkyl, halogen or phenyl, or phenyl;
A is -Z-CH₂CH₂CH₂-, -Z-CH₂CH₂CH₂CH₂-, -Z-CH₂CH=CHCH₂-, -Z-CH₂C(CH₃)=CHCH₂-, 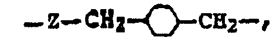 -Z-CH₂CH(CH₃)CH₂- or a linear -Z-C₇-C₁₀-alkylene radical, with Z being bonded to the triazole ring and with Z being CH₂, O and S, or A is -(CH₂)₄-, -(CH₂)₅-, -CH₂CH₂CH=CHCH₂-, 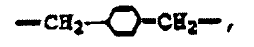 -CH₂CH₂C(CH₃)=CHCH₂- or -CH₂CH₂CH(CH₃)CH₂-;
B is a radical of the following formula: 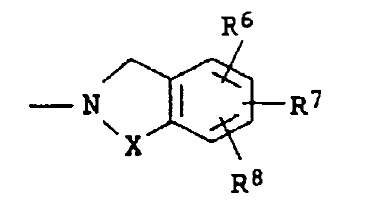 where
X is CH₂ or CH₂CH₂;
R⁶, R⁷ and R⁸ are, independently of each other, selected from H, C₁-C₆-alkyl, which may be substituted by OH, OC₁-C₆-alkyl, which may be substituted by amino, mono- or di-C₁-C₄- alkylamino, C₁-C₆-alkylthio, halogen or phenyl, OH, C₁-C₆-alkoxy, OCF₃, OSO₂CF₃, SH, C₁-C₆-alkylthio, C₂-C₆-alkenyl, C₂-C₆-alkynyl, halogen, CN, NO₂, CO₂R³, SO₂R³, SO₂NR³R⁴, where R³ and R⁴ have the abovementioned meanings and may also form together with the N atom to which they are bonded a saturated or unsaturated heterocycle with 5 to 7 ring atoms and 1 or 2 N and/or O heteroatoms, CONR³R⁴, NHSO₂R³, NR³R⁴, a 5- or 6-membered carbocyclic, aromatic or nonaromatic ring and a 5- or 6-membered heterocyclic, aromatic or nonaromatic ring with 1 or 2 heteroatoms which are selected, independently of each other, from O, N and S, with the carbocyclic or heterocyclic ring being able to have one or two substituents which are selected, independently of each other, from C₁-C₆-alkyl, phenyl, phenoxy, halogen, C₁-C₆-alkoxy, OH, NO₂, CF₃ and CHF₂, and with two of the substituents R⁶, R⁷ and R⁸ being able to form, together with the carbon atoms of the phenyl ring to which they are bonded, a phenyl, cyclopentyl or cyclohexyl ring which is fused to the phenyl ring, in which one or two of the CH or CH₂ groups can be replaced by a nitrogen atom or an NH or N-C₁-C₆-alkyl group;
and the salts thereof with physiologically tolerated acids.

2. Compounds according to claim 1 of the formula I, where X is CH₂CH₂.

3. Compounds according to one of the preceding claims of the formula I, where R² is an aromatic radical which is unsubstituted or has one or two substituents which are selected, independently of each other, from C₁-C₆-alkyl, OH, C₁-C₆-alkoxy, phenyl, CN and halogen.

4. Compounds according to one of the preceding claims of the formula I, where R² is H, C₁-C₆-alkyl, phenyl, thienyl, furanyl, tetrazolyl, pyrrolyl, pyridyl or pyrazinyl.

5. Compounds according to one of the preceding claims of the formula I, where R¹ is H, C₁-C₆-alkyl or C₃-C₆-cycloalkyl.

6. Compounds according to one of the preceding claims of the formula I, where R⁶, R⁷ and R⁸ are selected, independently of each other, from H, C₁-C₆-alkyl, OH, C₁-C₆-alkoxy, C₁-C₆-akylthio-C₁-C₆-alkyl, halogen, CN, NO₂, SO₂R³, SO₂NR³R⁴ and CONR³R⁴.

7. Compounds of the formula I, where
R¹ is H, C₁-C₆-alkyl or phenyl,
R² is H, C₁-C₆-alkyl, phenyl, thienyl, furanyl, tetrazolyl, pyrrolyl, thiazolyl or pyrazinyl,
A is -SC₃-C₁₀-alkylene which may comprise a double bond, and
B is a radical of the following formula: 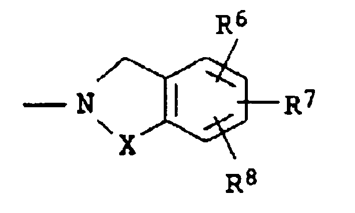 where
X is CH₂ or CH₂CH₂;
R⁶, R⁷ and R⁸ are selected from H, C₁-C₆-alkyl, C₁-C₆-alkoxy, halogen, SO₂NR³R⁴, CN, NO₂, CF₃, CONR³R⁴, CHF₂, OSO₂CF₃, OCF₃ and NHSO₂-C₁-C₆- alkyl.

8. Compound selected from 1-{2-[3-({4-methyl-5-[4-(trifluoromethyl)phenyl]-4H-1,2,4-triazol-3-yl}-sulphanyl)propyl]-1,2,3,4-tetrahydroisoquinolin-7-yl}ethanone;
1-(2-{3-[(4-methyl-5-(3-cyano)phenyl-4H-1,2,4-triazol-3-yl)sulphanyl]propyl}-1,2,3,4-tetrahydroisoquinolin-7-yl)ethanone;
1-{2-[3-({4-methyl-5-phenyl-4H-1,2,4-triazol-3-yl}sulphanyl)propyl]-1,2,3,4-tetrahydroisoquinolin-7-yl}ethanone;
1-{2-[3-({5-(2,4-dinitrophenyl)-4-methyl]-4H-1,2,4-triazol-3-yl})sulphanyl)propyl]-1,2,3,4-tetrahydroisoquinolin-7-yl}ethanone;
1-(2-{3-[(4-methyl-5-(3-methoxy)phenyl-4H-1,2,4-triazol-3-yl)oxy]propyl}-1,2,3,4-tetrahydroisoquinolin-7-yl)ethanone;
1-{2-[3-({4-isopropyl-5-phenyl-4H-1,2,4-triazol-3-yl}sulphanyl)propyl]-1,2,3,4-tetrahydroisoquinolin-7-yl}ethanone;
1-{2-[3-({4-butyl-5-phenyl-4H-1,2,4-triazol-3-yl}sulphanyl)propyl]-1,2,3,4-tetrahydroisoquinolin-7-yl}ethanone;
1-{2-[3-({5-phenyl-4-propyl-4H-1,2,4-triazol-3-yl}sulphanyl)propyl]-1,2,3,4-tetrahydroisoquinolin-7-yl} ethanone;
1-{2-[3-({4-cyclopropyl-5-phenyl-4H-1,2,4-triazol-3-yl}sulphanyl)propyl]-1,2,3,4-tetrahydroisoquinolin-7-yl}ethanone;
1-{2-[3-({4-ethyl-5-phenyl-4H-1,2,4-triazol-3-yl}sulphanyl)propyl]-1,2,3,4-tetrahydroisoquinolin-7-yl}ethanone;
1-(4-methyl-5-phenyl-4H-1,2,4-triazol-3-yl)-4-(7-(piperidin-1-ylsulphonyl)-1,2,3,4-tetrahydroisoquinolin-2-yl)butan-1-one; and
2-[2-({[4-methyl-5-phenyl-4H-1,2,4-triazol-3-yl]-sulphanyl}methyl)prop-2-enyl]-7-nitro-1,2,3,4-tetrahydroisoquinoline hydrochloride.

9. Pharmaceutical which comprises at least one compound according to one of claims 1 to 8, where appropriate together with physiologically acceptable excipients and/or adjuvants.

10. Use of at least one compound according to one of claims 1 to 8 for preparing a pharmaceutical for treating diseases which respond to the influence of dopamine D₃ receptor antagonists or agonists.

## Revendications

1. Composés de type triazole de formule générale I 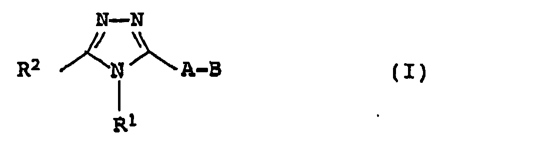 où
R¹ représente H, C₁-C₆-alkyle, qui est le cas échéant substitué par OH, OC₁-C₆-alkyle, halogène ou phényle, C₃-C₆-cycloalkyle ou phényle ;
R² représente H, C₁-C₆-alkyle, qui est le cas échéant substitué par OH, OC₁-C₆-alkyle, halogène ou phényle, C₁-C₆-alcoxy, C₁-C₆-alkylthio, C₂-C₆- alcényle, C₂-C₆-alcynyle, C₃-C₆-cycloalkyle, halogène, CN, COOR³, CONR³R⁴, NR³R⁴, SO₂R³, SO₂NR³R⁴ ou un radical aromatique, qui est choisi parmi phényle, naphtyle et un radical hétérocyclique de 5 ou 6 chaînons, comprenant 1, 2, 3 ou 4 hétéroatomes, qui sont choisis, indépendamment l'un de l'autre, parmi O, N et S, où le radical aromatique peut présenter un ou deux substituants, qui sont choisis, indépendamment l'un de l'autre, parmi C₁-C₆-alkyle, qui est le cas échéant substitué par OH, OC₁-C₆-alkyle, halogène ou phényle, C₁-C₆-alcoxy, C₂-C₆-alcényle, C₂-C₆- alcynyle, C₃-C₆-cycloalkyle, halogène, CN, COR³, NR³R⁴, NO₂, SO₂R³, SO₂NR³R⁴ et phényle, qui est le cas échéant substitué par un ou deux radicaux, qui sont choisis, indépendamment l'un de l'autre, parmi C₁-C₆-alkyle, C₁-C₆-alcoxy, NR³R⁴, CN, CF₃, CHF₂ ou halogène ;
R³ et R⁴ représentent, indépendamment l'un de l'autre H, C₁-C₆-alkyle, qui est le cas échéant substitué par OH, OC₁-C₆-alkyle, halogène ou phényle, ou phényle ;
A représente -Z-CH₂CH₂CH₂-, -Z-CH₂CH₂CH₂CH₂-, -Z-CH₂CH=CHCH₂-, -Z-CH₂C(CH₃)=CHCH₂-, 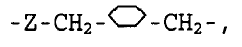 -Z-CH₂CH(CH₃)CH₂- ou un radical -Z-C₇-C₁₀-alkylène linéaire, où Z est lié au cycle triazole et où Z représente CH₂, O ou S, ou A représente -(CH₂)₄-, -(CH₂)₅-, -CH₂CH₂CH=CHCH₂-, 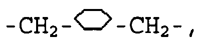 -CH₂CH₂C(CH₃)=CHCH₂- ou -CH₂CH₂CH(CH₃)CH₂- ;
B signifie un radical présentant la formule suivante : 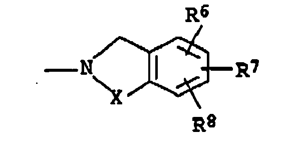 où
X représente CH₂ ou CH₂CH₂ ;
R⁶, R⁷ et R⁸ sont choisis, indépendamment l'un de l'autre, parmi H, C₁-C₆-alkyle, qui est le cas échéant substitué par OH, OC₁-C₆-alkyle, qui est le cas échéant substitué par amino, mono-C₁-C₄-alkylamino ou di-C₁-C₄-alkylamino, C₁-C₆-alkylthio, halogène ou phényle, OH, C₁-C₆-alcoxy, OCF₃, OSO₂CF₃, SH, C₁-C₆-alkylthio, C₂-C₆-alcényle, C₂-C₆-alcynyle, halogène, CN, NO₂, CO₂R³, SO₂R³, SO₂NR³R⁴, où R³ et R⁴ présentent les significations susmentionnées et peuvent également former avec l'atome de N auquel ils sont liés un hétérocycle saturé ou insaturé comprenant 5 à 7 atomes de cycle et 1 ou 2 hétéroatomes N et/ou O, CONR³R⁴, NHSO₂R³, NR³R⁴, un cycle carbocyclique, aromatique ou non aromatique de 5 ou 6 chaînons et un cycle hétérocyclique, aromatique ou non aromatique de 5 ou 6 chaînons avec 1 ou 2 hétéroatomes, qui sont choisis, indépendamment l'un de l'autre, parmi O, N et S, où le cycle carbocyclique ou hétérocyclique peut présenter un ou deux substituants, qui sont choisis indépendamment l'un de l'autre, parmi C₁-C₆-alkyle, phényle, phénoxy, halogène, C₁-C₆-alcoxy, OH, NO₂, CF₃ et CHF₂ et où deux des substituants R⁶, R⁷ et R⁸, ensemble avec les atomes de carbone du cycle phényle auxquels ils sont liés, peuvent former un cycle phényle, cyclopentyle ou cyclohexyle condensé sur le cycle phényle, où un ou deux des groupes CH ou CH₂ peuvent être remplacés par un atome d'azote ou un groupe NH ou N-C₁-C₆-alkyle ;
ainsi que leurs sels avec des acides physiologiquement acceptables.

2. Composés selon la revendication 1 de formule I, dans laquelle X représente CH₂CH₂.

3. Composés selon l'une quelconque des revendications précédentes de formule I, dans laquelle R² représente un radical aromatique, qui est non substitué ou qui présente un ou deux substituants, qui sont choisis, indépendamment l'un de l'autre, parmi C₁-C₆-alkyle, OH, C₁-C₆-alcoxy, phényle, CN et halogène.

4. Composés selon l'une quelconque des revendications précédentes de formule I, dans laquelle R² représente H, C₁-C₆-alkyle, phényle, thiényle, furannyle, tétrazolyle, pyrrolyle, pyridyle ou pyrazinyle.

5. Composés selon l'une quelconque des revendications précédentes de formule I, dans laquelle R¹ représente H, C₁-C₆-alkyle ou C₃-C₆-cycloalkyle.

6. Composés selon l'une quelconque des revendications précédentes de formule I, dans laquelle R⁶, R⁷ et R⁸ sont choisis, indépendamment l'un de l'autre, parmi H, C₁-C₆-alkyle, OH, C₁-C₆-alcoxy, C₁-C₆-alkylthio-C₁-C₆-alkyle, halogène, CN, NO₂, SO₂R³, SO₂NR³R⁴ et CONR³R⁴_{.}

7. Composés de formule I, dans laquelle
R¹ représente H, C₁-C₆-alkyle ou phényle,
R² représente H, C₁-C₆-alkyle, phényle, thiényle, furannyle, tétrazolyle, pyrrolyle, thiazolyle ou pyrazinyle,
A représente -SC₃-C₁₀-alkylène, qui peut le cas échéant comprendre une double liaison et
B signifie un radical présentant la formule suivante : 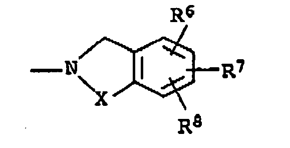 où
X représente CH₂ ou CH₂CH₂ ;
R⁶, R⁷ et R⁸ sont choisis parmi H, C₁-C₆-alkyle, C₁-C₆-alcoxy, halogène, SO₂NR³R⁴, CN, NO₂, CF₃, CONR³R⁴, CHF₂, OSO₂CF₃, OCF₂ et NHSO₂-C₁-C₆-alkyle.

8. Composé choisi parmi la 1-{2-[3-({4-méthyl-5-[4-(trifluorométhyl)phényl]-4H-1,2,4-triazol-3-yl}sulfanyl)propyl]-1,2,3,4-tétrahydroisoquinoléin-7-yl}-éthanone ;
la 1-(2-{3-[(4-méthyl-5-(3-cyano)phényl-4H-1,2,4-triazol-3-yl)sufanyl]propyl}-1,2,3,4-tétrahydroisoquinoléin-7-yl)éthanone ;
la 1-{2-[3-({4-méthyl-5-phényl-4H-1,2,4-triazol-3-yl}sulfanyl)propyl]-1,2,3,4-tétrahydroisoquinoléin-7-yl}-éthanone ;
la 1-{2-[3-({5-(2,4-dinitrophényl)-4-méthyl-4H-1,2,4-triazol-3-yl}-sulfanyl)propyl]-1,2,3,4-tétrahydroisoquinoléin-7-yl}-éthanone ;
la 1-(2-{3-[(4-méthyl-5-(3-méthoxy)phényl-4H-1,2,4-triazol-3-yl)-oxy]propyl}-1,2,3,4-tétrahydroisoquinoléin-7-yl)-éthanone ;
la 1-{2-[3-({4-isopropyl-5-phényl-4H-1,2,4-triazol-3-yl}sulfanyl)propyl]-1,2,3,4-tétrahydroisoquinoléin-7-yl}-éthanone ;
la 1-{2-[3-({4-butyl-5-phényl-4H-1,2,4-triazol-3-yl}sulfanyl)propyl]-1,2,3,4-tétrahydroisoquinoléin-7-yl}-éthanone ;
la 1-{2-[3-({5-phényl-4-propyl-4H-1,2,4-triazol-3-yl}sulfanyl)propyl]-1,2,3,4-tétrahydroisoquinoléin-7-yl}-éthanone ;
la 1-{2-[3-({4-cyclopropyl-5-phényl-4H-1,2,4-triazol-3-yl}sulfanyl)propyl]-1,2,3,4-tétrahydroisoquinoléin-7-yl}-éthanone ;
la 1-{2-[3-({4-éthyl-5-phényl-4H-1,2,4-triazol-3-yl}sulfanyl)propyl]-1,2,3,4-tétrahydroisoquinoléin-7-yl}-éthanone ;
la 1-(4-méthyl-5-phényl-4H-1,2,4-triazol-3-yl)-4-(7-(pipéridin-1-yl-sulfonyl)-1,2,3,4-tetrahydroisoquinoléin-2-yl)-butan-1-one ; et
le chlorhydrate de 2-[2-({[4-méthyl-5-phényl-4H-1,2,4-triazol-3-yl]sulfanyl}méthyl)prop-2-ényl]-7-nitro-1,2,3,4-tétrahydroisoquinoléine.

9. Agent pharmaceutique, contenant au moins un composé selon l'une quelconque des revendications 1 à 8, le cas échéant ensemble avec des supports et/ou des adjuvants physiologiquement acceptables.

10. Utilisation d'au moins un composé selon l'une quelconque des revendications 1 à 8 pour la préparation d'un agent pharmaceutique pour le traitement de maladies qui répondent à des influences par des antagonistes ou des agonistes des récepteurs dopamine-D₃.
